# EUROPEAN PATENT SPECIFICATION

(11) **EP 1 181 319 B1**
(45) Date of publication and mention of the grant of the patent: **22.04.2009**
(21) Application number: 00937844.9
(22) Date of filing: 25.05.2000
(51) Int. Cl.: C07K 16/28, C12N 5/20, C12N 15/13, C12N 15/63, A61K 39/395, A61P 37/00

(54) **CHIMERIC DR4 ANTIBODIES AND USES THEREOF**
CHIMÄRISCHE DR4 ANTIKÖRPER UND IHRE VERWENDUNG
ANTICORPS DR4 CHIMERIQUES ET UTILISATION DE CES DERNIERS

(30) Priority: 28.05.1999 US 322875
(43) Date of publication of application: 27.02.2002
(73) Proprietor: Genentech, Inc., South San Francisco CA 94080-4990 (US)
(72) Inventor: ASHKENAZI, Avi, J., San Mateo, CA 94402 (US); CHUNTHARAPAI, Anan, Colma, CA 94015 (US); DODGE, Kelly, H., San Mateo, CA 94402 (US); KIM, Kyung, Jin, Cupertino, CA 95014 (US)
(74) Representative: Kiddle, Simon John
(86) International application number: PCT/US2000/014599
(87) International publication number: WO 2000/073349

(56) References cited:
- WO-A-98/32856
- WO-A-98/51793
- WO-A-99/37684
- WO-A-99/64461
- CHUNTHARAPAI, A. ET AL: "The induction and blocking of apoptosis by anti Apo2 monoclonal antibodies." FASEB JOURNAL, (MARCH 12, 1999) VOL. 13, NO. 4 PART 1, PP. A518. MEETING INFO.: ANNUAL MEETING OF THE PROFESSIONAL RESEARCH SCIENTISTS FOR EXPERIMENTAL BIOLOGY 99 WASHINGTON, D.C., USA APRIL 17-21, 1999 , XP000940459
- ASHKENAZI, AVI ET AL: "Apoptosis control by death and decoy receptors." CURRENT OPINION IN CELL BIOLOGY, (APRIL, 1999) VOL. 11, NO. 2, PP. 255-260. , XP000940576
- ASHKENAZI, AVI (1): "Apoptosis regulation by death and decoy receptors." FASEB JOURNAL, (APRIL 23, 1999) VOL. 13, NO. 7, PP. A1336. MEETING INFO.: ANNUAL MEETING OF THE AMERICAN SOCIETIES FOR EXPERIMENTAL BIOLOGY ON BIOCHEMISTRY AND MOLECULAR BIOLOGY 99 SAN FRANCISCO, CALIFORNIA, USA MAY 16-20, 1999 AMERICAN SOCIETIES FOR, XP000940460

### Sequence Listing

<110> Genentech, Inc.
<120> DR4 Antibodies and Uses Thereof
<130> P1245R1P2BPCT
   <141> 2000-05-25
<150> US 09/322,875
   <151> 1999-05-28
<160> 12
<210> 1
   <211> 468
   <212> PRT
   <213> Human
<400> 1
   Met Ala Pro Pro Pro Ala Arg Val His Leu Gly Ala Phe Leu Ala
   1               5                   10                  15
   Val Thr Pro Asn Pro Gly Ser Ala Ala Ser Gly Thr Glu Ala Ala
                   20                  25                  30
   Ala Ala Thr Pro Ser Lys Val Trp Gly Ser Ser Ala Gly Arg Ile
                   35                  40                  45
   Glu Pro Arg Gly Gly Gly Arg Gly Ala Leu Pro Thr Ser Met Gly
                   50                  55                  60
   Gln His Gly Pro Ser Ala Arg Ala Arg Ala Gly Arg Ala Pro Gly
                   65                  70                  75
   Pro Arg Pro Ala Arg Glu Ala Ser Pro Arg Leu Arg Val His Lys
                   80                  85                  90
   Thr Phe Lys Phe Val Val Val Gly Val Leu Leu Gln Val Val Pro
                   95                  100                 105
   Ser Ser Ala Ala Thr Ile Lys Leu His Asp Gln Ser Ile Gly Thr
                   110                 115                 120
   Gln Gln Trp Glu His Ser Pro Leu Gly Glu Leu Cys Pro Pro Gly
                   125                 130                 135
   Ser His Arg Ser Glu Arg Pro Gly Ala Cys Asn Arg Cys Thr Glu
                   140                 145                 150
   Gly Val Gly Tyr Thr Asn Ala Ser Asn Asn Leu Phe Ala Cys Leu
                   155                 160                 165
   Pro Cys Thr Ala Cys Lys Ser Asp Glu Glu Glu Arg Ser Pro Cys
                   170                 175                 180
   Thr Thr Thr Arg Asn Thr Ala Cys Gln Cys Lys Pro Gly Thr Phe
                   185                 190                 195
   Arg Asn Asp Asn Ser Ala Glu Met Cys Arg Lys Cys Ser Thr Gly
                   200                 205                 210
   Cys Pro Arg Gly Met Val Lys Val Lys Asp Cys Thr Pro Trp Ser
                   215                 220                 225
   Asp Ile Glu Cys Val His Lys Glu Ser Gly Asn Gly His Asn Ile
                   230                 235                 240
   Trp Val Ile Leu Val Val Thr Leu Val Val Pro Leu Leu Leu Val
                   245                 250                 255
   Ala Val Leu Ile Val Cys Cys Cys Ile Gly Ser Gly Cys Gly Gly
                   260                 265                 270
   Asp Pro Lys Cys Met Asp Arg Val Cys Phe Trp Arg Leu Gly Leu
                   275                 280                 285
   Leu Arg Gly Pro Gly Ala Glu Asp Asn Ala His Asn Glu Ile Leu
                   290                 295                 300
   Ser Asn Ala Asp Ser Leu Ser Thr Phe Val Ser Glu Gln Gln Met
                   305                 310                 315
   Glu Ser Gln Glu Pro Ala Asp Leu Thr Gly Val Thr Val Gln Ser
                   320                 325                 330
   Pro Gly Glu Ala Gln Cys Leu Leu Gly Pro Ala Glu Ala Glu Gly
                   335                 340                 345
   Ser Gln Arg Arg Arg Leu Leu Val Pro Ala Asn Gly Ala Asp Pro
                   350                 355                 360
   Thr Glu Thr Leu Met Leu Phe Phe Asp Lys Phe Ala Asn Ile Val
                   365                 370                 375
   Pro Phe Asp Ser Trp Asp Gln Leu Met Arg Gln Leu Asp Leu Thr
                   380                 385                 390
   Lys Asn Glu Ile Asp Val Val Arg Ala Gly Thr Ala Gly Pro Gly
                   395                 400                 405
   Asp Ala Leu Tyr Ala Met Leu Met Lys Trp Val Asn Lys Thr Gly
                   410                 415                 420
   Arg Asn Ala Ser Ile His Thr Leu Leu Asp Ala Leu Glu Arg Met
                   425                 430                 435
   Glu Glu Arg His Ala Lys Glu Lys Ile Gln Asp Leu Leu Val Asp
                   440                 445                 450
   Ser Gly Lys Phe Ile Tyr Leu Glu Asp Gly Thr Gly Ser Ala Val
                   455                 460                 465
   Ser Leu Glu
<210> 2
   <211> 1407
   <212> DNA
   <213> Human
<400> 2
   atggcgccac caccagctag agtacatcta ggtgcgttcc tggcagtgac 50
   tccgaatccc gggagcgcag cgagtgggac agaggcagcc gcggccacac 100
   ccagcaaagt gtggggctct tccgcgggga ggattgaacc acgaggcggg 150
   ggccgaggag cgctccctac ctccatggga cagcacggac ccagtgcccg 200
   ggcccgggca gggcgcgccc caggacccag gccggcgcgg gaagccagcc 250
   ctcggctccg ggtccacaag accttcaagt ttgtcgtcgt cggggtcctg 300
   ctgcaggtcg tacctagctc agctgcaacc atgatcaatc aattggcaca 350
   aattggcaca cagcaatggg aacatagccc tttgggagag ttgtgtccac 400
   caggatctca tagatcagaa cgtcctggag cctgtaaccg gtgcacagag 450
   ggtgtgggtt acaccaatgc ttccaacaat ttgtttgctt gcctcccatg 500
   tacagcttgt aaatcagatg aagaagagag aagtccctgc accacgacca 550
   ggaacacagc atgtcagtgc aaaccaggaa ctttccggaa tgacaattct 600
   gctgagatgt gccggaagtg cagcacaggg tgccccagag ggatggtcaa 650
   ggtcaaggat tgtacgccct ggagtgacat cgagtgtgtc cacaaagaat 700
   caggcaatgg acataatata tgggtgattt tggttgtgac tttggttgtt 750
   ccgttgctgt tggtggctgt gctgattgtc tgttgttgca tcggctcagg 800
   ttgtggaggg gaccccaagt gcatggacag ggtgtgtttc tggcgcttgg 850
   gtctcctacg agggcctggg gctgaggaca atgctcacaa cgagattctg 900
   agcaacgcag actcgctgtc cactttcgtc tctgagcagc aaatggaaag 950
   ccaggagccg gcagatttga caggtgtcac tgtacagtcc ccaggggagg 1000
   cacagtgtct gctgggaccg gcagaagctg aagggtctca gaggaggagg 1050
   ctgctggttc cagcaaatgg tgctgacccc actgagactc tgatgctgtt 1100
   ctttgacaag tttgcaaaca tcgtgccctt tgactcctgg gaccagctca 1150
   tgaggcagct ggacctcacg aaaaatgaga tcgatgtggt cagagctggt 1200
   acagcaggcc caggggatgc cttgtatgca atgctgatga aatgggtcaa 1250
   caaaactgga cggaacgcct cgatccacac cctgctggat gccttggaga 1300
   ggatggaaga gagacatgca aaagagaaga ttcaggacct cttggtggac 1350
   tctggaaagt tcatctactt agaagatggc acaggctctg ccgtgtcctt 1400
   ggagtga 1407
<210> 3
   <211> 36
   <212> DNA
   <213> Artificial
<220>
   <221> Artificial Sequence
   <222> 2,16,17,19,21,22,27,28,31,32,34,35
   <223> w=a or t; k=g or t; b=g or t or c; y=c or t; r=a or g; s=g or c <400> 3
   tgcagccacg gwccgwktba kytccarytt kgtssc 36
<210> 4
   <211> 39
   <212> DNA
   <213> Artificial
<220>
   <221> Artificial Sequence
   <222> 27,28,31,34,39
   <223> m=a or c; r=a or g; n=a or g or t or c; s=g or c
<400> 4
   gaccgatggg cccgtcgttt tggctgmrga racngtgas 39
<210> 5
   <211> 36
   <212> DNA
   <213> Artificial
<220>
   <221> Artificial Sequence
   <222> 1-36
   <223> Sequence is synthesized.
<400> 5
   gctacaaatg catacgctga tatccagatg acacag 36
<210> 6
   <211> 36
   <212> DNA
   <213> Artificial
<220>
   <221> Artificial Sequence
   <222> 1-36
   <223> Sequence is synthesized.
<400> 6
   gctacaaacg cgtacgctca ggtgcagctg aaggag 36
<210> 7
   <211> 702
   <212> DNA
   <213> Artificial
<220>
   <221> Artificial Sequence
   <222> 1-57
   <223> Nucleotides 1-57 is vector-derived signal sequence
<220>
   <221> Artificial Sequence
   <222> 58-435
   <223> Nucleotides 58-435 are of murine origin
<220>
   <221> Artificial Sequence
   <222> 436-702
   <223> Nucleotides 436-702 are of human origin
<400> 7
   atgggatggt catgtatcat cctttttcta gtagcaactg caactggagt 50
   acattcagat atccagatga cacagactac atcctccctg tctgcctctc 100
   tgggagacag agtcaccatc agttgcaggg caagtcagga cattagcaat 150
   tatttaaact ggtatcagcg gaaaccagat ggaactgtta aactcctgat 200
   ctactacaca tcacgattac actcaggagt cccatcacgg ttcagtggca 250
   gtgggtctgg aacagattat tctctcacca ttagcaacct ggaacaagaa 300
   gatattgcca cttacttttg ccaacagggt aatacgcttc cattcacgtt 350
   cggctcggcc accaagctgg aactaactcg gaccgtggct gcaccatctg 400
   tcttcatctt cccgccatct gatgagcagt tgaaatctgg aactgcctct 450
   gttgtgtgcc tgctgaataa cttctatccc agagaggcca aagtacagtg 500
   gaaggtggat aacgccctcc aatcgggtaa ctcccaggag agtgtcacag 550
   agcaggacag caaggacagc acctacagcc tcagcagcac cctgacgctg 600
   agcaaagcag actacgagaa acacaaagtc tacgcctgcg aagtcaccca 650
   tcagggcctg agctcgcccg tcacaaagag cttcaacagg ggagagtgtt 700
   aa 702
<210> 8
   <211> 702
   <212> DNA
   <213> Artificial
<220>
   <221> Artificial Sequence
   <222> 1-702
   <223> Sequence is synthesized.
<400> 8
   ttaacactct cccctgttga agctctttgt gacgggcgag ctcaggccct 50
   gatgggtgac ttcgcaggcg tagactttgt gtttctcgta gtctgctttg 100
   ctcagcgtca gggtgctgct gaggctgtag gtgctgtcct tgctgtcctg 150
   ctctgtgaca ctctcctggg agttacccga ttggagggcg ttatccacct 200
   tccactgtac tttggcctct ctgggataga agttattcag caggcacaca 250
   acagaggcag ttccagattt caactgctca tcagatggcg ggaagatgaa 300
   gacagatggt gcagccacgg tccgagttag ttccagcttg gtggccgagc 350
   cgaacgtgaa tggaagcgta ttaccctgtt ggcaaaagta agtggcaata 400
   tcttcttgtt ccaggttgct aatggtgaga gaataatctg ttccagaccc 450
   actgccactg aaccgtgatg ggactcctga gtgtaatcgt gatgtgtagt 500
   agatcaggag tttaacagtt ccatctggtt tccgctgata ccagtttaaa 550
   taattgctaa tgtcctgact tgccctgcaa ctgatggtga ctctgtctcc 600
   cagagaggca gacagggagg atgtagtctg tgtcatctgg atatctgaat 650
   gtactccagt tgcagttgct actagaaaaa ggatgataca tgaccatccc 700
   at 702
<210> 9
   <211> 233
   <212> PRT
   <213> Artificial
<220>
   <221> Artificial Sequence
   <222> 1-19
   <223> Amino acids is vector-derived signal sequence
<220>
   <221> Artificial Sequence
   <222> 20-126
   <223> Amino acids 20-126 are of murine origin
<220>
   <221> Artificial Sequence
   <222> 127-233
   <223> Amino acids 127-233 are of human origin
<400> 9
   Met Gly Trp Ser Cys Ile Ile Leu Phe Leu Val Ala Thr Ala Thr
   1               5                   10                  15
   Gly Val His Ser Asp Ile Gln Met Thr Gln Thr Thr Ser Ser Leu
                   20                  25                  30
   Ser Ala Ser Leu Gly Asp Arg Val Thr Ile Ser Cys Arg Ala Ser
                   35                  40                  45
   Gln Asp Ile Ser Asn Tyr Leu Asn Trp Tyr Gln Arg Lys Pro Asp
                   50                  55                  60
   Gly Thr Val Lys Leu Leu Ile Tyr Tyr Thr Ser Arg Leu His Ser
                   65                  70                  75
   Gly Val Pro Ser Arg Phe Ser Gly Ser Gly Ser Gly Thr Asp Tyr
                   80                  85                  90
   Ser Leu Thr Ile Ser Asn Leu Glu Gln Glu Asp Ile Ala Thr Tyr
                   95                  100                 105
   Phe Cys Gln Gln Gly Asn Thr Leu Pro Phe Thr Phe Gly Ser Ala
                   110                 115                 120
   Thr Lys Leu Glu Leu Thr Arg Thr Val Ala Ala Pro Ser Val Phe
                   125                 130                 135
   Ile Phe Pro Pro Ser Asp Glu Gln Leu Lys Ser Gly Thr Ala Ser
                   140                 145                 150
   Val Val Cys Leu Leu Asn Asn Phe Tyr Pro Arg Glu Ala Lys Val
                   155                 160                 165
   Gln Trp Lys Val Asp Asn Ala Leu Gln Ser Gly Asn Ser Gln Glu
                   170                 175                 180
   Ser Val Thr Glu Gln Asp Ser Lys Asp Ser Thr Tyr Ser Leu Ser
                   185                 190                 195
   Ser Thr Leu Thr Leu Ser Lys Ala Asp Tyr Glu Lys His Lys Val
                   200                 205                 210
   Tyr Ala Cys Glu Val Thr His Gln Gly Leu Ser Ser Pro Val Thr
                   215                 220                 225
   Lys Ser Phe Asn Arg Gly Glu Cys
                   230
<210> 10
   <211> 1431
   <212> DNA
   <213> Artificial
<220>
   <221> Artificial sequence
   <222> 58,60,63
   <223> s=g or c; r=a or g; k=g or t
<400> 10
   atgggatggt catgtatcat cctttttcta gtagcaactg caactggagt 50
   acattcasar gtkcagctga aggagtcagg acctggcctg gtggcgccct 100
   cacagagcct gtccatcact tgcactgtct ctgggttttc attaaccagc 150
   tatggtgtac actgggttcg ccagcctcca ggaaagggtc tggagtggct 200
   gggagtaata tgggctgttg gaagcacaaa ttataattcg gctctcatgt 250
   ccagactgag catcagcaaa gacaactcca agagccaagt tttcttaaaa 300
   atgaacagtc tgcaaactga tgacacagcc atgtactact gtgccagaga 350
   gggggaattc gattactacg gtagtagtct cctatcttac cattctatga 400
   acttctgggg tcaaggaacc tcagtcaccg tctcctcagc caaaacgacg 450
   ggcccatcgg tcttccccct ggcaccctcc tccaagagca cctctggggg 500
   cacagcggcc ctgggctgcc tggtcaagga ctacttcccc gaaccggtga 550
   cggtgtcgtg gaactcaggc gccctgacca gcggcgtgca caccttcccg 600
   gctgtcctac agtcctcagg actctactcc ctcagcagcg tggtgactgt 650
   gccctctagc agcttgggca cccagaccta catctgcaac gtgaatcaca 700
   agcccagcaa caccaaggtg gacaagaaag ttgagcccaa atcttgtgac 750
   aaaactcaca catgcccacc gtgcccagca cctgaactcc tggggggacc 800
   gtcagtcttc ctcttccccc caaaacccaa ggacaccctc atgatctccc 850
   ggacccctga ggtcacatgc gtggtggtgg acgtgagcca cgaagaccct 900
   gaggtcaagt tcaactggta cgtggacggc gtggaggtgc ataatgccaa 950
   gacaaagccg cgggaggagc agtacaacag cacgtaccgg gtggtcagcg 1000
   tcctcaccgt cctgcaccag gactggctga atggcaagga gtacaagtgc 1050
   aaggtctcca acaaagccct cccagccccc atcgagaaaa ccatctccaa 1100
   agccaaaggg cagccccgag aaccacaggt gtacaccctg cccccatccc 1150
   gggaagagat gaccaagaac caggtcagcc tgacctgcct ggtcaaaggc 1200
   ttctatccca gcgacatcgc cgtggagtgg gagagcaatg ggcagccgga 1250
   gaacaactac aagaccacgc ctcccgtgct ggactccgac ggctccttct 1300
   tcctctacag caagctcacc gtggacaaga gcaggtggca gcaggggaac 1350
   gtcttctcat gctccgtgat gcatgaggct ctgcacaacc actacacgca 1400
   gaagagcctc tccctgtctc cgggtaaatg a 1431
<210> 11
   <211> 1431
   <212> DNA
   <213> Artificial
<220>
   <221> Artificial sequence
   <222> 1369, 1372, 1374
   <223> s=g or c; y=c or t; m=a or c
<400> 11
   tcatttaccc ggagacaggg agaggctctt ctgcgtgtag tggttgtgca 50
   gagcctcatg catcacggag catgagaaga cgttcccctg ctgccacctg 100
   ctcttgtcca cggtgagctt gctgtagagg aagaaggagc cgtcggagtc 150
   cagcacggga ggcgtggtct tgtagttgtt ctccggctgc ccattgctct 200
   cccactccac ggcgatgtcg ctgggataga agcctttgac caggcaggtc 250
   aggctgacct ggttcttggt catctcttcc cgggatgggg gcagggtgta 300
   cacctgtggt tctcggggct gccctttggc tttggagatg gttttctcga 350
   tgggggctgg gagggctttg ttggagacct tgcacttgta ctccttgcca 400
   ttcagccagt cctggtgcag gacggtgagg acgctgacca cccggtacgt 450
   gctgttgtac tgctcctccc gcggctttgt cttggcatta tgcacctcca 500
   cgccgtccac gtaccagttg aacttgacct cagggtcttc gtggctcacg 550
   tccaccacca cgcatgtgac ctcaggggtc cgggagatca tgagggtgtc 600
   cttgggtttt ggggggaaga ggaagactga cggtcccccc aggagttcag 650
   gtgctgggca cggtgggcat gtgtgagttt tgtcacaaga tttgggctca 700
   actttcttgt ccaccttggt gttgctgggc ttgtgattca cgttgcagat 750
   gtaggtctgg gtgcccaagc tgctagaggg cacagtcacc acgctgctga 800
   gggagtagag tcctgaggac tgtaggacag ccgggaaggt gtgcacgccg 850
   ctggtcaggg cgcctgagtt ccacgacacc gtcaccggtt cggggaagta 900
   gtccttgacc aggcagccca gggccgctgt gcccccagag gtgctcttgg 950
   aggagggtgc cagggggaag accgatgggc ccgtcgtttt ggctgaggag 1000
   acggtgactg aggttccttg accccagaag ttcatagaat ggtaagatag 1050
   gagactacta ccgtagtaat cgaattcccc ctctctggca cagtagtaca 1100
   tggctgtgtc atcagtttgc agactgttca tttttaagaa aacttggctc 1150
   ttggagttgt ctttgctgat gctcagtctg gacatgagag ccgaattata 1200
   atttgtgctt ccaacagccc atattactcc cagccactcc agaccctttc 1250
   ctggaggctg gcgaacccag tgtacaccat agctggttaa tgaaaaccca 1300
   gagacagtgc aagtgatgga caggctctgt gagggcgcca ccaggccagg 1350
   tcctgactcc ttcagctgma cytstgaatg tactccagtt gcagttgcta 1400
   ctagaaaaag gatgatacat gaccatccca t 1431
<210> 12
   <211> 476
   <212> PRT
   <213> Artificial
<220>
   <221> Artificial sequence
   <222> 1-19
   <223> amino acids 1-19 is vector-derived signal sequence
<220>
   <221> Artificial Sequence
   <222> 20-145
   <223> amino acids 20-145 are of murine origin
<220>
   <221> Artificial Sequence
   <222> 20
   <223> xaa may be glutamine or glutamic acid
<220>
   <221> Artificial sequence
   <222> 146-476
   <223> amino acids 146-476 are of human origin
<400> 12
   Met Gly Trp Ser Cys Ile Ile Leu Phe Leu Val Ala Thr Ala Thr
   1               5                   10                  15
   Gly Val His Ser Xaa Val Gln Leu Lys Glu Ser Gly Pro Gly Leu
                   20                  25                  30
   Val Ala Pro Ser Gln Ser Leu Ser Ile Thr Cys Thr Val Ser Gly
                   35                  40                  45
   Phe Ser Leu Thr Ser Tyr Gly Val His Trp Val Arg Gln Pro Pro
                   50                  55                  60
   Gly Lys Gly Leu Glu Trp Leu Gly Val Ile Trp Ala Val Gly Ser
                   65                  70                  75
   Thr Asn Tyr Asn Ser Ala Leu Met Ser Arg Leu Ser Ile Ser Lys
                   80                  85                  90
   Asp Asn Ser Lys Ser Gln Val Phe Leu Lys Met Asn Ser Leu Gln
                   95                  100                 105
   Thr Asp Asp Thr Ala Met Tyr Tyr Cys Ala Arg Glu Gly Glu Phe
                   110                 115                 120
   Asp Tyr Tyr Gly Ser Ser Leu Leu Ser Tyr His Ser Met Asn Phe
                   125                 130                 135
   Trp Gly Gln Gly Thr Ser Val Thr Val Ser Ser Ala Lys Thr Thr
                   140                 145                 150
   Gly Pro Ser Val Phe Pro Leu Ala Pro Ser Ser Lys Ser Thr Ser
                   155                 160                 165
   Gly Gly Thr Ala Ala Leu Gly Cys Leu Val Lys Asp Tyr Phe Pro
                   170                 175                 180
   Glu Pro Val Thr Val Ser Trp Asn Ser Gly Ala Leu Thr Ser Gly
                   185                 190                 195
   Val His Thr Phe Pro Ala Val Leu Gln Ser Ser Gly Leu Tyr Ser
                   200                 205                 210
   Leu Ser Ser Val Val Thr Val Pro Ser Ser Ser Leu Gly Thr Gln
                   215                 220                 225
   Thr Tyr Ile Cys Asn Val Asn His Lys Pro Ser Asn Thr Lys Val
                   230                 235                 240
   Asp Lys Lys Val Glu Pro Lys Ser Cys Asp Lys Thr His Thr Cys
                   245                 250                 255
   Pro Pro Cys Pro Ala Pro Glu Leu Leu Gly Gly Pro Ser Val Phe
                   260                 265                 270
   Leu Phe Pro Pro Lys Pro Lys Asp Thr Leu Met Ile Ser Arg Thr
                   275                 280                 285
   Pro Glu Val Thr Cys Val Val Val Asp Val Ser His Glu Asp Pro
                   290                 295                 300
   Glu Val Lys Phe Asn Trp Tyr Val Asp Gly Val Glu Val His Asn
                   305                 310                 315
   Ala Lys Thr Lys Pro Arg Glu Glu Gln Tyr Asn Ser Thr Tyr Arg
                   320                 325                 330
   Val Val Ser Val Leu Thr Val Leu His Gln Asp Trp Leu Asn Gly
                   335                 340                 345
   Lys Glu Tyr Lys Cys Lys Val Ser Asn Lys Ala Leu Pro Ala Pro
                   350                 355                 360
   Ile Glu Lys Thr Ile Ser Lys Ala Lys Gly Gln Pro Arg Glu Pro
                   365                 370                 375
   Gln Val Tyr Thr Leu Pro Pro Ser Arg Glu Glu Met Thr Lys Asn
                   380                 385                 390
   Gln Val Ser Leu Thr Cys Leu Val Lys Gly Phe Tyr Pro Ser Asp
                   395                 400                 405
   Ile Ala Val Glu Trp Glu Ser Asn Gly Gln Pro Glu Asn Asn Tyr
                   410                 415                 420
   Lys Thr Thr Pro Pro Val Leu Asp Ser Asp Gly Ser Phe Phe Leu
                   425                 430                 435
   Tyr Ser Lys Leu Thr Val Asp Lys Ser Arg Trp Gln Gln Gly Asn
                   440                 445                 450
   Val Phe Ser Cys Ser Val Met His Glu Ala Leu His Asn His Tyr
                   455                 460                 465
   Thr Gln Lys Ser Leu Ser Leu Ser Pro Gly Lys
                   470                 475

## Description

### FIELD OF THE INVENTION

The present invention relates generally to DR4 antibodies, as defined in the claims

### BACKGROUND OF THE INTENTION

Control of cell numbers in mammals is believed to be determined, in part, by a balance between cell proliferation and cell death. One form of cell death, sometimes referred to as necrotic cell death, is typically characterized as a pathologic form of cell death resulting from some trauma or cellular injury. In contrast, there is another, "physiologic" form of cell death which usually proceeds in an orderly or controlled manner. This orderly or controlled form of cell death is often referred to as "apoptosis" [see, e.g., Barr et al., Bio/Technology, 12:487-493 (1994); Steller et al., Science, 267:1445-1449 (1995)]. Apoptotic cell death naturally occurs in many physiological processes, including embryonic development and clonal selection in the immune system [Itoh et al., Cell, 66:233-243 (1991)]. Decreased levels of apoptotic cell death have been associated with a variety of pathological conditions, including cancer, lupus, and herpes virus infection [Thompson, Science, 267:1456-1462 (1995)]. Increased levels of apoptotic cell death may be associated with a variety of other pathological conditions, including AIDS, Alzheimer's disease, Parkinson's disease, amyotrophic lateral sclerosis, multiple sclerosis, retinitis pigmentosa, cerebellar degeneration, aplastic anemia, myocardial infarction, stroke, reperfusion injury, and toxin-induced liver disease [see, Thompson, supra].

Apoptotic cell death is typically accompanied by one or more characteristic morphological and biochemical changes in cells, such as condensation of cytoplasm, loss of plasma membrane microvilli, segmentation of the nucleus, degradation of chromosomal DNA or loss of mitochondrial function. A variety of extrinsic and intrinsic signals are believed to trigger or induce such morphological and biochemical cellular changes [Raff, Nature, 356:397-400 (1992); Steller, supra; Sachs et al., Blood, 82:15 (1993)]. For instance, they can be triggered by hormonal stimuli, such as glucocorticoid hormones for immature thymocytes, as well as withdrawal of certain growth factors [Watanabe-Fukunaga et al., Nature, 356:314-317 (1992)]. Also, some identified oncogenes such as *myc, rel,* and *E1A*, and tumor suppressors, like p53, have been reported to have a role in inducing apoptosis. Certain chemotherapy drugs and some forms of radiation have likewise been observed to have apoptosis-inducing activity [Thompson, supra].

Various molecules, such as tumor necrosis factor-α ("TNF-α"), tumor necrosis factor-β ("TNF-β" or "lymphotoxin-α"), lymphotoxin-β ("LT-β"), CD30 ligand, CD27 ligand, CD40 ligand, OX-40 ligand, 4-1BB ligand, Apo-1 ligand (also referred to as Fas ligand or CD95 ligand), and Apo-2 ligand (also referred to as TRAIL) have been identified as members of the tumor necrosis factor ("TNF") family of cytokines [See, e.g., Gruss and Dower, Blood, 85:3378-3404 (1995); WO 97/25428 published July 17, 1997; WO 97/01633 published January 16, 1997; Pitti et al., J. Biol. Chem., 271:12687-12690 (1996); Wiley et al., Immunity, 3:673-682 (1995); Browning et al., Cell, 72:847-856 (1993); Armitage et al. Nature, 357:80-82 (1992)]. Among these molecules, TNF-α, TNF-β, CD30 ligand, 4-1BB ligand, Apo-1 ligand, and Apo-2 ligand (TRAIL) have been reported to be involved in apoptotic cell death. Both TNF-α and TNF-β have been reported to induce apoptotic death in susceptible tumor cells [Schmid et al., Proc. Natl. Acad. Sci., 83:1881 (1986); Dealtry et al., Eur. J. Immunol., 17:689 (1987)]. Zheng et al. have reported that TNF-α is involved in post-stimulation apoptosis of CD8-positive T cells [Zheng et al., Nature, 377:348-351 (1995)]. Other investigators have reported that CD30 ligand may be involved in deletion of self-reactive T cells in the thymus [Amakawa et al., Cold Spring Harbor Laboratory Symposium on Programmed Cell Death, Abstr. No. 10, (1995)].

Mutations in the mouse Fas/Apo-1 receptor or ligand genes (called *1pr* and *gld*, respectively) have been associated with some autoimmune disorders, indicating that Apo-1 ligand may play a role in regulating the clonal deletion of self-reactive lymphocytes in the periphery [Krammer et al., Curr. Op. Immunol., 6:279-289 (1994); Nagata et al., Science, 267:1449-1456 (1995)]. Apo-1 ligand is also reported to induce post-stimulation apoptosis in CD4-positive T lymphocytes and in B lymphocytes, and may be involved in the elimination of activated lymphocytes when their function is no longer needed [Krammer et al., supra; Nagata et al., supra]. Agonist mouse monoclonal antibodies specifically binding to the Apo-1 receptor have been reported to exhibit cell killing activity that is comparable to or similar to that of TNF-α [Yonehara et al., J. Exp. Med., 169:1747-1756 (1989)].

Induction of various cellular responses mediated by such TNF family cytokines is believed to be initiated by their binding to specific cell receptors. Two distinct TNF receptors of approximately 55-kDa (TNFR1) and 75-kDa (TNFR2) have been identified [Hohmann et al., J. Biol. Chem., 264:14927-14934 (1989); Brockhaus et al., Proc. Natl. Acad. Sci., 87:3127-3131 (1990); EP 417,563, published March 20, 1991] and human and mouse cDNAs corresponding to both receptor types have been isolated and characterized [Loetscher et al., Cell, 61:351 (1990); Schall et al., Cell, 61:361 (1990); Smith et al., Science, 248:1019-1023 (1990); Lewis et al., Proc. Natl. Acad. Sci., 88:2830-2834 (1991); Goodwin et al., Mol. Cell. Biol., 11:3020-3026 (1991)]. Extensive polymorphisms have been associated with both TNF receptor genes [see, e.g., Takao et al., Immunogenetics, 37:199-203 (1993)]. Both TNFRs share the typical structure of cell surface receptors including extracellular, transmembrane and intracellular regions. The extracellular portions of both receptors are found naturally also as soluble TNF-binding proteins [Nophar, Y. et al., EMBO J., 9:3269 (1990); and Kohno, T. et al., Proc. Natl. Acad. Sci. U.S.A., 87:8331 (1990)]. More recently, the cloning of recombinant soluble TNF receptors was reported by Hale et al. [J. Cell. Biochem. Supplement 15F, 1991, p. 113 (P424)].

The extracellular portion of type 1 and type 2 TNFRs (TNFR1 and TNFR2) contains a repetitive amino acid sequence pattern of four cysteine-rich domains (CRDs) designated 1 through 4, starting from the NH₂-terminus. Each CRD is about 40 amino acids long and contains 4 to 6 cysteine residues at positions which are well conserved [Schall et al., supra; Loetscher et al., supra; Smith et al., supra; Nophar et al., supra; Kohno et al., supra]. In TNFR1, the approximate boundaries of the four CRDs are as follows: CRD1- amino acids 14 to about 53; CRD2- amino acids from about 54 to about 97; CRD3- amino acids from about 98 to about 138; CRD4- amino acids from about 139 to about 167. In TNFR2, CRD1 includes amino acids 17 to about 54; CRD2- amino acids from about 55 to about 97; CRD3- amino acids from about 98 to about 140; and CRD4- amino acids from about 141 to about 179 [Banner et al., Cell, 73:431-435 (1993)]. The potential role of the CRDs in ligand binding is also described by Banner et al., supra.

A similar repetitive pattern of CRDs exists in several other cell-surface proteins, including the p75 nerve growth factor receptor (NGFR) [Johnson et al., Cell, 47:545 (1986); Radeke et al., Nature, 325:593 (1987)], the B cell antigen CD40 [Stamenkovic et al., EMBO J., 8:1403 (1989)], the T cell antigen OX40 [Mallett et al., EMBO J., 9:1063 (1990)] and the Fas antigen [Yonehara et al., supra and Itoh et al., Cell, 66:233-243 (1991)]. CRDs are also found in the soluble TNFR (sTNFR)-like T2 proteins of the Shope and myxoma poxviruses [Upton et al., Virology, 160:20-29 (1987); Smith et al., Biochem. Biophys. Res. Commun., 176:335 (1991); Upton et al., Virology, 184:370 (1991)]. Optimal alignment of these sequences indicates that the positions of the cysteine residues are well conserved. These receptors are sometimes collectively referred to as members of the TNF/NGF receptor superfamily. Recent studies on p75NGFR showed that the deletion of CRD1 [Welcher, A.A. et al., Proc. Natl. Acad. Sci. USA, 88:159-163 (1991)] or a 5-amino acid insertion in this domain [Yan, H. and Chao, M.V., J. Biol. Chem., 266:12099-12104 (1991)] had little or no effect on NGF binding (Yan, H. and Chao, M.V., supra]. p75 NGFR contains a proline-rich stretch of about 60 amino acids, between its CRD4 and transmembrane region, which is not involved in NGF binding [Peetre, C. et al., Eur. J. Hematol., 41:414-419 (1988); Seckinger, P. et al., J. Biol. Chem., 264:11966-11973 (1989); Yan, H. and Chao, M.V., supra]. A similar proline-rich region is found in TNFR2 but not in TNFR1.

Itoh et al. disclose that the Apo-1 receptor can signal an apoptotic cell death similar to that signaled by the 55-kDa TNFR1 [Itoh et al., supra]. Expression of the Apo-1 antigen has also been reported to be down-regulated along with that of TNFR1 when cells are treated with either TNF-α or anti-Apo-1 mouse monoclonal antibody [Krammer et al., supra; Nagata et al., supra]. Accordingly, some investigators have hypothesized that cell lines that co-express both Apo-1 and TNFR1 receptors may mediate cell killing through common signaling pathways [Id.].

The TNF family ligands identified to date, with the exception of lymphotoxin-α, are typically type II transmembrane proteins, whose C-terminus is extracellular. In contrast, most receptors in the TNF receptor (TNFR) family identified to date are typically type I transmembrane proteins. In both the TNF ligand and receptor families, however, homology identified between family members has been found mainly in the extracellular domain ("ECD"). Several of the TNF family cytokines, including TNF-α, Apo-1 ligand and CD40 ligand, are cleaved proteolytically at the cell surface; the resulting protein in each case typically forms a homotrimeric molecule that functions as a soluble cytokine. TNF receptor family proteins are also usually cleaved proteolytically to release soluble receptor ECDs that can function as inhibitors of the cognate cytokines.

Recently, other members of the TNFR family have been identified. Such newly identified members of the TNFR family include CAR1, HVEM and osteoprotegerin (OPG) [Brojatsch et al., Cell, 87:845-855 (1996); Montgomery et al., Cell, 87:427-436 (1996); Marsters et al., J. Biol. Chem., 272:14029-14032 (1997); Simonet et al., Cell, 89:309-319 (1997)]. Unlike other known TNFR-like molecules, Simonet et al., supra, report that OPG contains no hydrophobic transmembrane-spanning sequence.

In Marsters et al., Curr. Biol., 6:750 (1996), investigators describe a full length native sequence human polypeptide, called Apo-3, which exhibits similarity to the TNFR family in its extracellular cysteine-rich repeats and resembles TNFR1 and CD95 in that it contains a cytoplasmic death domain sequence [see also Marsters et al., Curr. Biol., 6:1669 (1996)]. Apo-3 has also been referred to by other investigators as DR3, wsl-1 and TRAMP [Chinnaiyan et al., Science, 274:990 (1996); Kitson et al., Nature, 384:372 (1996); Bodmer et al., Immunity, 6:79 (1997)].

Pan et al. have disclosed another TNF receptor family member referred to as "DR4" [Pan et al., Science, 276:111-113 (1997)]. The DR4 cDNA encodes an open reading frame of 468 amino acids with features characteristic of a cell surface receptor. Pan et al. describe a putative signal peptide present at the beginning of the molecule (amino acids -23 to -1), with the mature protein predicted to start at amino acid 24 (Ala). Residues 108 to 206 contain two cysteine-rich pseudorepeats that resemble corresponding regions in TNFR-1 (four repeats), DR3 (four repeats), Fas (three repeats) and CAR1 (two repeats). Following the transmembrane domain is an intracellular region containing a 70 amino acid stretch with similarity to the death domains of TNFR1, DR3, Fas, and CAR1. The DR4 transcript was detected in spleen, peripheral blood leukocytes, small intestine, and thymus. In addition, DR4 expression was also found in K562 erythroleukemia cells, MCF7 breast carcinoma cells and activated T cells. Pan et al. further disclose that DR4 is believed to be a receptor for the ligand known as Apo-2 ligand or TRAIL.

In Sheridan et al., Science, 277:818-821 (1997) and Pan et al., Science, 277:815-818 (1997), another molecule believed to be a receptor for the Apo-2 ligand (TRAIL) is described. That molecule is referred to as Apo-2 (it has also been alternatively referred to as DR5). [*see* also, WO98/51793 published November 19, 1998; WO98/41629 published September 24, 1998]. That molecule has further been referred to as TRAIL-R, TR6, Tango-63, hAPO8, TRICK2 or KILLER [Screaton et al., Curr. Biol., 7:693-696 (1997); Walczak et al., EMBO J., 16:5386-5387 (1997); Wu et al., Nature Genetics, 17:141-143 (1997); WO98/35986 published August 20, 1998; EP870,827 published October 14, 1998; WO98/46643 published October 22, 1998; WO99/02653 published January 21, 1999; WO99/09165 published February 25, 1999; WO99/11791 published March 11, 1999]. Like DR4, DR5 is reported to contain a cytoplasmic death domain and be capable of signaling apoptosis. The crystal structure of the complex formed between Apo-2L/TRAIL and DR5 is described in Hymowitz et al., Molecular Cell, 4:563-571 (1999).

In Sheridan et al., supra, a receptor called DcR1 (or alternatively, Apo-2DcR) is disclosed as being a potential decoy receptor for Apo-2 ligand (TRAIL). Sheridan et al. report that DcR1 can inhibit Apo-2 ligand function in vitro. See also, Pan et al., supra, for disclosure on the same decoy receptor, referred to as TRID. DCR1 has also been referred to as LIT or TRAIL-R3 [McFarlane et al., J. Biol. Chem., 272:25417-25420 (1997); Schneider et al., FEBS Letters, 416:329-334 (1997); Degli-Esposti et al., J. Exp. Med., 186:1165-1170 (1997); and Mongkolsapaya et al., J. Immunol., 160:3-6 (1998)].

In Marsters et al., Curr. Biol., 7:1003-1006 (1997), a receptor referred to as DcR2 is disclosed. Marsters et al. report that DcR2 contains a cytoplasmic region with a truncated death domain and can function as an inhibitory Apo-2L receptor *in vitro.* DCR2 is also called TRUNDD or TRAIL-R4 [Pan et al., FEBS Letters, 424:41-45 (1998); Degli-Esposti et al., Immunity, 7:813-820 (1997)].

For a review of the TNF family of cytokines and their receptors, see Gruss and Dower, supra.

As presently understood, the cell death program contains at least three important elements - activators, inhibitors, and effectors; in *C*. *elegans,* these elements are encoded respectively by three genes, *Ced-4, Ced-9* and *Ced-3* [Steller, Science, 267:1445 (1995); Chinnaiyan et al., Science, 275:1122-1126 (1997); Zou et al., Cell, 90:405-413 (1997)]. Two of the TNFR family members, TNFR1 and Fas/Apol (CD95), can activate apoptotic cell death [Chinnaiyan and Dixit, Current Biology, 6:555-562 (1996); Fraser and Evan, Cell; 85:781-784 (1996)]. TNFR1 is also known to mediate activation of the transcription factor, NF-kB [Tartaglia et al., Cell, 74:845-853 (1993); Hsu et al., Cell, 84:299-308 (1996)]. In addition to some ECD homology, these two receptors share homology in their intracellular domain (ICD) in an oligomerization interface known as the death domain [Tartaglia et al., supra; Nagata, Cell, 88:355 (1997)]. Death domains are also found in several metazoan proteins that regulate apoptosis, namely, the Drosophila protein, Reaper, and the mammalian proteins referred to as FADD/MORT1, TRADD, and RIP [Cleveland and Ihle, Cell, 81:479-482 (1995)]. Upon ligand binding and receptor clustering, TNFR1 and CD95 are believed to recruit FADD into a death-inducing signaling complex. CD95 purportedly binds FADD directly, while TNFR1 binds FADD indirectly via TRADD [Chinnaiyan et al., Cell, 81:505-512 (1995); Boldin et al., J. Biol. Chem., 270:387-391 (1995); Hsu et al., supra; Chinnaiyan et al., J. Biol. Chem., 271:4961-4965 (1996)]. It has been reported that FADD serves as an adaptor protein which recruits the *Ced-3*-related protease, MACHα/FLICE (caspase 8), into the death signaling complex [Boldin et al., Cell, 85:803-815 (1996); Muzio et al., Cell, 85:817-827 (1996)]. MACHα/FLICE appears to be the trigger that sets off a cascade of apoptotic proteases, including the interleukin-1β converting enzyme (ICE) and CPP32/Yama, which may execute some critical aspects of the cell death program [Fraser and Evan, supra].

It was recently disclosed that programmed cell death involves the activity of members of a family of cysteine proteases related to the *C*. *elegans* cell death gene, *ced-3*, and to the mammalian IL-1-converting enzyme, ICE. The activity of the ICE and CPP32/Yama proteases can be inhibited by the product of the cowpox virus gene, *crmA* [Ray et al., Cell, 69:597-604 (1992); Tewari et al., Cell, 81:801-809 (1995)]. Recent studies show that CrmA can inhibit TNFR1- and CD95-induced cell death [Enari et al., Nature, 375:78-81 (1995); Tewari et al., J. Biol. Chem., 270:3255-3260 (1995)].

As reviewed recently by Tewari et al., TNFR1, TNFR2 and CD40 modulate the expression of proinflammatory and costimulatory cytokines, cytokine receptors, and cell adhesion molecules through activation of the transcription factor, NF-kB [Tewari et al., Curr. Op. Genet. Develop., 6: 39-44 (1996)]. NF-kB is the prototype of a family of dimeric transcription factors whose subunits contain conserved Rel regions [Verma et al., Genes Develop., 9:2723-2735 (1996); Baldwin, Ann. Rev. Immunol., 14:649-681 (1996)]. In its latent form, NF-kB is complexed with members of the IkB inhibitor family; upon inactivation of the IkB in response to certain stimuli, released NF-kB trans locates to the nucleus where it binds to specific DNA sequences and activates gene transcription.

### SUMMARY OF THE INVENTION

The present invention provides DR4 antibodies which are capable of specifically binding to DR4. Preferred DR4 antibodies are capable of modulating biological activities associated with DR4 and/or Apo-2 ligand (TRAIL), in particular, apoptosis, and thus are useful in the treatment of various diseases and pathological conditions, including cancer or immune related diseases.

Accordingly, in one aspect, the present invention provides an isolated chimeric anti-DR4 antibody which comprises:
(a) a light chain variable domain, wherein said variable domain comprises amino acid residues 20 to 126 of SEQ ID NO:9;
(b) a light chain CH1 domain comprising amino acid residues 127 to 233 of SEQ ID NO:9;
(c) a heavy chain variable domain comprising amino acid residues 20 to 145 or 22 to 145 of SEQ ID NO:12; and
(d) heavy chain CH1, CH2, and CH3 domains comprising amino acid residues 146 to 476 of SEQ ID NO:12.

In a further aspect, the present invention provides isolated nucleic acid encoding the chimeric anti-DR4 antibody as defined above, vectors comprising the nucleic acid and host cells comprising the vectors.

In a further aspect, the present invention provides a method of producing a chimeric anti-DR4 antibody comprising culturing the above defined host cell, and recovering the antibody from the host cell culture.

In a further aspect, the present invention provides the use of a chimeric anti-DR4 antibody as defined above in the preparation of a medicament for inducing apoptosis in mammalian cancer cells, for example where said mammalian cancer cells comprise colon cancer cells, small cell lung cancer cells, non-small cell lung cancer cells, lung adenocarcinoma cells or lung squamous cell carcinoma cells.

### BRIEF DESCRIPTION OF THE DRAWINGS

Figure 1 shows the nucleotide sequence (SEQ ID NO:2) of a cDNA for human DR4 and its derived amino acid sequence (SEQ ID NO: 1). The respective nucleotide and amino acid sequences for human DR4 are also reported in Pan et al., Science, 276:111 (1997).
Figures 2 shows the FACS analysis of DR4 binding by two anti-DR4 antibodies, 4E7.24.3 ("4E7") and 4H6.17.8 ("4H6") (illustrated by the bold lines) as compared to IgG controls (dotted lines). Both antibodies recognized the DR4 receptor expressed in human 9D cells.
Figure 3 is a graph showing percent (%) apoptosis induced in 9D cells by DR4 antibodies, 4E7.24.3 and 4H6.17.8.
Figure 4 is a bar diagram showing percent (%) apoptosis, as compared to Apo-2L, in 9D cells by DR4 antibodies, 4E7.24.3 and 4H6.17.8, in the presence or absence of goat anti-mouse IgG Fc antibodies.
Figure 5 is a bar diagram illustrating the ability of DR4 antibody 4H6.17.8 to block the apoptosis induced by Apo-2L in 9D cells.
Figure 6 is a graph showing results of an ELISA testing binding of DR4 antibodies, 4E7.24.3 and 4H6.17.8, to DR4 and to other known Apo-2L receptors referred to as Apo-2, DcR1, and DcR2.
Figure 7 shows the binding affinities of DR4 antibodies, 4E7, 4H6, and 5G11.17.1 ("5G11"), to DR4-IgG, as determined in a KinExA™ assay. Binding affinities, e.g., of DR4 and DR5 immunoadhesins to Apo-2L are shown for comparison.
Figure 8A shows graphs illustrating percent (%) apoptosis (as determined by FACS analysis) induced in 9D cells by various concentrations of DR4 antibodies 1H5.25.9 ("1H5"), 4G7.18.8 ("4G7"), and 5G11, in the absence or presence of goat anti-mouse IgG Fc antibody or rabbit complement.
Figure 8B shows graphs illustrating apoptotic activity (as determined by FACS analysis) of DR4 antibodies 4G7 and 5G11 on 9D cells in the presence of goat anti-mouse IgG Fc antibody or rabbit complement.
Figure 9 shows apoptotic activity of DR4 antibodies, 4H6, 4E7, 4G7, 4G10.20.6 ("4G10"), 3G1.17.2 ("3G1"), 5G11, 1H8.17.5 ("1H8"), and 1H5.24.9 ("1H5") on SKMES-1 lung tumor cells in the presence of goat anti-mouse IgG Fc antibodies.
Figure 10A shows apoptotic activity of DR4 antibodies 4G7 and 5G11 on SKMES-1 lung tumor cells in the presence or absence of goat anti-mouse IgG Fc antibodies.
Figure 10B shows apoptotic activity of DR4 antibodies, 4G7 and 5G11, on SKMES-1 lung tumor cells in the presence or absence of rabbit complement.
Figure 11A shows apoptotic activity of DR4 antibodies, 4G7 and 5G11, on HCT116 colon tumor cells in the presence or absence of goat anti-mouse IgG Fc antibodies.
Figure 11B shows apoptotic activity of DR4 antibodies, 4G7 and 5G11, on HCT116 colon tumor cells in the presence or absence of rabbit complement.
Figure 12 shows the results of a PARP assay.
Figure 13 shows the effects of DR4 antibodies, 4G7 and 5G11, on the growth of HCT116 colon tumors in athymic nude mice, as measured by tumor volume.
Figure 14 shows the effects of DR4 antibodies, 4G7 and 5G11, on the growth of HCT116 colon tumors in athymic nude mice, as measured by tumor weight.
Figures 15 and 16 show the effects of DR4 antibodies, 4G7 and 4H6, on the growth of Colo205 colon tumors in athymic nude mice, as measured by tumor volume.
Figure 17 provides a table identifying DR4 antibodies 1H5.24.9; 1H8.17.5; 3G1.17.2; 4E7.24.3; 4G7.18.8; 4H6.17.8; 4G10.20.6; and 5G11.17.1, as well as various properties and activities identified with each respective antibody.

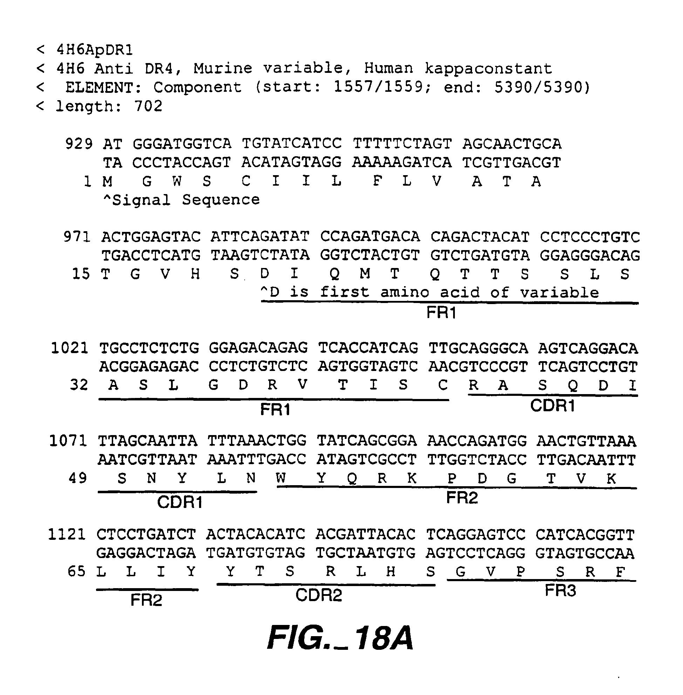

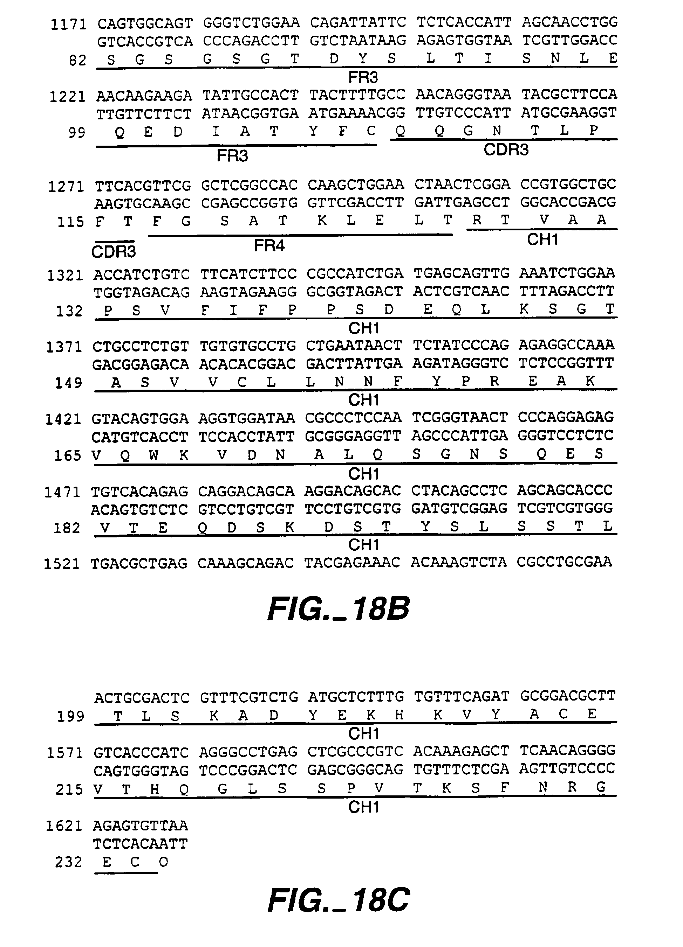

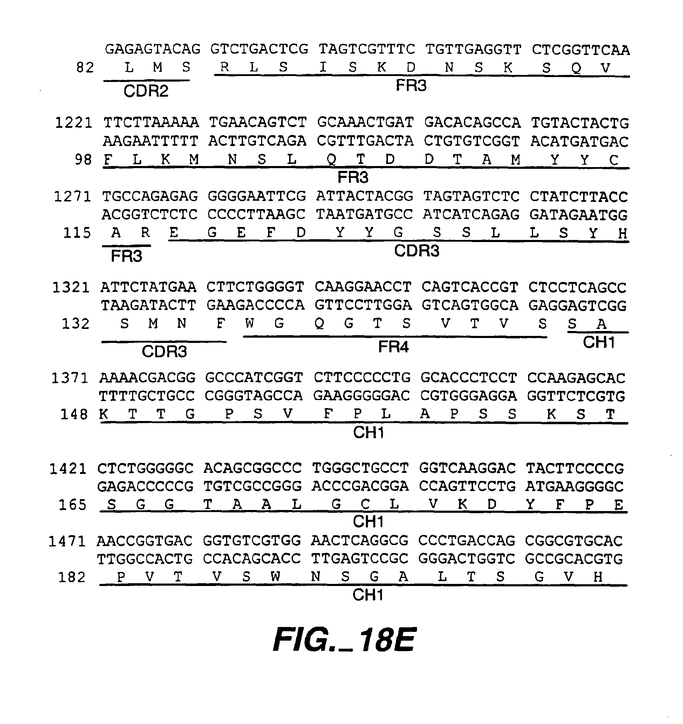

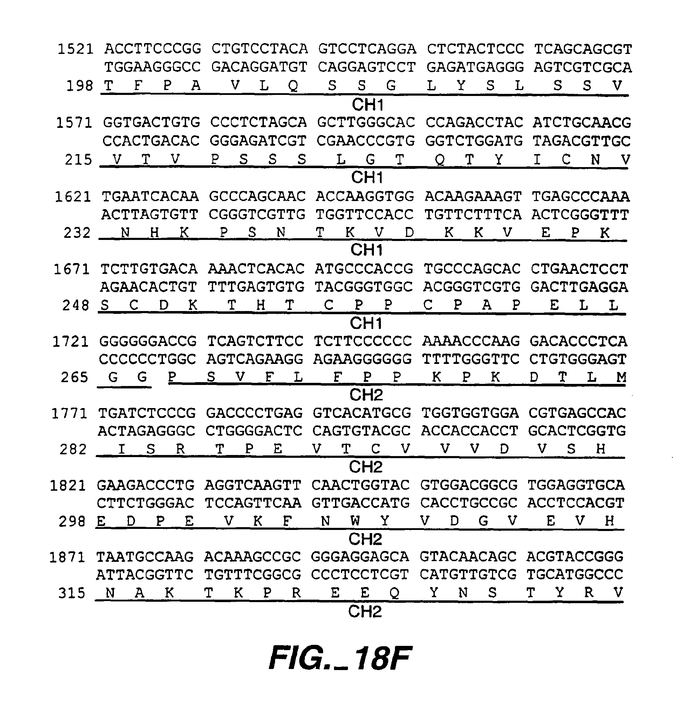

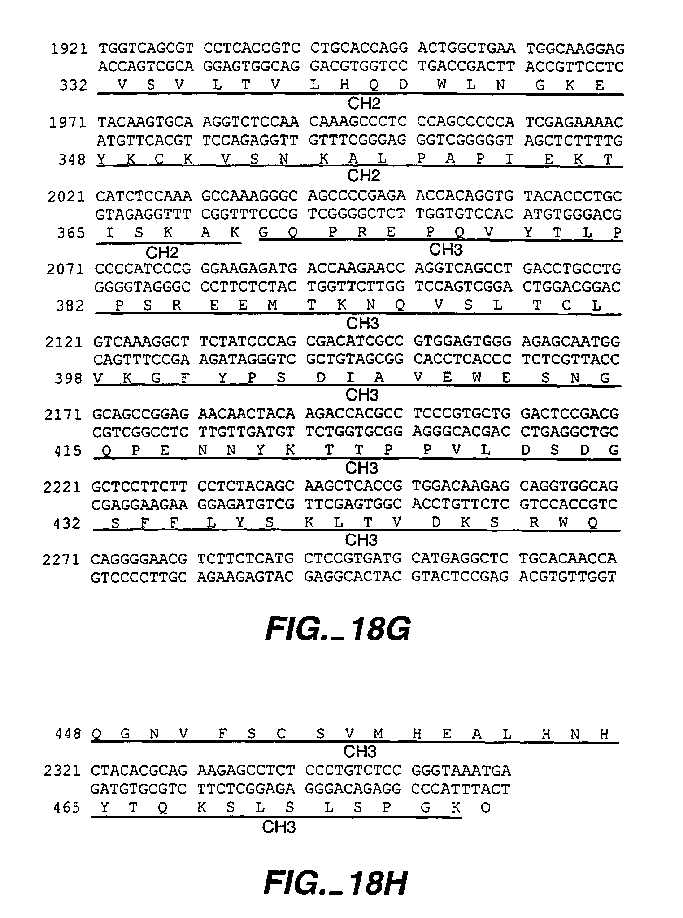

Figures 18A-18C show the light chain of the chimeric 4H6 anti-DR4 antibody, and include the encoding polynucleotide sequence (SEQ ID NO:7) and its complementary DNA sequence (SEQ ID NO:8), and the putative amino acid sequence (SEQ ID NO:9) which comprises the signal sequence (vector derived) (identified as amino acid residues 1 to 19 of SEQ ID NO:9); the light chain variable domain (identified as amino acid residues 20 to 126 of SEQ ID NO:9); and the human kappa CH1 constant domain (identified as amino acid residues 127 to 233 of SEQ ID NO:9). The respective Framework (FR1, FR2, FR3, and FR4) and CDR (CDR1, CDR2, CDR3) regions are also shown; the respective regions are underlined.

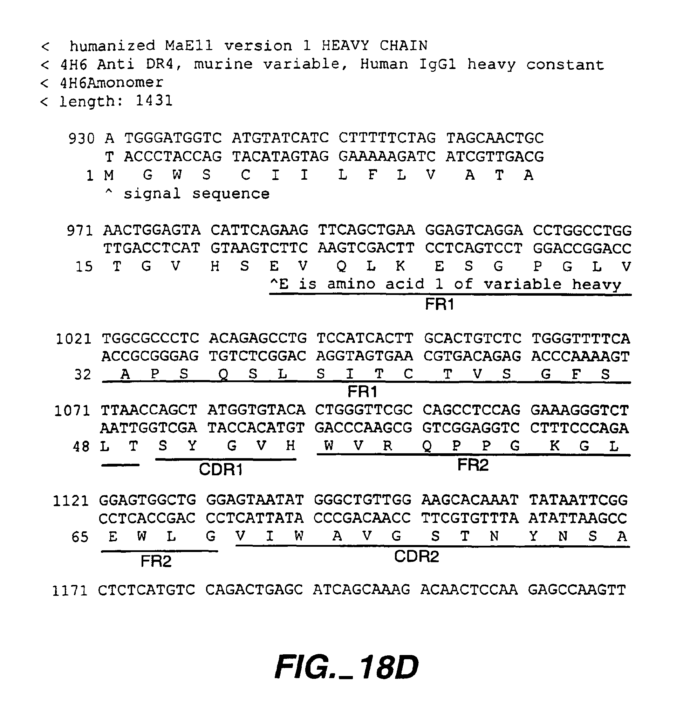

Figures 18D-18H show the heavy chain of the chimeric 4H6 anti-DR4 antibody, and include the encoding polynucleotide sequence (SEQ ID NO:10) and its complementary DNA sequence (SEQ ID NO:11), and the putative amino acid sequence (SEQ ID NO: 12) which comprises the signal sequence (vector derived) (identified as amino acid residues 1 to 19 of SEQ ID NO:12); the heavy chain variable domain (identified as amino acid residues 20 to 145 of SEQ ID NO:12); and the human IgG1 CH1, CH2, and CH3 constant domains (identified as amino acid residues 146 to 476 of SEQ ID NO:12). The amino acid residue at position 20 (which corresponds to the first amino acid of the 4H6 murine heavy chain variable domain) is shown to be a glutamic acid residue. It is noted that in the native 4H6 murine heavy chain variable domain sequenced from the 4H6.17.8 hybridoma, the first amino acid is a glutamine residue, not glutamic acid. The respective Framework (FR1, FR2, FR3, and FR4) and CDR (CDR1, CDR2, CDR3) regions are also shown; the respective regions are underlined.
Figure 19 shows the effects (*in vitro* cell killing of SK-MES-1 cells) of chimeric 4H6 antibody ("Ch4H6") (plus goat anti-human IgG Fc), as determined by crystal violet staining. The effects of murine 4H6 monoclonal antibody ("4H6"), F(ab)'2 4H6 and Apo2L are also shown.
Figure 20 shows the ADCC effects of chimeric 4H6 antibody ("c4H6") (plus goat anti-human IgG Fc) on Colo205 cells, as measured in a ⁵¹Cr release assay.
Figure 21 shows the effects of chimeric 4H6 antibody ("ch-4H6") on the growth of Colo205 colon tumors in athymic nude mice, as measured by tumor volume. The effects of murine monoclonal antibody ("4H6") and IgG1 are also shown.

### DETAILED DESCRIPTION OF THE PREFERRED EMBODIMENTS

### I. Definitions

As used herein, the term "Apo-2 ligand" or "Apo-2L" (also known as TRAIL) refers to a specific member of the tumor necrosis factor (TNF) ligand family that, among other things, induces apoptosis in a variety of cell lineages [see WO 97/25428 published July 17, 1997; WO97/01633 published January 16, 1997; Pitti et al., J. Biol. Chem, 271:12687 (1996); Marsters et al., Curr. Biol., 6:79 (1997); Wiley, S. et al., Immunity, 3:637 (1995)].

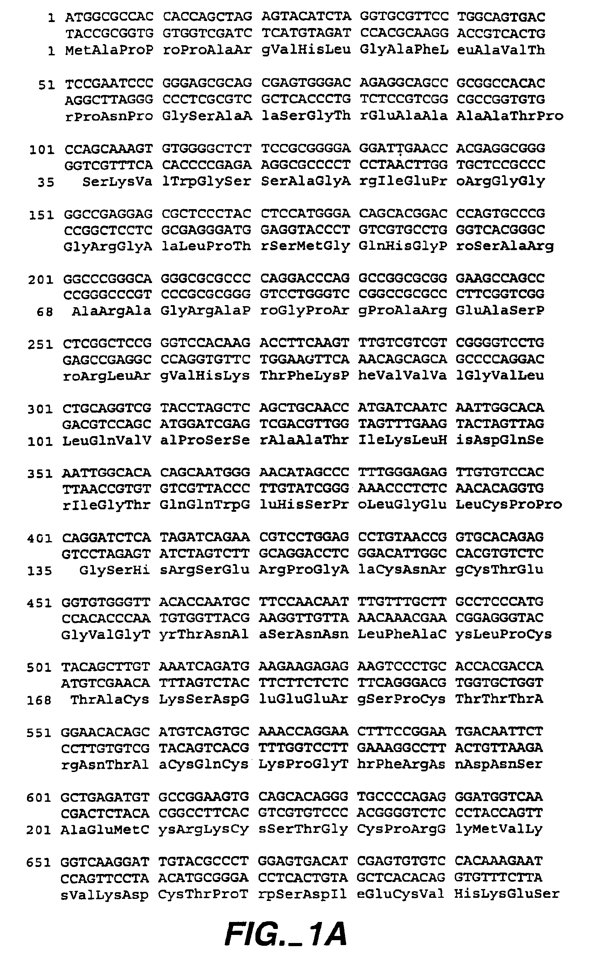

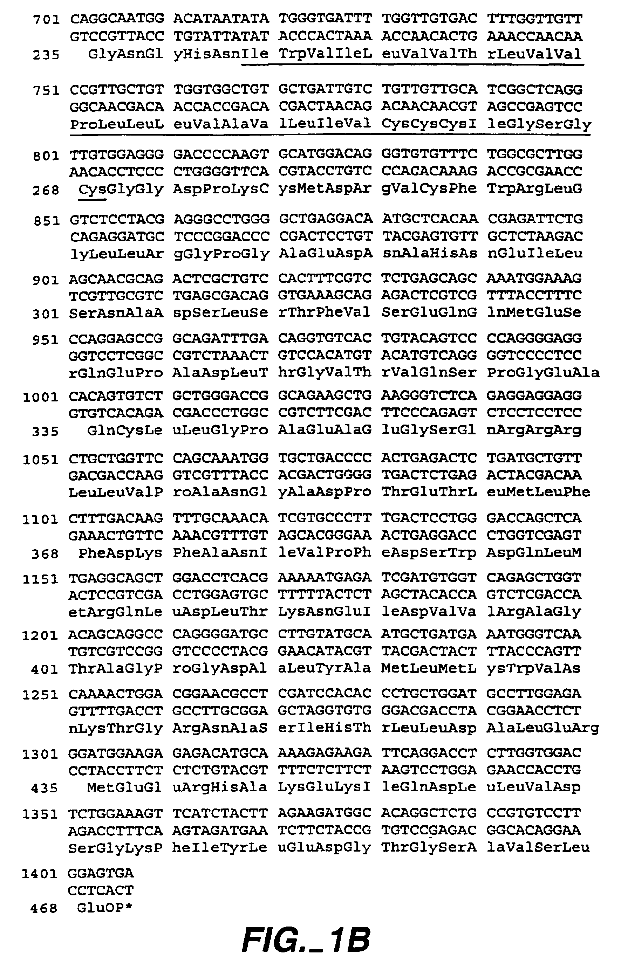

A receptor for Apo-2L has been identified and referred to as DR4, a member of the TNF-receptor family that contains a cytoplasmic "death domain" capable of engaging the cell suicide apparatus [see Pan et al., Science, 276:111 (1997)]. DR4 has also been described in WO98/32856 published July 30, 1998. The term "Death Receptor 4" or "DR4" when used herein encompasses native sequence DR4 and DR4 variants (which are further defined herein). These terms encompass DR4 expressed in a variety of mammals, including humans. DR4 may be endogenously expressed as occurs naturally in a variety of human tissue lineages, or may be expressed by recombinant or synthetic methods. A "native sequence DR4" comprises a polypeptide having the same amino acid sequence as a DR4 derived from nature. Thus, a native sequence DR4 can have the amino acid sequence of naturally-occurring DR4 from any mammal. Such native sequence DR4 can be isolated from nature or can be produced by recombinant or synthetic means. The term "native sequence DR4" specifically encompasses naturally-occurring truncated or secreted forms of the DR4 (e.g., a soluble form containing, for instance, an extracellular domain sequence), naturally-occurring variant forms (e.g., alternatively spliced forms) and naturally-occurring allelic variants of the DR4. In one embodiment of the invention, the native sequence DR4 is a mature or full-length native sequence DR4 comprising amino acids 1 to 468 of Fig. 1 (SEQ ID NO:1).

The terms "extracellular domain" or "ECD" herein refer to a form of DR4 which is essentially free of the transmembrane and cytoplasmic domains of DR4. Ordinarily, DR4 ECD will have less than 1% of such transmembrane and/or cytoplasmic domains and preferably, will have less than 0.5% of such domains. Optionally, DR4 ECD will comprise amino acid residues 1 to 218 or residues 24 to 218 of Fig. 1 (SEQ ID NO:1).

"DR4 variant" means a biologically active DR4 having at least about 80% or 85% amino acid sequence identity with the DR4 having the deduced amino acid sequence shown in Fig. 1 (SEQ ID NO:1) for a full-length native sequence or extracellular domain sequence of human DR4. Such DR4 variants include, for instance, DR4 polypeptides wherein one or more amino acid residues are added, or deleted (i.e., fragments), at the N- or C-terminus of the sequence of Fig. 1 (SEQ ID NO:1). Ordinarily, an DR4 variant will have at least about 80% amino acid sequence identity, more preferably at least about 90% amino acid sequence identity, and even more preferably at least about 95% amino acid sequence identity with the amino acid sequence of Fig. 1 (SEQ ID NO:1).

"Percent (%) amino acid sequence identity" with respect to the DR4 sequences (or DR4 antibody sequences) identified herein is defined as the percentage of amino acid residues in a candidate sequence that are identical with the amino acid residues in the DR4 sequence (or DR4 antibody sequence), after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as ALIGN™ , Megalign (DNASTAR), or ALIGN-2 (authored by Genentech, Inc. and filed with the U.S. Copyright Office on December 10, 1991). The ALIGN-2 software is publicly available from Genentech, Inc. The ALIGN-2 program should be compiled for use on a UNIX operating system, preferably digital UNIX V4.0D. All sequence comparison parameters are set by the ALIGN-2 program and do not vary. Those skilled in the art can determine appropriate parameters for measuring alignment, including any algorithms needed to achieve maximal alignment over the full length of the sequences being compared.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide in situ within recombinant cells, since at least one component of the DR4 or DR4 antibody natural environment will not be present. Ordinarily, however, isolated polypeptide will be prepared by at least one purification step.

An "isolated" nucleic acid molecule is a nucleic acid molecule that is identified and separated from at least one contaminant nucleic acid molecule with which it is ordinarily associated in the natural source of the polypeptide nucleic acid. An isolated nucleic acid molecule is other than in the form or setting in which it is found in nature. Isolated nucleic acid molecules therefore are distinguished from the nucleic acid molecule as it exists in natural cells. However, an isolated nucleic acid molecule includes a nucleic acid molecule contained in cells that ordinarily express the polypeptide where, for example, the nucleic acid molecule is in a chromosomal location different from that of natural cells.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to re-anneal when complementary strands are present in an environment below their melting temperature. The higher the degree of desired identity between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The expression "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The terms "amino acid" and "amino acids" refer to all naturally occurring L-alpha-amino acids. This definition is meant to include norleucine, ornithine, and homocysteine. The amino acids are identified by either the single-letter or three-letter designations:

| | | | | | |
|---|---|---|---|---|---|
| Asp | D | aspartic acid | Ile | I | isoleucine |
| Thr | T | threonine | Leu | L | leucine |
| Ser | S | serine | Tyr | Y | tyrosine |
| Glu | E | glutamic acid | Phe | F | phenylalanine |
| Pro | P | proline | His | H | histidine |
| Gly | G | glycine | Lys | K | lysine |
| Ala | A | alanine | Arg | R | arginine |
| Cys | C | cysteine | Trp | W | tryptophan |
| Val | V | valine | Gln | Q | glutamine |
| Met | M | methionine | Asn | N | asparagine |

In the Sequence Listing and Figures, certain other single-letter or three-letter designations are employed to refer to and identify two or more amino acids or nucleotides at a given position in the sequence. For instance, at amino acid residue 20 in SEQ ID NO:12, the three-letter designation "Xaa" is employed to identify that at residue 20, the amino acid may be a glutamine or a glutamic acid residue. In the nucleotide sequences referred to in Example 16 and in Figure 18D, the designation "w" indicates the nucleotide may be an "a" or "t"; "k" indicates the nucleotide may be "g" or "t"; "b" indicates the nucleotide may be "g" or "t" or "c"; "y" indicates the nucleotide may be "c" or "t"; "r" indicates the nucleotide may be "a" or "g"; "s" indicates the nucleotide may be "g" or "c"; "m indicates the nucleotide may be "a" or "c"; and "n" indicates the nucleotide may be "a" or "t" or "c" or "g".

The terms "agonist" and "agonistic" when used herein refer to or describe a molecule which is capable of, directly or indirectly, substantially inducing, promoting or enhancing DR4 biological activity or activation. Optionally, an "agonist DR4 antibody" is an antibody which has activity comparable to the ligand for DR4, known as Apo-2 ligand (TRAIL).

The terms "antagonist" and "antagonistic" when used herein refer to or describe a molecule which is capable of, directly or indirectly, substantially counteracting, reducing or inhibiting DR4 biological activity or DR4 activation.

The term "antibody" is used in the broadest sense and specifically covers single anti-DR4 monoclonal antibodies (including agonist, antagonist, and neutralizing or blocking antibodies) and anti-DR4 antibody compositions with polyepitopic specificity. "Antibody" as used herein includes intact immunoglobulin or antibody molecules, polyclonal antibodies, multispecific antibodies (i.e., bispecific antibodies formed from at least two intact antibodies) and immunoglobulin fragments (such as Fab, F(ab')₂, or Fv), so long as they exhibit any of the desired agonistic or antagonistic properties described herein.

Antibodies are typically proteins or polypeptides which exhibit binding specificity to a specific antigen. Native antibodies are usually heterotetrameric glycoproteins, composed of two identical light (L) chains and two identical heavy (H) chains. Typically, each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains [Chothia et al., J. Mol. Biol., 186:651-663 (1985); Novotny and Haber, Proc. Natl. Acad. Sci. USA, 82:4592-4596 (1985)]. The light chains of antibodies from any vertebrate species can be assigned to one of two clearly distinct types, called kappa and lambda, based on the amino acid sequences of their constant domains. Depending on the amino acid sequence of the constant domain of their heavy chains, immunoglobulins can be assigned to different classes. There are five major classes of immunoglobulins: IgA, IgD, IgE, IgG and IgM, and several of these may be further divided into subclasses (isotypes), e.g., IgG-1, IgG-2, IgG-3, and IgG-4; IgA-1 and IgA-2. The heavy chain constant domains that correspond to the different classes of immunoglobulins are called alpha, delta, epsilon, gamma, and mu, respectively.

"Antibody fragments" comprise a portion of an intact antibody, generally the antigen binding or variable region of the intact antibody. Examples of antibody fragments include Fab, Fab', F(ab')2, and Fv fragments, diabodies, single chain antibody molecules, and multispecific antibodies formed from antibody fragments.

The term "variable" is used herein to describe certain portions of the variable domains which differ in sequence among antibodies and are used in the binding and specificity of each particular antibody for its particular antigen. However, the variability is not usually evenly distributed through the variable domains of antibodies. It is typically concentrated in three segments called complementarity determining regions (CDRs) or hypervariable regions both in the light chain and the heavy chain variable domains. The more highly conserved portions of the variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen binding site of antibodies [see Kabat, E.A. et al., Sequences of Proteins of Immunological Interest, National Institutes of Health, Bethesda, MD (1987)]. The constant domains are not involved directly in binding an antibody to an antigen, but exhibit various effector functions, such as participation of the antibody in antibody-dependent cellular toxicity.

The term "monoclonal antibody" as used herein refers to an antibody obtained from a population of substantially homogeneous antibodies, i.e., the individual antibodies comprising the population are identical except for possible naturally-occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen.

The monoclonal antibodies herein include chimeric, hybrid and recombinant antibodies produced by splicing a variable (including hypervariable) domain of an anti-DR4 antibody with a constant domain (*e.g*. "humanized" antibodies), or a light chain with a heavy chain, or a chain from one species with a chain from another species, or fusions with heterologous proteins, regardless of species of origin or immunoglobulin class or subclass designation, as well as antibody fragments (*e.g*., Fab, F(ab')₂, and Fv), so long as they exhibit the desired biological activity or properties. *See, e.g.* U.S. Pat. No. 4,816,567 and Mage et al., in Monoclonal Antibody Production Techniques and Applications, pp.79-97 (Marcel Dekker, Inc.: New York, 1987).

Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler and Milstein, Nature, 256:495 (1975), or may be made by recombinant DNA methods such as described in U.S. Pat. No. 4,816,567. The "monoclonal antibodies" may also be isolated from phage libraries generated using the techniques described in McCafferty et al., Nature, 348:552-554 (1990), for example.

"Humanized" forms of non-human (e.g. murine) antibodies are specific chimeric immunoglobulins, immunoglobulin chains, or fragments thereof (such as Fv, Fab, Fab', F(ab')₂ or other antigen-binding subsequences of antibodies) which contain minimal sequence derived from non-human immunoglobulin. For the most part, humanized antibodies are human immunoglobulins (recipient antibody) in which residues from a complementary determining region (CDR) of the recipient are replaced by residues from a CDR of a non-human species (donor antibody) such as mouse, rat, or rabbit having the desired specificity, affinity, and capacity. In some instances, Fv framework region (FR) residues of the human immunoglobulin are replaced by corresponding non-human residues. Furthermore, the humanized antibody may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region or domain (Fc), typically that of a human immunoglobulin.

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology, 14:309-314 (1996): Sheets et al. PNAS, (USA) 95:6157-6162 (1998)); Hoogenboom and Winter, J. Mol. Biol., 227:381 (1991); Marks et al., J. Mol. Biol., 222:581 (1991)). Human antibodies can also be made by introducing human immunoglobulin loci into transgenic animals, *e.g*., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology, 10: 779-783 (1992); Lonberg et al., Nature, 368: 856-859 (1994); Morrison, Nature, 368:812-13 (1994); Fishwild et al., Nature Biotechnology, 14: 845-51 (1996); Neuberger, Nature Biotechnology, 14: 826 (1996); Lonberg and Huszar, Intern. Rev. Immunol., 13:65-93 (1995). Alternatively, the human antibody may be prepared via immortalization of human B lymphocytes producing an antibody directed against a target antigen (such B lymphocytes may be recovered from an individual or may have been immunized *in vitro*). See, *e.g*., Cole et al., Monoclonal Antibodies and Cancer Therapy, Alan R. Liss, p. 77 (1985); Boerner et al., J. Immunol., 147 (1):86-95 (1991); and US Pat No. 5,750,373.

The term "Fc region" is used to define the C-terminal region of an immunoglobulin heavy chain which may be generated by papain digestion of an intact antibody. The Fc region may be a native sequence Fc region or a variant Fc region. Although the boundaries of the Fc region of an immunoglobulin heavy chain might vary, the human IgG heavy chain Fc region is usually defined to stretch from an amino acid residue at about position Cys226, or from about position Pro230, to the carboxyl-terminus of the Fc region (using herein the numbering system according to Kabat et al., supra). The Fc region of an immunoglobulin generally comprises two constant domains, a CH2 domain and a CH3 domain, and optionally comprises a CH4 domain.

By "Fc region chain" herein is meant one of the two polypeptide chains of an Fc region.

The "CH2 domain" of a human IgG Fc region (also referred to as "cγ2" domain) usually extends from an amino acid residue at about position 231 to an amino acid residue at about position 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec. Immunol.22:161-206 (1985). The CH2 domain herein may be a native sequence CH2 domain or variant CH2 domain.

The "CH3 domain" comprises the stretch of residues C-terminal to a CH2 domain in an Fc region (*i.e.* from an amino acid residue at about position 341 to an amino acid residue at about position 447 of an IgG). The CH3 region herein may be a native sequence CH3 domain or a variant CH3 domain (*e*.*g*. a CH3 domain with an introduced "protroberance" in one chain thereof and a corresponding introduced "cavity" in the other chain thereof; see US Patent No. 5,821,333). Such variant CH3 domains may be used to make multispecific (*e.g*. bispecific) antibodies as herein described.

"Hinge region" is generally defined as stretching from about Glu216, or about Cys226, to about Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions. The hinge region herein may be a native sequence hinge region or a variant hinge region. The two polypeptide chains of a variant hinge region generally retain at least one cysteine residue per polypeptide chain, so that the two polypeptide chains of the variant hinge region can form a disulfide bond between the two chains. The preferred hinge region herein is a native sequence human hinge region, *e.g*. a native sequence human IgG1 hinge region.

A "functional Fc region" possesses at least one "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (*e.g*. B cell receptor; BCR), etc. Such effector functions generally require the Fc region to be combined with a binding domain (*e*.*g*. an antibody variable domain) and can be assessed using various assays known in the art for evaluating such antibody effector functions.

A "native sequence Fc region" comprises an amino acid sequence identical to the amino acid sequence of an Fc region found in nature. A "variant Fc region" comprises an amino acid sequence which differs from that of a native sequence Fc region by virtue of at least one amino acid modification. Preferably, the variant Fc region has at least one amino acid substitution compared to a native sequence Fc region or to the Fc region of a parent polypeptide, *e.g*. from about one to about ten amino acid substitutions, and preferably from about one to about five amino acid substitutions in a native sequence Fc region or in the Fc region of the parent polypeptide. The variant Fc region herein will preferably possess at least about 80% sequence identity with a native sequence Fc region and/or with an Fc region of a parent polypeptide, and most preferably at least about 90% sequence identity therewith, more preferably at least about 95% sequence identity therewith.

"Antibody-dependent cell-mediated cytotoxicity" and "ADCC" refer to a cell-mediated reaction in which nonspecific cytotoxic cells that express Fc receptors (FcRs) (*e.g*. Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol., 9:457-92 (1991). To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. PNAS (USA), 95:652-656 (1998).

"Human effector cells" are leukocytes which express one or more FcRs and perform effector functions. Preferably, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred. The effector cells may be isolated from a native source thereof, *e*.*g*. from blood or PBMCs as described herein.

The terms "Fc receptor" and "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (reviewed in Daëron, Annu. Rev. Immunol., 15:203-234 (1997)). FcRs are reviewed in Ravetch and Kinet, Annu. Rev. Immunol., 9:457-92 (1991); Capel et al., Immunomethods, 4:25-34 (1994); and de Haas et al., J. Lab. Clin. Med., 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol., 117:587 (1976); and Kim et al., J. Immunol., 24:249 (1994)).

"Complement dependent cytotoxicity" and "CDC" refer to the lysing of a target in the presence of complement. The complement activation pathway is initiated by the binding of the first component of the complement system (C1q) to a molecule (*e*.*g*. an antibody) complexed with a cognate antigen. To assess complement activation, a CDC assay, *e.g*. as described in Gazzano-Santoro et al., J. Immunol. Methods, 202:163 (1996), may be performed.

An "affinity matured" antibody is one with one or more alterations in one or more CDRs thereof which result an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Hio/Technology, 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene, 169:147-155 (1995); Yelton et al. J. Immunol., 155:1994-2004 (1995); Jackson et al., J. Immunol., 154(7):3310-9 (1995); and Hawkins et al, J. Mol. Biol., 226:889-896 (1992).

"Biologically active" and "desired biological activity" for the purposes herein mean having the ability to modulate DR4 activity or DR4 activation, including, by way of example, apoptosis (either in an agonistic or stimulating manner or in an antagonistic or blocking manner) in at least one type of mammalian cell *in vivo* or *ex vivo* or binding to Apo-2 ligand (TRAIL).

The terms "apoptosis" and "apoptotic activity" are used in a broad sense and refer to the orderly or controlled form of cell death in mammals that is typically accompanied by one or more characteristic cell changes, including condensation of cytoplasm, loss of plasma membrane microvilli, segmentation of the nucleus, degradation of chromosomal DNA or loss of mitochondrial function. This activity can be determined and measured, for instance, by cell viability assays, FACS analysis or DNA electrophoresis, all of which are known in the art.

The terms "cancer," "cancerous," and "malignant" refer to or describe the physiological condition in mammals that is typically characterized by unregulated cell growth. Examples of cancer include but are not limited to, carcinoma, including adenocarcinoma, lymphoma, blastoma, melanoma, sarcoma, and leukemia. More particular examples of such cancers include squamous cell cancer, small-cell lung cancer, non-small cell lung cancer, lung adenocarcinoma, lung squamous cell carcinoma, gastrointestinal cancer, Hodgkin's and non-Hodgkin's lymphoma, pancreatic cancer, glioblastoma, cervical cancer, glioma, ovarian cancer, liver cancer such as hepatic carcinoma and hepatoma, bladder cancer, breast cancer, colon cancer, colorectal cancer, endometrial or uterine carcinoma, salivary gland carcinoma, kidney cancer such as renal cell carcinoma and Wilms' tumors, basal cell carcinoma, melanoma, prostate cancer, vulval cancer, thyroid cancer, testicular cancer, esophageal cancer, and various types of head and neck cancer.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are autoimmune diseases, immune-mediated inflammatory diseases, non-immune-mediated inflammatory diseases, infectious diseases, and immunodeficiency diseases. Examples of immune-related and inflammatory diseases, some of which are immune or T cell mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjogren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory and fibrotic lung diseases such as inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft-versus-host-disease. Infectious diseases include AIDS (HIV infection), hepatitis A, B, C, D, and E, bacterial infections, fungal infections, protozoal infections and parasitic infections.

"Autoimmune disease" is used herein in a broad, general sense to refer to disorders or conditions in mammals in which destruction of normal or healthy tissue arises from humoral or cellular immune responses of the individual mammal to his or her own tissue constituents. Examples include, but are not limited to, lupus erythematous, thyroiditis, rheumatoid arthritis, psoriasis, multiple sclerosis, autoimmune diabetes, and inflammatory bowel disease (IBD).

A "growth inhibitory agent" when used herein refers to a compound or composition which inhibits growth of a cell *in vitro* and/or *in vivo.* Thus, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), TAXOL®, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13.

The term "prodrug" as used in this application refers to a precursor or derivative form of a pharmaceutically active substance that is less cytotoxic to cancer cells compared to the parent drug and is capable of being enzymatically activated or converted into the more active parent form. See, e.g., Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glycosylated prodrugs, beta-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described below.

The term "cytotoxic agent" as used herein refers to a substance that inhibits or prevents the function of cells and/or causes destruction of cells. The term is intended to include radioactive isotopes (*e.g.* At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of conditions like cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine,trietylenephosphoramide,triethylenethiophosphaoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (*e.g*. calicheamicin, especially calicheamicin γ₁^{I} and calicheamicin θ^{I}₁, see, *e*.*g*., Agnew Chem Intl. Ed. Engl. 33:183-186 (1994); dynemicin, including dynemicin A; an esperamicin; as well as neocarzinostatin chromophore and related chromoprotein enediyne antiobiotic chromomophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; antimetabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; podophyllinic acid; 2-ethylhydrazide; procarbazine; PSK®; razoxane; rhizoxin; sizofiran; spirogermanium; tenuazonic acid; triaziquone; 2, 2',2''-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL^{®}, Bristol-Myers Squibb Oncology, Princeton, NJ) and doxetaxel (TAXOTERE^{®}, Rhône-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; capecitabine; and pharmaceutically acceptable salts, acids or derivatives of any of the above. Also included in this definition are anti-hormonal agents that act to regulate or inhibit hormone action on tumors such as antiestrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and - beta; mullerian-inhibiting substance; mouse gonadotropin-associated peptide; inhibin; activin; vascular endothelial growth factor; integrin; thrombopoietin (TPO); nerve growth factors such as NGF-alpha; platelet-growth factor; transforming growth factors (TGFs) such as TGF-alpha and TGF-beta; insulin-like growth factor-I and -II; erythropoietin (EPO); osteoinductive factors; interferons such as interferon-alpha, -beta and - gamma colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; a tumor necrosis factor such as TNF-alpha or TNF-beta; and other polypeptide factors including LIF and kit ligand (KL). As used herein, the term cytokine includes proteins from natural sources or from recombinant cell culture and biologically active equivalents of the native sequence cytokines.

The terms "treating," "treatment," and "therapy" as used herein refer to curative therapy, prophylactic therapy, and preventative therapy.

The term "therapeutically effective amount" refers to an amount of a drug effective to treat a disease or disorder in a mammal. In the case of cancer, the therapeutically effective amount of the drug may reduce the number of cancer cells; reduce the tumor size; inhibit (*i.e*., slow to some extent and preferably stop) cancer cell infiltration into peripheral organs; inhibit (*i.e*., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy *in vivo* can, for example, be measured by assessing tumor burden or volume, the time to disease progression (TTP) and/or determining the response rates (RR).

The term "mammal" as used herein refers to any mammal classified as a mammal, including humans, cows, horses, dogs and cats. In a preferred embodiment of the invention, the mammal is a human.

### II. Compositions and Methods of the Invention

### A. DR4 Antibodies

The present invention relates to chimeric anti-DR4 antibodies as set out in the claims.

These antibodies may be agonists, antagonists or blocking antibodies.

### 1. Polyclonal Antibodies

The antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the DR4 polypeptide (or a DR4 ECD) or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation. The mammal can then be bled, and the serum assayed for DR4 antibody titer. If desired, the mammal can be boosted until the antibody titer increases or plateaus.

### 2. Monoclonal Antibodies

The antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the DR4 polypeptide (or a DR4 ECD) or a fusion protein thereof, such as a DR4 ECD-IgG fusion protein. The immunizing agent may alternatively comprise a fragment or portion of DR4 having one or more amino acids that participate in the binding of Apo-2L to DR4. In a preferred embodiment, the immunizing agent comprises an extracellular domain sequence of DR4 fused to an IgG sequence, such as described in Example 1.

Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. An example of such a murine myeloma cell line is P3X63AgU.1 described in Example 2 below. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against DR4. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium or RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (*e.g*., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

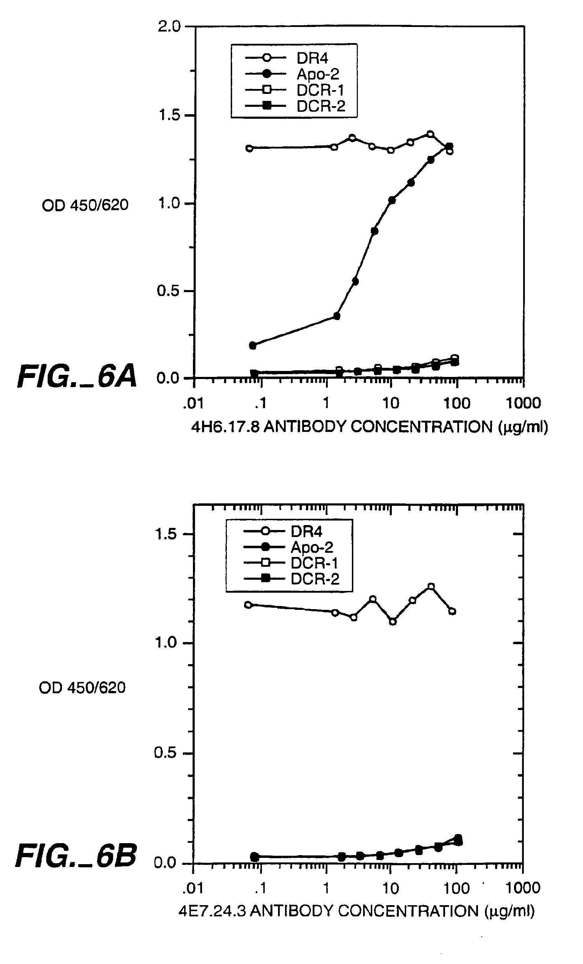

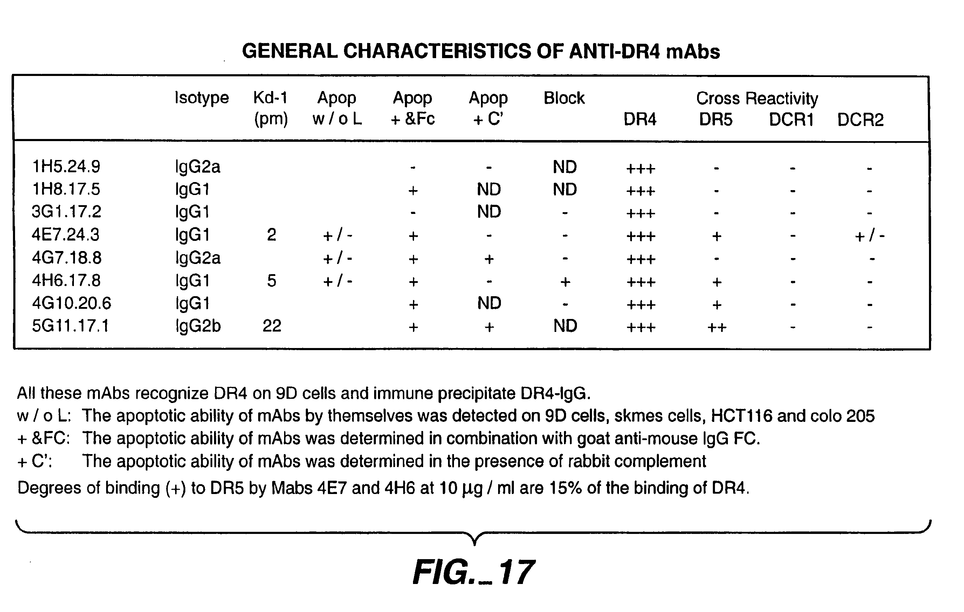

As described in the Examples below, various anti-DR4 monoclonal antibodies have been identified and prepared some of which are the subject of the claims. Certain of those antibodies, referred to as 4E7.24.3, 4H6.17.8, 1H5.25.9, 4G7.18.8, and 5G11.17.1 herein, have been deposited with ATCC. In one embodiment, the monoclonal antibodies of the invention will have the same biological characteristics as the monoclonal antibodies secreted by the hybridoma cell line(s) referred to above which have been deposited with ATCC. The term "biological characteristics" is used to refer to the *in vitro* and/or *in vivo* activities or properties of the monoclonal antibody, such as the ability to specifically bind to DR4 or to block, induce or enhance DR4 activation (or DR4-related activities). By way of example, a blocking antibody may block binding of Apo-2 ligand to DR4 or block Apo-2 ligand-induced apoptosis in a mammalian cell (such as a cancer cell). As disclosed in the present specification (see Figure 6), the monoclonal antibody 4E7.24.3 is characterized as specifically binding to DR4 (and having some cross reactivity to Apo-2), capable of inducing apoptosis, and not capable of blocking DR4. The monoclonal antibody 4H6.17.8 is characterized as specifically binding to DR4 (and having some cross-reactivity to Apo-2), capable of inducing apoptosis, and capable of blocking Apo-2 ligand binding to DR4. As disclosed herein, the 4H6.17.8 antibody exhibited more potent anti-cancer activity than the 4E7.24.3 antibody in an *in vivo* tumor model. Yet, the 4E7.24.3 antibody did exhibit anti-tumor activity even though it was not capable of blocking Apo-2 ligand to DR4. This observation suggests that an anti-DR4 antibody having an epitope which is the same as the Apo-2 ligand binding site on DR4, or alternatively, either overlaps with the Apo-2 ligand binding site on DR4 or creates a steric conformation which prevents Apo-2 ligand from binding DR4, is not essential or required for apoptotic or anti-tumor activity. However, a DR4 antibody having such an epitope or steric conformation may exhibit enhanced efficiency or potency of such apoptotic or anti-tumor activity. The properties and activities of the 1H5.25.9 4G7.18.8 and 5G11.17.1 antibodies are also described in the Examples below (and also referred to in Fig. 17). Optionally, the monoclonal antibodies of the present invention will bind to the same epitope(s) as the 4E7.24.3, 4H6.17.8, 1H5.25.9, 4G7.18.8, and/or 5G11.17.1 antibodies disclosed herein. This can be determined by conducting various assays, such as described herein and in the Examples. For instance, to determine whether a monoclonal antibody has the same specificity as the DR4 antibodies specifically referred to herein, one can compare its activity in DR4 blocking assays or apoptosis induction assays, such as those described in the Examples below.

As further described in the Examples below, the light and heavy chain variable domains of the murine 4H6.17.8 monoclonal antibody were sequenced, and a chimeric form of the 4H6.17.8 antibody was constructed (referred to herein as the "chimeric 4H6 antibody"). The present invention contemplates that the claimed anti-DR4 chimeric antibodies will have therapeutic and/or diagnostic utility, such as described herein. Chimeric, hybrid or recombinant anti-DR4 antibodies (as well as, for instance, diabodies or triabodies described further below) may comprise an antibody having full length heavy and light chains (such as, e.g., the light and heavy chains shown in Figures 18A-18H) or fragments thereof, such as a Fab, Fab', F(ab')₂ or Fv fragment, a monomer or dimer of such light chain or heavy chain, a single chain Fv in which such heavy or light chain(s) are joined by a linker molecule, or having variable domains (or hypervariable domains) of such light or heavy chain(s) combined with still other types of antibody domains.

In the present invention, the DR4 antibody comprises a light chain, wherein the light chain includes a variable domain comprising amino acids 20 to 126 of Figures 18A-18C (SEQ ID NO:9). The light chain in such a DR4 antibody may optionally comprise a signal sequence comprising amino acids 1 to 19 of Figures 18A-18C (SEQ ID NO:9). The antibodies of the present invention further comprise a human CH1 domain comprising amino acids 127 to 233 of Figures 18A-18C (SEQ ID NO:9), a heavy chain, wherein the heavy chain includes a variable domain comprising amino acids 20 to 145 of Figures 18D-18H (SEQ ID NO:12) or amino acids 22 to 145 of Figures 18D-18H (SEQ ID NO:12). The heavy chain in such a DR4 antibody may optionally comprise a signal sequence comprising amino acids 1 to 19 of Figures 18D-18H (SEQ ID NO:12). The antibodies of the present invention further comprise human CH1, CH2, and CH3 domains comprising amino acids 146 to 476 of Figures 18D-18H (SEQ ID NO:12).

It is contemplated that various regions or domains of the antibody sequences described herein, including the variable domain (or hypervariable domain) sequences (identified in Figures 18A-18H) of the light and/or heavy chains of the murine 4H6 monoclonal antibody, may be modified in terms of amino acid composition. For instance, it is contemplated that one or more conservative substitution(s) of amino acids may be made in the variable domains provided in Figures 18A-18C or in Figures 18D-18H. It is also contemplated that amino acid modications can be made in any one or more of the CDR or framework regions identified in the variable domains shown in Figures 18A-18H.

Such amino acid sequence modification(s) of the antibodies described herein may, for example, be desirable to improve the binding affinity and/or other biological properties of the antibody. Amino acid sequence variants of the antibody can be prepared by introducing appropriate nucleotide changes into the antibody nucleic acid, or by peptide synthesis. Such modifications include, for example, deletions from, and/or insertions into and/or substitutions of, residues within the amino acid sequences of the antibody. Any combination of deletion, insertion, and substitution is made to arrive at the final construct, provided that the final construct possesses the desired characteristics. The amino acid changes also may alter post-translational processes of the antibody, such as changing the number or position of glycosylation sites. Such alterations may be made to the parent antibody and/or may be introduced in the modified antibody amino acid sequence at the time that sequence is made.

A useful method for identification of certain residues or regions of the antibody that are preferred locations for mutagenesis is called "alanine scanning mutagenesis" as described by Cunningham and Wells Science, 244:1081-1085 (1989). Here, a residue or group of target residues are identified (*e.g*., charged residues such as arg, asp, his, lys, and glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per* se need not be predetermined. For example, to analyze the performance of a mutation at a given site, ala scanning or random mutagenesis is conducted at the target codon or region and the expressed antibodies are screened for the desired property or activity.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic agent. Other insertional variants of the antibody molecule include the fusion to the N- or C-terminus of the antibody to an enzyme (*e*.*g*. for ADEPT) or a polypeptide which increases the serum half-life of the antibody.

Another type of variant is an amino acid substitution variant. These variants have at least one amino acid residue in the antibody molecule replaced by a different residue. The sites of greatest interest for substitutional mutagenesis include the hypervariable regions, but framework alterations are also contemplated. Conservative substitutions are shown in Table 1 under the heading of "preferred substitutions". If such substitutions result in a change in biological activity or properties, then more substantial changes, denominated "exemplary substitutions" in Table 1, or as further described below in reference to amino acid classes, may be introduced and the products screened.

**Table 1**

| **Original Residue** | **Exemplary Substitutions** | **Preferred Substitutions** |
|---|---|---|
| Ala (A) | val; leu; ile | Val |
| Arg (R) | lys; gln; asn | Lys |
| Asn (N) | gln; his; asp, lys; arg | Gln |
| Asp (D) | glu; asn | Glu |
| Cys (C) | ser; ala | Ser |
| Gln (Q) | asn; glu | Asn |
| Glu (E) | asp; gln | Asp |
| Gly (G) | Ala | Ala |
| His (H) | asn; gln; lys; arg | Arg |
| Ile (I) | leu; val; met; ala; phe; norleucine | Leu |
| Leu (L) | Norleucine; ile; val; met; ala; phe | Ile |
| Lys (K) | arg; gln; asn | Arg |
| Met (M) | leu; phe; ile | Leu |
| Phe (F) | leu; val; ile; ala; tyr | Tyr |
| Pro (P) | Ala | Ala |
| Ser (S) | Thr | Thr |
| Thr (T) | Ser | Ser |
| Trp (W) | tyr; phe | Tyr |
| Tyr (Y) | trp; phe; thr; ser | Phe |
| Val (V) | ile; leu; met; phe; ala; norleucine | Leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the antibody to improve its stability.

A particularly preferred type of substitutional variant involves substituting one or more hypervariable region residues of a parent antibody (*e*.*g*. a humanized or human antibody). Generally, the resulting variant(s) selected for further development will have improved biological activity or properties relative to the parent antibody from which they are generated. A convenient way for generating such substitutional variants involves affinity maturation using phage display. Briefly, several hypervariable region sites (*e.g*. 6-7 sites) are mutated to generate all possible amino substitutions at each site. The antibodies thus generated are displayed in a monovalent fashion from filamentous phage particles as fusions to the gene III product of M13 packaged within each particle. The phage-displayed variants are then screened for their biological activity (*e.g*. binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and antigen. Such contact residues and neighboring residues are candidates for substitution according to the techniques elaborated herein. Once such variants are generated, the panel of variants is subjected to screening as described herein and antibodies with superior properties in one or more relevant assays may be selected for further development.

In one embodiment, the DR4 antibody may comprise a light and/or heavy chain comprising a variable domain sequence having at least 80%, preferably at least 90%, and more preferably, at least 95% amino acid sequence identity to one or more of the variable domain, hypervariable domain, or framework sequences identified herein for the 4H6 antibody.

The antibodies of the invention include "cross-linked" DR4 antibodies. The term "cross-linked" as used herein refers to binding of at least two IgG molecules together to form one (or single) molecule. The DR4 antibodies may be cross-linked using various linker molecules, preferably the DR4 antibodies are cross-linked using an anti-IgG molecule, complement, chemical modification or molecular engineering. It is appreciated by those skilled in the art that complement has a relatively high affinity to antibody molecules once the antibodies bind to cell surface membrane. Accordingly, it is believed that complement may be used as a cross-linking molecule to link two or more anti-DR4 antibodies bound to cell surface membrane. Among the various murine Ig isotypes, IgM, IgG2a and IgG2b (such as the 1H5, 4G7, and 5G11 antibodies) are known to fix complement. The antibodies described in the Examples below, belonging to the murine IgG2 classes, were thus tested for apoptotic activity in the presence of rabbit complement. The apoptotic activity, *in vitro,* of the cross-linked antibodies (which was comparable to Apo-2L) suggests that complement or IgG-Fc cross-linkers may be useful in inducing oligomerization of such DR4 antibodies for, e.g., apoptosis of cancer cells. Cross-linking of the various other anti-DR4 antibodies is also described in the Examples using either goat anti-mouse IgG Fc or goat anti-human IgG Fc. It is noted that for the *in vivo* studies described in the Examples, apoptotic activity was still observed even though the administered DR4 antibodies had not been cross-linked prior to administration.

The antibodies of the invention may optionally comprise dimeric antibodies, as well as multivalent forms of antibodies. Those skilled in the art may contruct such dimers or multivalent forms by techniques known in the art and using the DR4 antibodies herein.

The antibodies may also comprise monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art. For instance, digestion can be performed using papain. Examples of papain digestion are described in WO 94/29348 published 12/22/94 and U.S. Patent No. 4,342,566. Papain digestion of antibodies typically produces two identical antigen binding fragments, called Fab fragments, each with a single antigen binding site, and a residual Fc fragment. Pepsin treatment yields an F(ab')₂ fragment that has two antigen combining sites and is still capable of cross-linking antigen.

The Fab fragments produced in the antibody digestion also contain the constant domains of the light chain and the first constant domain (CH₁) of the heavy chain. Fab' fragments differ from Fab fragments by the addition of a few residues at the carboxy terminus of the heavy chain CH₁ domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

Single chain Fv fragments may also be produced, such as described in Iliades et al., FEBS Letters, 409:437-441 (1997). Coupling of such single chain fragments using various linkers is described in Kortt et al., Protein Engineering, 10:423-433 (1997).

In addition to the antibodies described above, it is contemplated that chimeric or hybrid antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate.

The DR4 antibodies may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important in order to reduce antigenicity. According to the "best-fit" method, the sequence of the variable domain of a rodent antibody is screened against the entire library of known human variable domain sequences. The human sequence which is closest to that of the rodent is then accepted as the human framework (FR) for the humanized antibody [Sims et al., J. Immunol., 151:2296-2308 (1993); Chothia and Lesk, J. Mol. Biol., 196:901-917 (1987)]. Another method uses a particular framework derived from the consensus sequence of all human antibodies of a particular subgroup of light or heavy chains. The same framework may be used for several different humanized antibodies [Carter et al., Proc. Natl. Acad. Sci. USA, 89:4285-4289 (1992); Presta et al., J. Immunol., 151:2623-2632 (1993)].

It is further important that antibodies be humanized with retention of high affinity for the antigen and other favorable biological properties. To achieve this goal, according to a preferred method, humanized antibodies are prepared by a process of analysis of the parental sequences and various conceptual humanized products using three dimensional models of the parental and humanized sequences. Three dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the consensus and import sequence so that the desired antibody characteristic, such as increased affinity for the target antigen(s), is achieved. In general, the CDR residues are directly and most substantially involved in influencing antigen binding [see, WO 94/04679 published 3 March 1994].

Human monoclonal antibodies may be made via an adaptation of the hybridoma method first described by Kohler and Milstein by using human B lymphocytes as the fusion partner. Human B lymphocytes producing an antibody of interest may, for example, be isolated from a human individual, after obtaining informed consent. For instance, the individual may be producing antibodies against an autoantigen as occurs with certain disorders such as systemic lupus erythematosus (Shoenfeld et al. J. Clin. Invest., 70:205 (1982)), immune-mediated thrombocytopenic purpura (ITP) (Nugent et al. Blood, 70(1):16-22 (1987)), or cancer. Alternatively, or additionally, lymphocytes may be immunized *in vitro.* For instance, one may expose isolated human periperal blood lymphocytes *in vitro* to a lysomotrophic agent (*e.g*. L-leucine-O-methyl ester, L-glutamic acid dimethly ester or L-leucyl-L-leucine-O-methyl ester) (US Patent No. 5,567,610, Borrebaeck et al.); and/or T-cell depleted human peripheral blood lymphocytes may be treated *in vitro* with adjuvants such as 8-mercaptoguanosine and cytokines (US Patent No. 5,229,275, Goroff et al.).

The B lymphocytes recovered from the subject or immunized *in vitro*, are then generally immortalized in order to generate a human monoclonal antibody. Techniques for immortalizing the B lymphocyte include, but are not limited to: (a) fusion of the human B lymphocyte with human, murine myelomas or mouse-human heteromyeloma cells; (b) viral transformation (e.g. with an Epstein-Barr virus; see Nugent *et al*., *supra,* for example); (c) fusion with a lymphoblastoid cell line; or (d) fusion with lymphoma cells.

Lymphocytes may be fused with myeloma cells using a suitable fusing agent, such as polyethylene glycol, to form a hybridoma cell (Goding, Monoclonal Antibodies: Principles and Practice, pp.59-103 (Academic Press, 1986)). The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells. Suitable human myeloma and mouse-human heteromyeloma cell lines have been described (Kozbor, J. Immunol., 133:3001 (1984); Brodeur et al., Monoclonal Antibody Production Techniques and Applications, pp. 51-63 (Marcel Dekker, Inc., New York, 1987)). Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunoabsorbent assay (ELISA).

After hybridoma cells are identified that produce antibodies of the desired specificity, affinity, and/or activity, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. The monoclonal antibodies secreted by the subclones are suitably separated from the culture medium, ascites fluid, or serum by conventional immunoglobulin purification procedures such as, for example, protein A chromatography, gel electrophoresis, dialysis, or affinity chromatography.

Human antibodies may also be generated using a non-human host, such as a mouse, which is capable of producing human antibodies. As noted above, transgenic mice are now available that are capable, upon immunization, of producing a full repertoire of human antibodies in the absence of endogenous immunoglobulin production. For example, it has been described that the homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, *e.g*., Jakobovits et al., Proc. Natl. Acad. Sci. USA, 90:2551 (1993); Jakobovits et al., Nature, 362:255-258 (1993); Bruggermann et al., Year in Immuno., 7:33 (1993); US Patent No. 5,591,669; US Patent No. 5,589,369; and US Patent No. 5,545,807. Human antibodies may also be prepared using SCID-hu mice (Duchosal et al. Nature 355:258-262 (1992)).

In another embodiment, the human antibody may be selected from a human antibody phage display library. The preparation of libraries of antibodies or fragments thereof is well known in the art and any of the known methods may be used to construct a family of transformation vectors which may be introduced into host cells. Libraries of antibody light and heavy chains in phage (Huse et al., Science, 246:1275 (1989)) or of fusion proteins in phage or phagemid can be prepared according to known procedures. See, for example, Vaughan et al., Nature Biotechnology 14:309-314 (1996); Barbas et al., Proc. Natl. Acad. Sci., USA, 88:7978-7982 (1991); Marks et al., J. Mol. Biol., 222:581-597 (1991); Hoogenboom and Winter, J. Mol. Biol., 227:381-388 (1992); Barbas et al., Proc. Natl. Acad. Sci., USA, 89:4457-4461 (1992); Griffiths et al., EMBO Journal, 13:3245-3260 (1994); de Kruif et al., J. Mol. Biol., 248:97-105 (1995); WO 98/05344; WO 98/15833; WO 97/47314; WO 97/44491; WO 97/35196; WO 95/34648; US Patent No. 5,712.089; US Patent No. 5,702,892; US Patent No. 5,427,908; US Patent No. 5,403,484; US Patent No. 5,432,018; US Patent No. 5,270,170; WO 92/06176; WO 99/06587; US Patent No. 5,514,548; WO97/08320; and US Patent No. 5,702,892. The antigen of interest is panned against the phage library using procedures known in the field for selecting phage-antibodies which bind to the target antigen.

The DR4 antibodies, as described herein, will optionally possess one or more desired biological activities or properties. Such DR4 antibodies may include but are not limited to chimeric, humanized, human, and affinity matured antibodies. As described above, the DR4 antibodies may be constructed or engineered using various techniques to achieve these desired activities or properties. In one embodiment, the DR4 antibody will have a DR4 receptor binding affinity of at least 10⁵ M⁻¹, preferably at least in the range of 10⁶ M⁻¹ to 10⁷ M⁻¹, more preferably, at least in the range of 10⁸ M⁻¹ to 10¹² M⁻¹ and even more preferably, at least in the range of 10⁹ M⁻¹ to 10¹² M⁻¹. The binding affinity of the DR4 antibody can be determined without undue experimentation by testing the DR4 antibody in accordance with techniques known in the art, including Scatchard analysis (see Munson et al., supra) and the KinExA^{™} assay (see Example 9). Optionally, the DR4 antibody can be assayed for binding affinity using the KinExA^{™} assay described in Example 9 and determining the binding affinity of the DR4 antibody for the DR4-IgG receptor construct, as described in Example 9.

In another embodiment, the DR4 antibody of the invention may bind the same epitope on DR4 to which Apo-2L binds, or bind an epitope on DR4 which coincides or overlaps with the epitope on DR4 to which Apo-2L binds. The DR4 antibody may also interact in such a way to create a steric conformation which prevents Apo-2 ligand binding to DR4. The epitope binding property of a DR4 antibody of the present invention may be determined using techniques known in the art. For instance, the DR4 antibody may be tested in an *in vitro* assay, such as a competitive inhibition assay, to determine the ability of the DR4 antibody to block or inhibit binding of Apo-2L to DR4. Optionally, the DR4 antibody may be tested in a competitive inhibition assay to determine the ability of the DR4 antibody to inhibit binding of an Apo-2L polypeptide (such as described in Example 17) to a DR4-IgG construct (such as described in Example 1) or to a cell expressing DR4. Optionally, the DR4 antibody will be capable of blocking or inhibiting binding of Apo-2L to DR4 by at least 50%, preferably by at least 75% and even more preferably by at least 90%, which may be determined, by way of example, in an in *vitro* competitive inhibition assay using a soluble form of Apo-2 ligand (TRAIL) and a DR4 ECD-IgG (such as described in Example 1). The epitope binding property of a DR4 antibody may also be determined using *in vitro* assays to test the ability of the DR4 antibody to block Apo-2L induced apoptosis. For example, the DR4 antibody may be tested in the assay described in Example 4 to determine the ability of the DR4 antibody to block Apo-2L induced apoptosis in 9D cells (or other cancer cells expressing DR4 receptor). Optionally, the DR4 antibody will be capable of blocking or inhibiting Apo-2L induced apoptosis in a selected mammalian cancer cell type by at least 50%, preferably by at least 75% and even more preferably, by at least 90% or 95%, which may be determined, for example, in an *in vitro* assay described in Example 4.

In a further embodiment, the DR4 antibody will comprise an agonist antibody having activity comparable to Apo-2 ligand (TRAIL). Preferably, such an agonist DR4 antibody will induce apoptosis in at least one type of cancer or tumor cell line or primary tumor. The apoptotic activity of an agonist DR4 antibody may be determined using known in *vitro* or *in vivo* assays. Examples of such *in vitro* and *in vivo* assays are described in detail in the Examples section below. *In vitro,* apoptotic activity can be determined using known techniques such as Annexin V binding. *In vivo*, apoptotic activity may be determined, e.g., by measuring reduction in tumor burden or volume.

### 3. Bispecific Antibodies

Bispecific antibodies are monoclonal, preferably human or humanized, antibodies that have binding specificities for at least two different antigens. In the present case, one of the binding specificities is for the DR4, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

### 4. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 5. Triabodies

Triabodies are also within the scope of the invention. Such antibodies are described for instance in Iliades et al., supra and Kortt et al., supra.

### 6. Other Modifications

Other modifications of the DR4 antibodies are contemplated herein. The antibodies of the present invention may be modified by conjugating the antibody to a cytotoxic agent (like a toxin molecule) or a prodrug-activating enzyme which converts a prodrug (*e*.*g*. a peptidyl chemotherapeutic agent, see WO81/01145) to an active anti-cancer drug. See, for example, WO 88/07378 and U.S. Patent No. 4,975,278. This technology is also referred to as "Antibody Dependent Enzyme Mediated Prodrug Therapy" (ADEPT).

The enzyme component of the immunoconjugate useful for ADEPT includes any enzyme capable of acting on a prodrug in such a way so as to covert it into its more active, cytotoxic form. Enzymes that are useful in the method
of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; caspases such as caspase-3; D-alanylcarboxypeptidases, useful for converting prodrugs that contain D-amino acid substituents; carbohydrate-cleaving enzymes such as beta-galactosidase and neuraminidase useful for converting glycosylated prodrugs into free drugs; beta-lactamase useful for converting drugs derivatized with beta-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, *e*.*g*., Massey, Nature 328: 457-458 (1987)). Antibody-abzyme conjugates can be prepared as described herein for delivery of the abzyme to a tumor cell population.

The enzymes can be covalently bound to the antibodies by techniques well known in the art such as the use of heterobifunctional crosslinking reagents. Alternatively, fusion proteins comprising at least the antigen binding region of an antibody of the invention linked to at least a functionally active portion of an enzyme of the invention can be constructed using recombinant DNA techniques well known in the art (see, *e*.*g*., Neuberger et al., Nature, 312: 604-608 (1984).

Further antibody modifications are contemplated. For example, the antibody may be linked to one of a variety of nonproteinaceous polymers, *e*.*g*., polyethylene glycol, polypropylene glycol, polyoxyalkylenes, or copolymers of polyethylene glycol and polypropylene glycol. The antibody also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Oslo, A., Ed., (1980). To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (*e*.*g*., IgG₁, IgG₂, IgG₃, or IgG₄) that is responsible for increasing the *in vivo* serum half-life of the IgG molecule.

### 7. Recombinant Methods

The invention also provides isolated nucleic acids encoding DR4 antibodies as disclosed herein, vectors and host cells comprising the nucleic acid, and recombinant techniques for the production of the antibody.

For recombinant production of the antibody, the nucleic acid encoding it is isolated and inserted into a replicable vector for further cloning (amplification of the DNA) or for expression. DNA encoding the antibody is readily isolated and sequenced using conventional procedures (*e*.*g.,* by using oligonucleotide probes that are capable of binding specifically to genes encoding the antibody). Many vectors are available. The vector components generally include, but are not limited to, one or more of the following: a signal sequence, an origin of replication, one or more marker genes, an enhancer element, a promoter, and a transcription termination sequence.

The methods herein include methods for the production of chimeric or recombinant anti-DR4 antibodies which comprise the steps of providing a vector comprising a DNA sequence encoding an anti-DR4 antibody light chain or heavy chain (or both a light chain and a heavy chain), transfecting or transforming a host cell with the vector, and culturing the host cell(s) under conditions sufficient to produce the recombinant anti-DR4 antibody product. In one embodiment, it is contemplated that the light chain and/or heavy chain of the recombinantly produced antibody may comprise all or part of the variable domains of the murine 4H6 antibody disclosed here.

### (i) Signal sequence component

The anti-DR4 antibody of this invention may be produced recombinantly not only directly, but also as a fusion polypeptide with a heterologous polypeptide, which is preferably a signal sequence or other polypeptide having a specific cleavage site at the N-terminus of the mature protein or polypeptide. The heterologous signal sequence selected preferably is one that is recognized and processed (*i*.*e*., cleaved by a signal peptidase) by the host cell. For prokaryotic host cells that do not recognize and process the native antibody signal sequence, the signal sequence is substituted by a prokaryotic signal sequence selected, for example, from the group of the alkaline phosphatase, penicillinase, lpp, or heat-stable enterotoxin II leaders. For yeast secretion the native signal sequence may be substituted by, *e*.*g*., the yeast invertase leader, α factor leader (including *Saccharomyces* and *Kluyveromyces* α-factor leaders), or acid phosphatase leader, the *C*. *albicans* glucoamylase leader, or the signal described in WO 90/13646. In mammalian cell expression, mammalian signal sequences as well as viral secretory leaders, for example, the herpes simplex gD signal, are available.

The DNA for such precursor region is ligated in reading frame to DNA encoding the antibody.

### (ii) Origin of replication component

Both expression and cloning vectors contain a nucleic acid sequence that enables the vector to replicate in one or more selected host cells. Generally, in cloning vectors this sequence is one that enables the vector to replicate independently of the host chromosomal DNA, and includes origins of replication or autonomously replicating sequences. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells. Generally, the origin of replication component is not needed for mammalian expression vectors (the SV40 origin may typically be used only because it contains the early promoter).

### (iii) Selection gene component

Expression and cloning vectors may contain a selection gene, also termed a selectable marker. Typical selection genes encode proteins that (a) confer resistance to antibiotics or other toxins, *e*.*g*., ampicillin, neomycin, methotrexate, or tetracycline, (b) complement auxotrophic deficiencies, or (c) supply critical nutrients not available from complex media, *e*.*g*., the gene encoding D-alanine racemase for *Bacilli*.

One example of a selection scheme utilizes a drug to arrest growth of a host cell. Those cells that are successfully transformed with a heterologous gene produce a protein conferring drug resistance and thus survive the selection regimen. Examples of such dominant selection use the drugs neomycin, mycophenolic acid and hygromycin.

Another example of suitable selectable markers for mammalian cells are those that enable the identification of cells competent to take up the antibody nucleic acid, such as DHFR, thymidine kinase, metallothionein-I and -II, preferably primate metallothionein genes, adenosine deaminase, ornithine decarboxylase, *etc*.

For example, cells transformed with the DHFR selection gene are first identified by culturing all of the transformants in a culture medium that contains methotrexate (Mtx), a competitive antagonist of DHFR. An appropriate host cell when wild-type DHFR is employed is the Chinese hamster ovary (CHO) cell line deficient in DHFR activity.

Alternatively, host cells (particularly wild-type hosts that contain endogenous DHFR) transformed or co-transformed with DNA sequences encoding the anti-DR4 antibody, wild-type DHFR protein, and another selectable marker such as aminoglycoside 3'-phosphotransferase (APH) can be selected by cell growth in medium containing a selection agent for the selectable marker such as an aminoglycosidic antibiotic, *e*.*g*., kanamycin, neomycin, or G418. See U.S. Patent No. 4,965,199.

A suitable selection gene for use in yeast is the *trp*1 gene present in the yeast plasmid YRp7 (Stinchcomb et al., Nature, 282:39 (1979)). The *trp*1 gene provides a selection marker for a mutant strain of yeast lacking the ability to grow in tryptophan, for example, ATCC No. 44076 or PEP4-1. Jones, Genetics, 85:12 (1977). The presence of the *trp*1 lesion in the yeast host cell genome then provides an effective environment for detecting transformation by growth in the absence of tryptophan. Similarly, Leu2-deficient yeast strains (ATCC 20,622 or 38,626) are complemented by known plasmids bearing the *Leu2* gene.

In addition, vectors derived from the 1.6 µm circular plasmid pKD1 can be used for transformation of *Kluyveromyces* yeasts. Alternatively, an expression system for large-scale production of recombinant calf chymosin was reported for *K. lactis.* Van den Berg, Bio/Technology, 8:135 (1990). Stable multi-copy expression vectors for secretion of mature recombinant human serum albumin by industrial strains of *Kluyveromyces* have also been disclosed. Fleer et al., Bio/Technology, 9:968-975 (1991).

### (iv) Promoter component

Expression and cloning vectors usually contain a promoter that is recognized by the host organism and is operably linked to the antibody nucleic acid. Promoters suitable for use with prokaryotic hosts include the phoA promoter, β-lactamase and lactose promoter systems, alkaline phosphatase, a tryptophan (trp) promoter system, and hybrid promoters such as the tac promoter. However, other known bacterial promoters are suitable. Promoters for use in bacterial systems also will contain a Shine-Dalgarno (S.D.) sequence operably linked to the DNA encoding the anti-DR4 antibody.

Promoter sequences are known for eukaryotes. Virtually all eukaryotic genes have an AT-rich region located approximately 25 to 30 bases upstream from the site where transcription is initiated. Another sequence found 70 to 80 bases upstream from the start of transcription of many genes is a CNCAAT region where N may be any nucleotide. At the 3' end of most eukaryotic genes is an AATAAA sequence that may be the signal for addition of the poly A tail to the 3' end of the coding sequence. All of these sequences are suitably inserted into eukaryotic expression vectors.

Examples of suitable promoting sequences for use with yeast hosts include the promoters for 3-phosphoglycerate kinase or other glycolytic enzymes, such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. Yeast enhancers also are advantageously used with yeast promoters.

Anti-DR4 antibody transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and most preferably Simian Virus 40 (SV40), from heterologous mammalian promoters, *e*.*g*., the actin promoter or an immunoglobulin promoter, from heat-shock promoters, provided such promoters are compatible with the host cell systems.

The early and late promoters of the SV40 virus are conveniently obtained as an SV40 restriction fragment that also contains the SV40 viral origin of replication. The immediate early promoter of the human cytomegalovirus is conveniently obtained as a HindIII E restriction fragment. A system for expressing DNA in mammalian hosts using the bovine papilloma virus as a vector is disclosed in U.S. Patent No. 4,419,446. A modification of this system is described in U.S. Patent No. 4,601,978. See also Reyes et al., Nature 297:598-601 (1982) on expression of human β-interferon cDNA in mouse cells under the control of a thymidine kinase promoter from herpes simplex virus. Alternatively, the rous sarcoma virus long terminal repeat can be used as the promoter.

### (v) Enhancer element component

Transcription of a DNA encoding the anti-DR4 antibody of this invention by higher eukaryotes is often increased by inserting an enhancer sequence into the vector. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. See also Yaniv, Nature 297:17-18 (1982) on enhancing elements for activation of eukaryotic promoters. The enhancer may be spliced into the vector at a position 5' or 3' to the antibody-encoding sequence, but is preferably located at a site 5' from the promoter.

### (vi) Transcription termination component

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding the multivalent antibody. One useful transcription termination component is the bovine growth hormone polyadenylation region. See WO94/11026 and the expression vector disclosed therein.

### (vii) Selection and transformation of host cells

Suitable host cells for cloning or expressing the DNA in the vectors herein are the prokaryote, yeast, or higher eukaryote cells described above. Suitable prokaryotes for this purpose include eubacteria, such as Gram-negative or Gram-positive organisms, for example, Enterobacteriaceae such as *Escherichia, e.g., E. coli*, *Enterobacter, Erwinia, Klebsiella, Proteus, Salmonella, e.g., Salmonella typhimurium, Serratia, e*.*g*., *Serratia marcescans,* and *Shigella,* as well as *Bacilli* such as *B*. *subtilis* and *B*. *licheniformis* (*e*.*g., B*. *licheniformis* 41P disclosed in DD 266,710 published 12 April 1989), *Pseudomonas* such as *P*. *aeruginosa,* and *Streptomyces*. One preferred *E*. *coli* cloning host is *E*. *coli* 294 (ATCC 31,446), although other strains such as *E*. *coli* B, *E*. *coli* X1776 (ATCC 31,537), and *E*. *coli* W3110 (ATCC 27,325) are suitable. These examples are illustrative rather than limiting.

In addition to prokaryotes, eukaryotic microbes such as filamentous fungi or yeast are suitable cloning or expression hosts for DR4 antibody-encoding vectors. *Saccharomyces cerevisiae,* or common baker's yeast, is the most commonly used among lower eukaryotic host microorganisms. However, a number of other genera, species, and strains are commonly available and useful herein, such as *Schizosaccharomyces pombe; Kluyveromyces* hosts such as, *e.g., K*. *lactis, K*. *fragilis* (ATCC 12,424), *K*. *bulgaricus* (ATCC 16,045), *K*. *wickeramii* (ATCC 24,178), *K*. *waltii* (ATCC 56,500), *K*. *drosophilarum* (ATCC 36,906), *K . thermotolerans,* and *K*. *marxianus; yarrowia* (EP 402,226); *Pichia pastoris* (EP 183,070); *Candida; Trichoderma reesia* (EP 244,234); *Neurospora crassa; Schwanniomyces* such as *Schwanniomyces occidentalis;* and filamentous fungi such as, *e*.*g*., *Neurospora, Penicillium, Tolypocladium,* and *Aspergillus* hosts such as *A*. *nidulans* and *A*. *niger.*

Suitable host cells for the expression of glycosylated antibody are derived from multicellular organisms. Examples of invertebrate cells include plant and insect cells. Numerous baculoviral strains and variants and corresponding permissive insect host cells from hosts such as *Spodoptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, *e*.*g*., the L-1 variant of *Autographa californica* NPV and the Bm-5 strain of *Bombyx mori* NPV, and such viruses may be used as the virus herein according to the present invention, particularly for transfection of *Spodoptera frugiperda* cells.

Plant cell cultures of cotton, corn, potato, soybean, petunia, tomato, and tobacco can also be utilized as hosts.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/- DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; a human hepatoma line (Hep G2); and myeloma or lymphoma cells (*e*.*g*. Y0, J558L, P3 and NS0 cells; (see US Patent No. 5,807,715).

Host cells are transformed with the above-described expression or cloning vectors for antibody production and cultured in conventional nutrient media modified as appropriate for inducing promoters, selecting transformants, or amplifying the genes encoding the desired sequences.

### (viii) Culturing the host cells

The host cells used to produce the antibody of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem.102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN^{™} drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### (ix) Purification

When using recombinant techniques, the antibody can be produced intracellularly, in the periplasmic space, or directly secreted into the medium. If the antibody is produced intracellularly, as a first step, the particulate debris, either host cells or lysed fragments, is removed, for example, by centrifugation or ultrafiltration. Carter et al., Bio/Technology 10:163-167 (1992) describe a procedure for isolating antibodies which are secreted to the periplasmic space of *E*. *coli*. Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc region that is present in the antibody. Protein A can be used to purify antibodies that are based on human γ1, γ2, or γ4 heavy chains (Lindmark et al., J. Immunol. Meth. 62:1-13 (1983)). Protein G is recommended for all mouse isotypes and for human γ3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a C_{H}3 domain, the Bakerbond ABX^{™} resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE^{™} chromatography on an anion or cation exchange resin (such as a polyaspartic acid column), chromatofocusing, SDS-PAGE, and ammonium sulfate precipitation are also available depending on the antibody to be recovered.

### B. Uses for DR4 Antibodies

The DR4 antibodies of the invention have various utilities. For example, DR4 agonistic antibodies may be employed in medical uses for treating pathological conditions in mammals such as cancer or immune-related diseases. In the medical uses, the DR4 antibody, preferably an agonistic antibody, is administered to a mammal, alone or in combination with still other therapeutic agents or techniques.

Diagnosis in mammals of the various pathological conditions described herein can be made by the skilled practitioner. Diagnostic techniques are available in the art which allow, e.g., for the diagnosis or detection of cancer or immune related disease in a mammal. For instance, cancers may be identified through techniques, including but not limited to, palpation, blood analysis, x-ray, NMR and the like. Immune related diseases can also be readily identified. In systemic lupus erythematosus, the central mediator of disease is the production of auto-reactive antibodies to self proteins/tissues and the subsequent generation of immune-mediated inflammation. Multiple organs and systems are affected clinically including kidney, lung, musculoskeletal system, mucocutaneous, eye, central nervous system, cardiovascular system, gastrointestinal tract, bone marrow and blood.

Rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, interstitial pneumonitis with pulmonary fibrosis, keratoconjunctivitis sicca, and rheumatoid nodules.

Juvenile chronic arthritis is a chronic idiopathic inflammatory disease which begins often at less than 16 years of age. Its phenotype has some similarities to RA; some patients which are rheumatoid factor positive are classified as juvenile rheumatoid arthritis. The disease is sub-classified into three major categories: pauciarticular, polyarticular, and systemic. The arthritis can be severe and is typically destructive and leads to joint ankylosis and retarded growth. Other manifestations can include chronic anterior uveitis and systemic amyloidosis.

Spondyloarthropathies are a group of disorders with some common clinical features and the common association with the expression of HLA-B27 gene product. The disorders include: ankylosing sponylitis, Reiter's syndrome (reactive arthritis), arthritis associated with inflammatory bowel disease, spondylitis associated with psoriasis, juvenile onset spondyloarthropathy and undifferentiated spondyloarthropathy. Distinguishing features include sacroileitis with or without spondylitis; inflammatory asymmetric arthritis; association with HLA-B27 (a serologically defined allele of the HLA-B locus of class I MHC); ocular inflammation, and absence of autoantibodies associated with other rheumatoid disease. The cell most implicated as key to induction of the disease is the CD8+ T lymphocyte, a cell which targets antigen presented by class I MHC molecules. CD8+ T cells may react against the class I MHC allele HLA-B27 as if it were a foreign peptide expressed by MHC class I molecules. It has been hypothesized that an epitope of HLA-B27 may mimic a bacterial or other microbial antigenic epitope and thus induce a CD8+ T cells response.

Systemic sclerosis (scleroderma) has an unknown etiology. A hallmark of the disease is induration of the skin; likely this is induced by an active inflammatory process. Scleroderma can be localized or systemic; vascular lesions are common and endothelial cell injury in the microvasculature is an early and important event in the development of systemic sclerosis; the vascular injury may be immune mediated. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. Other organs involved include: the gastrointestinal tract: smooth muscle atrophy and fibrosis resulting in abnormal peristalsis/motility; kidney: concentric subendothelial intimal proliferation affecting small arcuate and interlobular arteries with resultant reduced renal cortical blood flow, results in proteinuria, azotemia and hypertension; skeletal muscle: atrophy, interstitial fibrosis; inflammation; lung: interstitial pneumonitis and interstitial fibrosis; and heart: contraction band necrosis, scarring/fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components, proteins and RNA's, involved in protein synthesis.

Sjogren's syndrome is due to immune-mediated inflammation and subsequent functional destruction of the tear glands and salivary glands. The disease can be associated with or accompanied by inflammatory connective tissue diseases. The disease is associated with autoantibody production against Ro and La antigens, both of which are small RNA-protein complexes. Lesions result in keratoconjunctivitis sicca, xerostomia, with other manifestations or associations including bilary cirrhosis, peripheral or sensory neuropathy, and palpable purpura.

Systemic vasculitis are diseases in which the primary lesion is inflammation and subsequent damage to blood vessels which results in ischemia/necrosis/degeneration to tissues supplied by the affected vessels and eventual end-organ dysfunction in some cases. Vasculitides can also occur as a secondary lesion or sequelae to other immune-inflammatory mediated diseases such as rheumatoid arthritis, systemic sclerosis, etc., particularly in diseases also associated with the formation of immune complexes. Diseases in the primary systemic vasculitis group include: systemic necrotizing vasculitis: polyarteritis nodosa, allergic angiitis and granulomatosis, polyangiitis; Wegener's granulomatosis; lymphomatoid granulomatosis; and giant cell arteritis. Miscellaneous vasculitides include: mucocutaneous lymph node syndrome (MLNS or Kawasaki's disease), isolated CNS vasculitis, Behet's disease, thromboangiitis obliterans (Buerger's disease) and cutaneous necrotizing venulitis. The pathogenic mechanism of most of the types of vasculitis listed is believed to be primarily due to the deposition of immunoglobulin complexes in the vessel wall and subsequent induction of an inflammatory response either via ADCC, complement activation, or both.

Sarcoidosis is a condition of unknown etiology which is characterized by the presence of epithelioid granulomas in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

In autoimmune thrombocytopenia including thrombocytopenic purpura, and immune-mediated thrombocytopenia in other clinical settings, platelet destruction/removal occurs as a result of either antibody or complement attaching to platelets and subsequent removal by complement lysis, ADCC or FC-receptor mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase).

Type I diabetes mellitus or insulin-dependent diabetes is the autoimmune destruction of pancreatic islet β cells; this destruction is mediated by auto-antibodies and auto-reactive T cells. Antibodies to insulin or the insulin receptor can also produce the phenotype of insulin-non-responsiveness.

Immune mediated renal diseases, including glomerulonephritis and tubulointerstitial nephritis, are the result of antibody or T lymphocyte mediated injury to renal tissue either directly as a result of the production of autoreactive antibodies or T cells against renal antigens or indirectly as a result of the deposition of antibodies and/or immune complexes in the kidney that are reactive against other, non-renal antigens. Thus other immune-mediated diseases that result in the formation of immune-complexes can also induce immune mediated renal disease as an indirect sequelae. Both direct and indirect immune mechanisms result in inflammatory response that produces/induces lesion development in renal tissues with resultant organ function impairment and in some cases progression to renal failure. Both humoral and cellular immune mechanisms can be involved in the pathogenesis of lesions.

Demyelinating diseases of the central and peripheral nervous systems, including Multiple Sclerosis; idiopathic demyelinating polyneuropathy or Guillain-Barr syndrome; and Chronic Inflammatory Demyelinating Polyneuropathy, are believed to have an autoimmune basis and result in nerve demyelination as a result of damage caused to oligodendrocytes or to myelin directly. In MS there is evidence to suggest that disease induction and progression is dependent on T lymphocytes. Multiple Sclerosis is a demyelinating disease that is T lymphocyte-dependent and has either a relapsing-remitting course or a chronic progressive course. The etiology is unknown; however, viral infections, genetic predisposition, environment, and autoimmunity all contribute. Lesions contain infiltrates of predominantly T lymphocyte mediated, microglial cells and infiltrating macrophages; CD4+T lymphocytes are the predominant cell type at lesions. The mechanism of oligodendrocyte cell death and subsequent demyelination is not known but is likely T lymphocyte driven.

Inflammatory and Fibrotic Lung Disease, including Eosinophilic Pneumonias; Idiopathic Pulmonary Fibrosis, and Hypersensitivity Pneumonitis may involve a disregulated immune-inflammatory response. Inhibition of that response would be of therapeutic benefit.

Autoimmune or Immune-mediated Skin Disease including Bullous Skin Diseases, Erythema Multiforme, and Contact Dermatitis are mediated by auto-antibodies, the genesis of which is T lymphocyte-dependent.

Psoriasis is a T lymphocyte-mediated inflammatory disease. Lesions contain infiltrates of T lymphocytes, macrophages and antigen processing cells, and some neutrophils.

Allergic diseases, including asthma; allergic rhinitis; atopic dermatitis; food hypersensitivity; and urticaria are T lymphocyte dependent. These diseases are predominantly mediated by T lymphocyte induced inflammation, IgE mediated-inflammation or a combination of both.

Transplantation associated diseases, including Graft rejection and Graft-Versus-Host-Disease (GVHD) are T lymphocyte-dependent; inhibition of T lymphocyte function is ameliorative.

Other diseases in which intervention of the immune and/or inflammatory response have benefit are Infectious disease including but not limited to viral infection (including but not limited to AIDS, hepatitis A, B, C, D, E) bacterial infection, fungal infections, and protozoal and parasitic infections (molecules (or derivatives/agonists) which stimulate the MLR can be utilized therapeutically to enhance the immune response to infectious agents), diseases of immunodeficiency (molecules/derivatives/agonists) which stimulate the MLR can be utilized therapeutically to enhance the immune response for conditions of inherited, acquired, infectious induced (as in HIV infection), or iatrogenic (i.e. as from chemotherapy) immunodeficiency), and neoplasia.

The antibody is preferably administered to the mammal in a carrier; preferably a pharmaceutically-acceptable carrier. Suitable carriers and their formulations are described in Remington's Pharmaceutical Sciences, 16th ed., 1980, Mack Publishing Co., edited by Oslo et al. Typically, an appropriate amount of a pharmaceutically-acceptable salt is used in the formulation to render the formulation isotonic. Examples of the carrier include saline, Ringer's solution and dextrose solution. The pH of the solution is preferably from about 5 to about 8, and more preferably from about 7 to about 7.5. Further carriers include sustained release preparations such as semipermeable matrices of solid hydrophobic polymers containing the antibody, which matrices are in the form of shaped articles, e.g., films, liposomes or microparticles. It will be apparent to those persons skilled in the art that certain carriers may be more preferable depending upon, for instance, the route of administration and concentration of antibody being administered.

The antibody can be administered to the mammal by injection (e.g., intravenous, intraperitoneal, subcutaneous, intramuscular, intraportal), or by other methods such as infusion that ensure its delivery to the bloodstream in an effective form. The antibody may also be administered by isolated perfusion techniques, such as isolated tissue perfusion, to exert local therapeutic effects. Local or intravenous injection is preferred.

Effective dosages and schedules for administering the antibody may be determined empirically, and making such determinations is within the skill in the art. Those skilled in the art will understand that the dosage of antibody that must be administered will vary depending on, for example, the mammal which will receive the antibody, the route of administration, the particular type of antibody used and other drugs being administered to the mammal. Guidance in selecting appropriate doses for antibody is found in the literature on therapeutic uses of antibodies, e.g., Handbook of Monoclonal Antibodies, Ferrone et al., eds., Noges Publications, Park Ridge, N.J., (1985) ch. 22 and pp. 303-357; Smith et al., Antibodies in Human Diagnosis and Therapy, Haber et al., eds., Raven Press, New York (1977) pp. 365-389. A typical daily dosage of the antibody used alone might range from about 1 µg/kg to up to 100 mg/kg of body weight or more per day, depending on the factors mentioned above.

The antibody may also be administered to the mammal in combination with effective amounts of one or more other therapeutic agents. The one or more other therapeutic agents or therapies may include, but are not limited to, chemotherapy (chemotherapeutic agents), radiation therapy, immunoadjuvants, growth inhibitory agents, cytotoxic agents, and cytokines. Other agents known to induce apoptosis in mammalian cells may also be employed, and such agents include TNF-alpha, TNF-beta, CD30 ligand, 4-1BB ligand and Apo-2 ligand, as well as other antibodies which can induce apoptosis. The one or more other therapies may include therapeutic antibodies (other than the DR4 antibody), and such antibodies may include anti-Her receptor antibodies (such as Herceptin^{™} ), anti-VEGF antibodies, and antibodies against other receptors for Apo-2 ligand, such as anti-Apo-2 (DR5) antibodies.

Chemotherapies contemplated by the invention include chemical substances or drugs which are known in the art and are commercially available, such as Doxorubicin, 5-Fluorouracil, etoposide, camptothecin, Leucovorin, Cytosine arabinoside, Cyclophosphamide, Thiotepa, Busulfan, Cytoxin, Taxol, Methotrexate, Cisplatin, Melphalan, Vinblastine and Carboplatin. Preparation and dosing schedules for such chemotherapy may be used according to manufacturer's instructions or as determined empirically by the skilled practitioner. Preparation and dosing schedules for such chemotherapy are also described in Chemotherapy Service Ed., M.C. Perry, Williams & Wilkins, Baltimore, MD (1992).

The chemotherapy is preferably administered in a pharmaceutically-acceptable carrier, such as those described above. The mode of administration of the chemotherapy may be the same as employed for the DR4 antibody or it may be administered to the mammal via a different mode. For example, the DR4 antibody may be injected while the chemotherapy is administered orally to the mammal.

Radiation therapy can be administered to the mammal according to protocols commonly employed in the art and known to the skilled artisan. Such therapy may include cesium, iridium, iodine or cobalt radiation. The radiation therapy may be whole body radiation, or may be directed locally to a specific site or tissue in or on the body. Typically, radiation therapy is administered in pulses over a period of time from about 1 to about 2 weeks. The radiation therapy may, however, be administered over longer periods of time. Optionally, the radiation therapy may be administered as a single dose or as multiple, sequential doses.

The antibody may be administered sequentially or concurrently with the one or more other therapeutic agents. The amounts of antibody and therapeutic agent depend, for example, on what type of drugs are used, the pathological condition being treated, and the scheduling and routes of administration but would generally be less than if each were used individually.

Following administration of antibody to the mammal, the mammal's physiological condition can be monitored in various ways well known to the skilled practitioner. It is contemplated that the antagonist or blocking DR4 antibodies may also be used in therapy. For example, a DR4 antibody could be administered to a mammal (such as described above) to block DR4 receptor binding to Apo-2L, thus increasing the bioavailability of Apo-2L administered during Apo-2L therapy to induce apoptosis in cancer cells.

The therapeutic effects of the DR4 antibodies of the invention can be examined in *in vitro* assays and using *in vivo* animal models. A variety of well known animal models can be used to further understand the role of the DR4 antibodies identified herein in the development and pathogenesis of for instance, immune related disease or cancer, and to test the efficacy of the candidate therapeutic agents. The *in vivo* nature of such models makes them particularly predictive of responses in human patients. Animal models of immune related diseases include both non-recombinant and recombinant (transgenic) animals. Non-recombinant animal models include, for example, rodent, e.g., murine models. Such models can be generated by introducing cells into syngeneic mice using standard techniques, e.g. subcutaneous injection, tail vein injection, spleen implantation, intraperitoneal implantation, and implantation under the renal capsule.

Animal models, for example, for graft-versus-host disease are known. Graft-versus-host disease occurs when immunocompetent cells are transplanted into immunosuppressed or tolerant patients. The donor cells recognize and respond to host antigens. The response can vary from life threatening severe inflammation to mild cases of diarrhea and weight loss. Graft-versus-host disease models provide a means of assessing T cell reactivity against MHC antigens and minor transplant antigens. A suitable procedure is described in detail in Current Protocols in Immunology, unit 4.3.

An animal model for skin allograft rejection is a means of testing the ability of T cells to mediate *in vivo* tissue destruction which is indicative of and a measure of their role in anti-viral and tumor immunity. The most common and accepted models use murine tail-skin grafts. Repeated experiments have shown that skin allograft rejection is mediated by T cells, helper T cells and killer-effector T cells, and not antibodies. [Auchincloss, H. Jr. and Sachs, D. H., Fundamental Immunology, 2nd ed., W. E. Paul ed. , Raven Press, NY, 1989, 889-992]. A suitable procedure is described in detail in Current Protocols in Immunology, unit 4.4. Other transplant rejection models which can be used to test the compositions of the invention are the allogeneic heart transplant models described by Tanabe, M. et al., Transplantation, (1994) 58:23 and Tinubu, S. A. et al., J. Immunol., (1994) 4330-4338.

Animal models for delayed type hypersensitivity provides an assay of cell mediated immune function as well. Delayed type hypersensitivity reactions are a T cell mediated in vivo immune response characterized by inflammation which does not reach a peak until after a period of time has elapsed after challenge with an antigen. These reactions also occur in tissue specific autoimmune diseases such as multiple sclerosis (MS) and experimental autoimmune encephalomyelitis (EAE, a model for MS). A suitable procedure is described in detail in Current Protocols in Immunology, unit 4.5.

An animal model for arthritis is collagen-induced arthritis. This model shares clinical, histological and immunological characteristics of human autoimmune rheumatoid arthritis and is an acceptable model for human autoimmune arthritis. Mouse and rat models are characterized by synovitis, erosion of cartilage and subchondral bone. The DR4 antibodies of the invention can be tested for activity against autoimmune arthritis using the protocols described in Current Protocols in Immunology, above, units 15.5. See also the model using a monoclonal antibody to CD18 and VLA-4 integrins described in Issekutz, A. C. et al., Immunology, (1996) 88:569.

A model of asthma has been described in which antigen-induced airway hyper-reactivity, pulmonary eosinophilia and inflammation are induced by sensitizing an animal with ovalbumin and then challenging the animal with the same protein delivered by aerosol. Several animal models (guinea pig, rat, non-human primate) show symptoms similar to atopic asthma in humans upon challenge with aerosol antigens. Murine models have many of the features of human asthma. Suitable procedures to test the compositions of the invention for activity and effectiveness in the treatment of asthma are described by Wolyniec, W. W. et al., Am. J. Respir. Cell Mol. Biol., (1998) 18:777 and the references cited therein.

Additionally, the DR4 antibodies of the invention can be tested on animal models for psoriasis like diseases. The DR4 antibodies of the invention can be tested in the scid/scid mouse model described by Schon, M. P. et al., Nat. Med., (1997) 3:183, in which the mice demonstrate histopathologic skin lesions resembling psoriasis. Another suitable model is the human skin/scid mouse chimera prepared as described by Nickoloff, B. J. et al., Am. J. Path., (1995) 146:580.

Various animal models are well known for testing anti-cancer activity of a candidate therapeutic composition. These include human tumor xenografting into athymic nude mice or scid/scid mice, or genetic murine tumor models such as p53 knockout mice.

Recombinant (transgenic) animal models can be engineered by introducing the coding portion of the molecules identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, e.g. baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (e.g., Van der Putten et al., Proc. Natl. Acad. Sci. USA, 82, 6148-615 [1985]); gene targeting in embryonic stem cells (Thompson et al., Cell, 56, 313-321 [1989]); electroporation of embryos (Lo, Mol. Cel. Biol., 3, 1803-1814 [1983]); sperm-mediated gene transfer (Lavitrano et al., Cell, 57, 717-73 [1989]). For review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, e.g., head-to-head or head-to-tail tandems. Selective introduction of a transgene into a particular cell type is also possible by following, for example, the technique of Lasko et al., Proc. Natl. Acad. Sci. USA, 89, 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry. The animals may be further examined for signs of immune disease pathology, for example by histological examination to determine infiltration of immune cells into specific tissues or for the presence of cancerous or malignant tissue.

Alternatively, "knock out" animals can be constructed which have a defective or altered gene encoding a polypeptide identified herein, as a result of homologous recombination between the endogenous gene encoding the polypeptide and altered genomic DNA encoding the same polypeptide introduced into an embryonic cell of the animal. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

In another embodiment of the invention, methods for employing the antibody in diagnostic assays are provided. For instance, the antibodies may be employed in diagnostic assays to detect expression or overexpression of DR4 in specific cells and tissues. Various diagnostic assay techniques known in the art may be used, such as *in vivo* imaging assays, *in vitro* competitive binding assays, direct or indirect sandwich assays and immunoprecipitation assays conducted in either heterogeneous or homogeneous phases [Zola, Monoclonal Antibodies: A Manual of Techniques, CRC Press, Inc. (1987) pp. 147-158]. The antibodies used in the diagnostic assays can be labeled with a detectable moiety. The detectable moiety should be capable of producing, either directly or indirectly, a detectable signal. For example, the detectable moiety may be a radioisotope, such as ³H_{,} ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter et al., Nature, 144:945 (1962); David et al., Biochemistry, 13:1014-1021 (1974); Pain et al., J. Immunol. Meth., 40:219-230 (1981); and Nygren, J. Histochem. and Cytochem., 30:407-412 (1982).

DR4 antibodies also are useful for the affinity purification of DR4 from recombinant cell culture or natural sources. In this process, the antibodies against DR4 are immobilized on a suitable support, such a Sephadex resin or filter paper, using methods well known in the art. The immobilized antibody then is contacted with a sample containing the DR4 to be purified, and thereafter the support is washed with a suitable solvent that will remove substantially all the material in the sample except the DR4, which is bound to the immobilized antibody. Finally, the support is washed with another suitable solvent that will release the DR4 from the antibody.

In a further embodiment of the invention, there are provided articles of manufacture and kits containing materials useful for treating pathological conditions or detecting or purifying DR4. The article of manufacture comprises a container with a label. Suitable containers include, for example, bottles, vials, and test tubes. The containers may be formed from a variety of materials such as glass or plastic. The container holds a composition having an active agent which is effective for treating pathological conditions or for detecting or purifying DR4. The active agent in the composition is a DR4 antibody and preferably, comprises monoclonal antibodies specific for DR4. The label on the container indicates that the composition is used for treating pathological conditions or detecting or purifying DR4, and may also indicate directions for either *in vivo* or *in vitro* use, such as those described above.

The following examples are offered for illustrative purposes only, and are not intended to limit the scope of the present invention in any way. Some of the examples are provided by way of reference.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, Virginia.

### EXAMPLE 1

### Expression of DR4 ECD as an Immunoadhesin

A soluble DR4 ECD immunoadhesin construct was prepared. A mature DR4 ECD sequence (amino acids 1-218 shown in Fig. 1) was cloned into a pCMV-1 Flag vector (Kodak) downstream of the Flag signal sequence and fused to the CH1, hinge and Fc region of human immunoglobulin G₁ heavy chain as described previously [Aruffo et al., Cell, 61:1303-1313 (1990)]. The immunoadhesin was expressed by transient transfection into human 293 cells and purified from cell supernatants by protein A affinity chromatography, as described by Ashkenazi et al., supra.

### EXAMPLE 2

### Preparation of Monoclonal Antibodies Specific for DR4

Balb/c mice (obtained from Charles River Laboratories) were immunized by injecting 0.5 (µg/50 µl of a DR4 ECD immunoadhesin protein (as described in Example 1 above)(diluted in MPL-TDM adjuvant purchased from Ribi Immunochemical Research Inc., Hamilton, MT) 11 times into each hind foot pad at 3-4 day intervals.

Three days after the final boost, popliteal lymph nodes were removed from the mice and a single cell suspension was prepared in DMEM media (obtained from Biowhitakker Corp.) supplemented with 1% penicillin-streptomycin. The lymph node cells were then fused with murine myeloma cells P3X63AgU.1 (ATCC CRL 1597) using 35% polyethylene glycol and cultured in 96-well culture plates. Hybridomas resulting from the fusion were selected in HAT medium. Ten days after the fusion, hybridoma culture supernatants were screened in an ELISA to test for the presence of monoclonal antibodies binding to the DR4 ECD immunoadhesin protein (described in Example 1).

In the ELISA, 96-well microtiter plates (Maxisorp; Nunc, Kamstrup, Denmark) were coated by adding 50 µl of 2 µg/ml goat anti-human IgG Fc (purchased from Cappel Laboratories) in PBS to each well and incubating at 4°C overnight. The plates were then washed three times with wash buffer (PBS containing 0.05% Tween 20). The wells in the microtiter plates were then blocked with 200 µl of 2.0% bovine serum albumin in PBS and incubated at room temperature for 1 hour. The plates were then washed again three times with wash buffer.

After the washing step, 50 µl of 0.4 µg/ml DR4 ECD immunoadhesin protein in assay buffer was added to each well. The plates were incubated for 1 hour at room temperature on a shaker apparatus, followed by washing three times with wash buffer.

Following the wash steps, 100 µl of the hybridoma supernatants or Protein G-sepharose column purified antibody (10 µg/ml) was added to designated wells. 100 µl of P3X63AgU.1 myeloma cell conditioned medium was added to other designated wells as controls. The plates were incubated at room temperature for 1 hour on a shaker apparatus and then washed three times with wash buffer.

Next, 50 µl HRP-conjugated goat anti-mouse IgG Fc (purchased from Cappel Laboratories), diluted 1:1000 in assay buffer (0.5% bovine serum albumin, 0.05% Tween-20 in PBS), was added to each well and the plates incubated for 1 hour at room temperature on a shaker apparatus. The plates were washed three times with wash buffer, followed by addition of 50 µl of substrate (TMB Microwell Peroxidase Substrate; Kirkegaard & Perry, Gaithersburg, MD) to each well and incubation at room temperature for 10 minutes. The reaction was stopped by adding 50 µl of TMB 1-Component Stop Solution (Diethyl Glycol; Kirkegaard & Perry) to each well, and absorbance at 450 nm was read in an automated microtiter plate reader.

Hybridoma supernatants initially screened in the ELISA were considered for their ability to bind to DR4-IgG but not to CD4-IgG. The supernatants testing positive in the ELISA were further analyzed by FACS analysis using 9D cells (a human B lymphoid cell line expressing DR4; Genentech, Inc.) and FITC-conjugated goat anti-mouse IgG. For this analysis, 25 µl of cells suspended (at 4 X 10⁶ cells/ml) in cell sorter buffer (PBS containing 1% FCS and 0.02% NaN₃) were added to U-bottom microtiter wells, mixed with 100µl of culture supernatant or purified antibody (10µg/ml) in cell sorter buffer, and incubated for 30 minutes on ice. The cells were then washed and incubated with 100 µl FITC-conjugated goat anti-mouse IgG for 30 minutes at 4°C. Cells were then washed twice, resuspended in 150 µl of cell sorter buffer and then analyzed by FACScan (Becton Dickinson, Mountain View, CA).

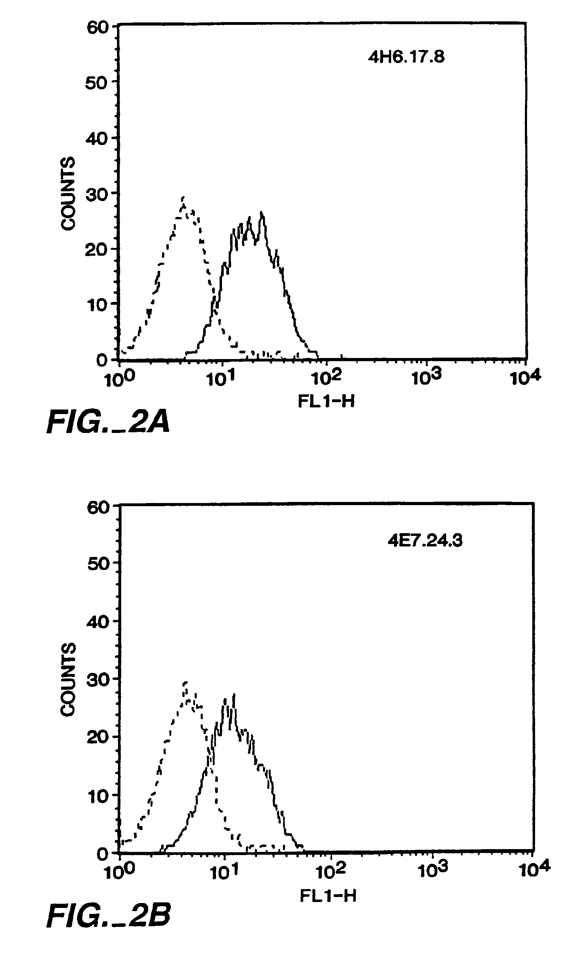

Figure 2 shows the FACS staining of 9D cells. Two particular antibodies, 4E7.24.3 and 4H6.17.8, recognized the DR4 receptor on the 9D cells.

### EXAMPLE 3

### Assay for Ability of DR4 Antibodies to Agonistically induce Apoptosis

Hybridoma supernatants and purified antibodies (as described in Example 2 above) were tested for activity to induce DR4 mediated 9D cell apoptosis. The 9D cells (5 X 10⁵ cells/0.5ml) were incubated with 5 µg of DR4 mAbs (4E7.24.3 or 4H6.17.8; see Example 2 above) or IgG control antibodies in 200 µl complete RPMI media at 4°C for 15 minutes. The cells were then incubated for 5 minutes at 37°C with or without 10 µg of goat anti-mouse IgG Fc antibody (ICN Pharmaceuticals) in 300 µl of complete RPMI. At this point, the cells were incubated overnight at 37°C and in the presence of 7% CO₂. The cells were then harvested and washed once with PBS. The apoptosis of the cells was determined by staining of FITC-annexin V binding to phosphatidylserine according to manufacturer recommendations (Clontech). The cells were washed in PBS and resuspended in 200 µl binding buffer. Ten µl of annexin-V-FITC (1 µg/ml) and 10 µl of propidium iodide were added to the cells. After incubation for 15 minutes in the dark, the 9D cells were analyzed by FACS.

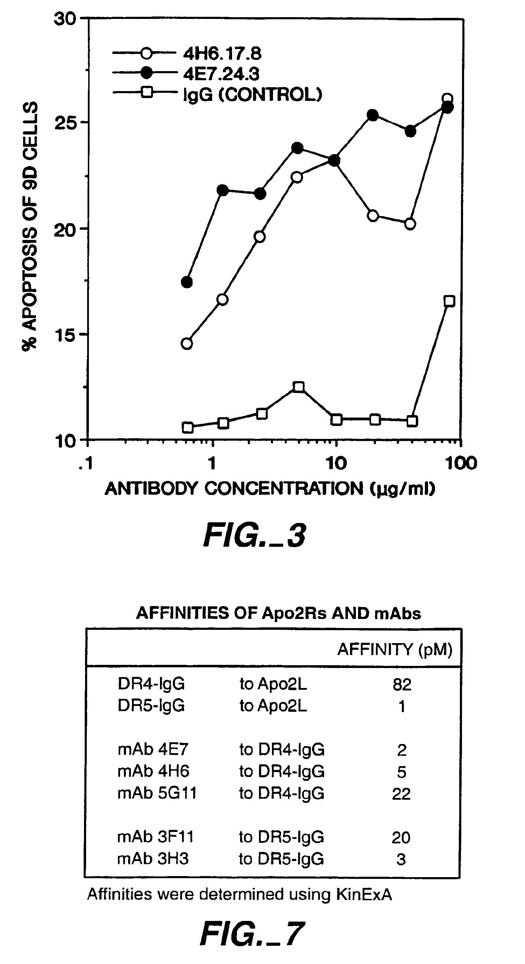

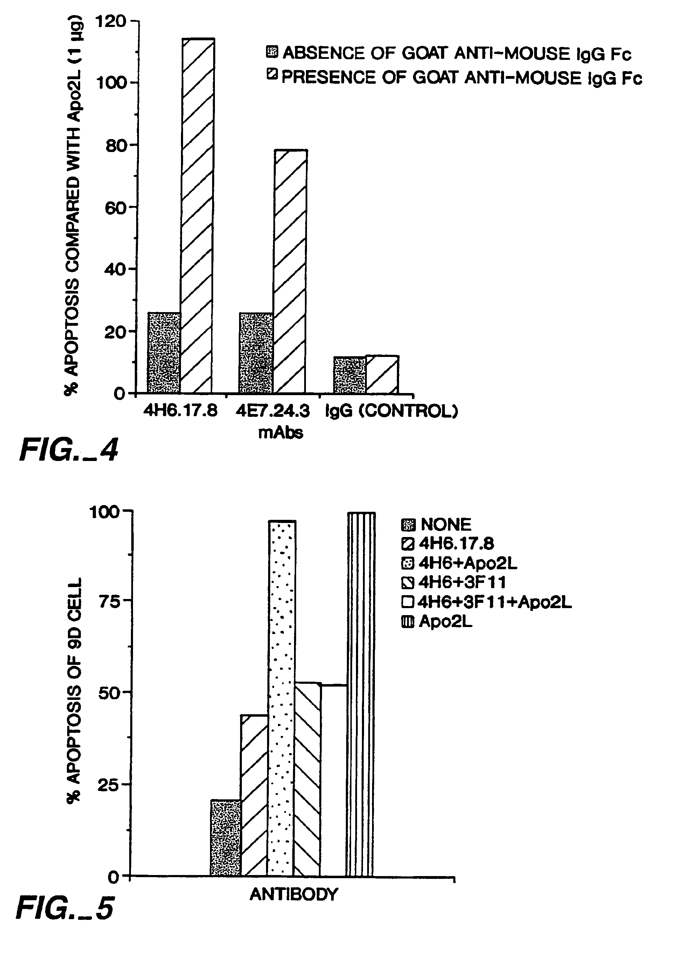

As shown in Figure 3, both DR4 antibodies (in the absence of the goat anti-mouse IgG Fc) induced apoptosis in the 9D cells as compared to the control antibodies. Agonistic activity of both DR4 antibodies, however, was enhanced by DR4 receptor cross-linking in the presence of the goat anti-mouse IgG Fc (See Figure 4). This enhanced apoptosis (Figure 4) by both DR4 antibodies is comparable to the apoptotic activity of Apo-2L in 9D cells.

### EXAMPLE 4

### Assay for DR4 Antibody Ability to Block Apo-2L-induced 9D Apoptosis

Hybridoma supernatants and purified antibodies (as described in Example 2 above) were tested for activity to block Apo-2 ligand induced 9D cell apoptosis.

The 9D cells (5 X 10⁵ cells/0.5 ml) were suspended in complete RPMI media (RPMI plus 10% FCS, glutamine, nonessential amino acids, penicillin, streptomycin, sodium pyruvate) and preincubated with serially diluted DR4 antibody (4H6.17.8) and/or an Apo-2 antibody (mAb 3F11, ATCC No. HB-12456) in individual Falcon 2052 tubes. The tubes containing the cells were incubated on ice for 15 minutes and then about 0.5 ml of Apo-2L (1 µg/ml; soluble His-tagged Apo-2L prepared as described in WO 97/25428) was suspended into complete RPMI media, added to the tubes containing the 9D cells and antibody, and then incubated overnight at 37°C and in the presence of 7% CO₂. The incubated cells were then harvested and washed once with PBS. The viability of the cells was determined by staining of FITC-annexin V binding to phosphatidylserine according to manufacturer recommendations (Clontech). Specifically, the cells were washed in PBS and resuspended in 200 µl binding buffer. Ten ml of annexin-V-FITC (1 µ g/ml) and 10 µl of propidium iodide were added to the cells. After incubation for 15 minutes in the dark, the 9D cells were analyzed by FACS.

The results are shown in Figure 5. Since 9D cells express more than one receptor for Apo-2L, Apo-2L can induce apoptosis in the 9D cells by interacting with either DR4 or the receptor referred to as Apo-2. Thus, to detect any blocking activity of the DR4 antibodies, the interaction between Apo-2 and Apo-2L needed to be blocked. In combination with the blocking anti-Apo-2 antibody, 3F11, the DR4 antibody 4H6.17.8 was able to block approximately 50% of apoptosis induced by Apo-2L. The remaining approximately 50% apoptotic activity is believed to be due to the agonistic activity of the DR4 antibodies alone, as shown in Figure 5. Accordingly, it is believed that 4H6.17.8 is a blocking DR4 antibody. (In a similarly conducted assay, Applicants found the 1H5 antibody, described in Example 7, blocked apoptosis of 9D cells by Apo-2L).

### EXAMPLE 5

### Antibody Isotyping

The isotypes of the 4H6.17.8 and 4E7.24.3 antibodies (as described above) were determined by coating microtiter plates with isotype specific goat anti-mouse Ig (Fisher Biotech, Pittsburgh, PA) overnight at 4°C. The plates were then washed with wash buffer (as described in Example 2 above). The wells in the microtiter plates were then blocked with 200 µl of 2% bovine serum albumin and incubated at room temperature for one hour. The plates were washed again three times with wash buffer.

Next, 100 µl of 5 µg/ml of purified DR4 antibodies or 100 µl of the hybridoma culture supernatant was added to designated wells. The plates were incubated at room temperature for 30 minutes and then 50 µl HRP-conjugated goat anti-mouse IgG (as described above) was added to each well. The plates were incubated for 30 minutes at room temperature. The level of HRP bound to the plate was detected using HRP substrate as described above.

The isotyping analysis showed that the 4E7.24.3 and 4H6.17.8 antibodies are IgG1 antibodies.

### EXAMPLE 6

### ELISA Assay to Test Binding of DR4 Antibodies to Other Apo-2L Receptors

An ELISA was conducted to determine if the two DR4 antibodies described in Example 2 were able to bind other known Apo-2L receptors beside DR4. Specifically, the DR4 antibodies were tested for binding to Apo-2 [see, e.g., Sheridan et al., Science, 277:818-821 (1997)], DcR1 [Sheridan et al., supra], and DcR2 [Marsters et al., Curr. Biol., al., 7:1003-1006 (1997)]. The ELISA was performed essentially as described in Example 2 above.

The results are shown in Figure 6. The DR4 antibodies 4E7.24.3 and 4H6.17.8 bound to DR4, and showed some cross-reactivity to Apo-2, DcR1 or DcR2.

### EXAMPLE 7

### Preparation of Monoclonal Antibodies Specific for DR4

Monoclonal antibodies to DR4 were produced essentially as described in Example 2. Using the capture ELISA described in Example 2, additional anti-DR4 antibodies, referred to as 1H5.24.9, 1H8.17.5, 3G1.17.2, 4G7.18.8, 4G10.20.6 and 5G11.17.1 were identified. (See Table in Figure 17) Further analysis by FACS (using the technique described in Example 2) confirmed binding of these antibodies to 9D cells expressing DR4 (data not shown).

### EXAMPLE 8

### Antibody Isotyping

The isotypes of the 1H5.24.9, 1H8.17.5, 3G1.17.2, 4G7.18.8, 4G10.20.6 and 5G11.17.1 anti-DR4 antibodies (described in Example 7) were determined essentially as described in Example 5.

The isotyping analysis showed that the 1H8.17.5, 3G1.17.2 and 4H10.20.6 are IgG1 antibodies. Anti-DR4 antibodies 1H5.24.9 and 4G7.18.8 are IgG2a antibodies, and antibody 5G11.17.1 is an IgG2b antibody.

### EXAMPLE 9

### Determination of Monoclonal Antibody Affinities

The equilibrium dissociation and association constant rates of various DR4 antibodies (described in the Examples above) were determined using KinExA™, an automated immunoassay system (Sapidyne Instruments, Inc., Boise, ID), as described with a modification by Blake et al., Journal of Biological Chemistry, 271:27677-685 (1996); and Craig et al., Journal of Molecular Biology, 281:183-201 (1998). Briefly, 1.0 ml of anti-human IgG agarose beads (56 µm, Sigma, St. Louis, MO) were coated with 20 µg of DR4-IgG (described in Example 1) in PBS by gentle mixing at room temperature for 1 hour. After washing with PBS, non-specific binding sites were blocked by incubating with 10% human serum in PBS for 1 hour at room temperature.

A bead pack (-4 mm high) was created in the observation flow cell by the KinExA™ instrument. The blocked beads were diluted into 30 ml of assay buffer (0.01% BSA/PBS). The diluted beads (550 µl) were next drawn through the flow cell with a 20 µm screen and washed with 1 ml of running buffer (0.01% BSA; 0.05% Tween 20 in PBS). The beads were then disrupted gently with a brief backflush of running buffer, followed by a 20 second setting period to create a uniform and reproducible bead pack. For equilibrium measurements, the selected DR4 antibodies (5 ng/ml in 0.01 % BSA/PBS) were mixed with a serial dilution of DR4-IgG (starting from 2.5 nM to 5.0 pM) and were incubated at room temperature for 2 hours. Once equilibrium was reached, 4.5 ml of this mixture was drawn through the beads, followed by 250 µl of running buffer to wash out the unbound antibodies. The primary antibodies bound to beads were detected by 1.5 ml of phycoerythrin labeled goat anti-mouse IgG (Jackson Immunoresearch). Unbound labeled material was removed by drawing 4.5 ml of 0.5 M NaCl through the bead pack over a 3 minute period. The equilibrium constant was calculated using the software provided by the manufacturer (Sapidyne, Inc.).

The affinity determinations for the DR4 antibodies are shown in Figure 7. Affinity determinations for immunoadhesin constructs of the DR4 and DR5 receptors for Apo-2L, and for the DR5 antibody, 3F11, for an Ig construct of DR5, are shown for comparison. The affinities (Kd-1) of the 4E7.24.3, 4H6.17.8 and 5G11 antibodies were 2 pM, 5 pM, and 22 pM, respectively, demonstrating that these monoclonal antibodies have strong binding affinities to DR4-IgG. (The affinities (Kd-1) of the 4G7 and 3G1 antibodies were 20 pM and 40 pM, respectively, data not shown in Figure 7)

### EXAMPLE 10

### Apoptosis Assay of Lymphoid Tumor Cells Using DR4 Antibodies

Apoptosis of human 9D B lymphoid tumor cells induced by anti-DR4 monoclonal antibodies was examined.

Human 9D cells (5x10⁵) were suspended in 100 microliter complete RPMI medium (RPMI plus 10% FCS, glutamine, nonessential amino acids, penicillin, streptomycin and sodium pyruvate) and added to 24 well macrotiter wells (5x10⁵ cells/0.5 ml/well). 100 microliter of 10 microgram/ml of purified DR4 antibody or 100 microliter of culture supernatant and then added into the wells containing 9D cells. The cells were then incubated overnight at 37º C in the presence of 7% CO2.

At the end of the incubation, cells were washed once with PBS. The washed cells were resuspended in 200 microliter binding buffer (Clontech) and 10 microliter of FITC-Annexin V (Clontech) and 10 microliter of propidium iodide were added to the cells. [See, Moore et al., Meth. In Cell Biol., 57:265 (1998)]. After incubation for 15 minutes in the dark, the cells were analyzed by FACScan.

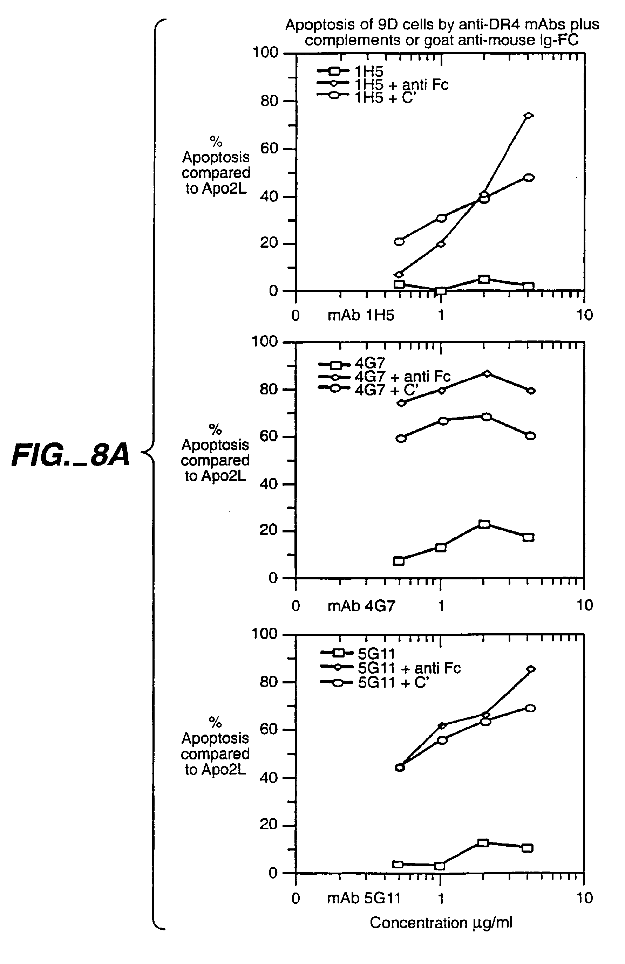

The results are shown in Figure 8A. The graphs in Figure 8A show that the 1H5, 4G7, and 5G11 antibodies by themselves induced some (weak) apoptosis in the 9D cells, but the apoptotic activity of each antibody was markedly increased when these monoclonal antibodies were cross-linked by either goat anti-mouse IgG-Fc or complement (as described in Example 11 below).

### EXAMPLE 11

### Apoptosis Assay of 9D Cells Using Cross-linked DR4 Antibodies

The apoptotic activity of cross-linked DR4 antibodies on 9D cells was also examined. The 9D cells (5x10⁵) were suspended in 100 microliter complete RPMI medium (RPMI plus 10% FCS, glutamine, nonessential amino acids, penicillin, streptomycin and sodium pyruvate) and incubated with 1 microgram of DR4 antibody/100 microliter on ice for 15 minutes. The cells were incubated with a 1:10 final dilution of rabbit complement (Cedar Lane) or 100 microgram/ml of goat anti-mouse IgG-Fc (Cappel Laboratories) in 300 microliter complete medium overnight at 37º C in the presence of 7% CO2.

At the end of the incubation, cells were washed once with PBS and suspended in 200 microliter of binding buffer (Clontech). Next, 10 microliter of FITC-Annexin V (Clontech) and 10 microliter of propidium iodide were added to the cells. [See, Moore et al., Cell Biol., 57:265 (1998)]. After incubation for 15 minutes in the dark, the cells were analyzed by FACScan.

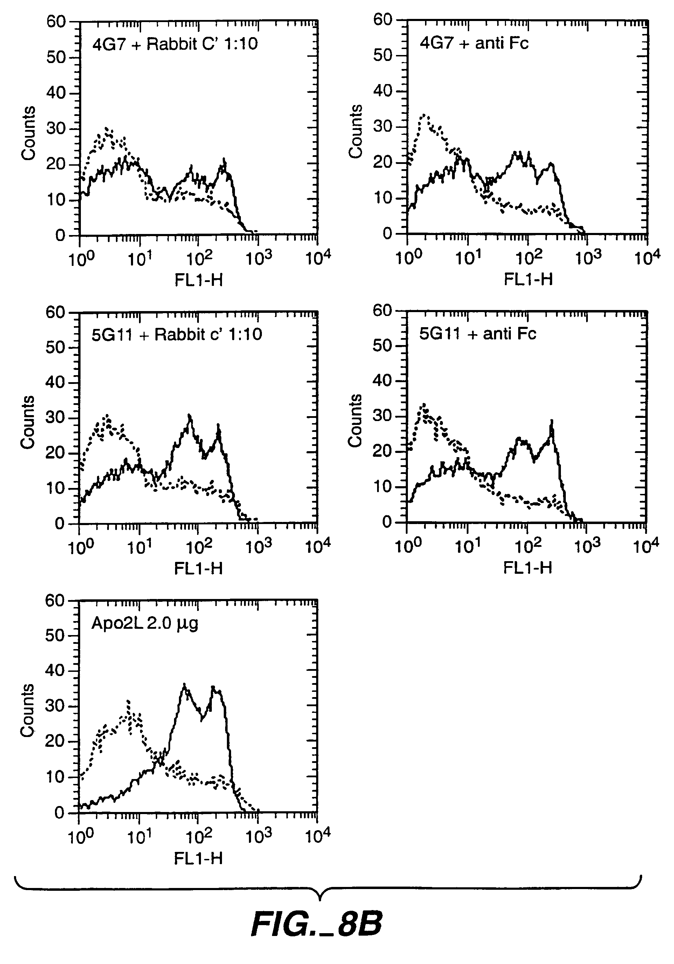

The results are shown in Figures 8A and 8B. The results show that the 4G7.17.8, 5G11.17.1 and 1H5.24.9 anti-DR4 antibodies induced apoptosis of 9D cells when cross-linked with goat anti-mouse IgG or rabbit complement, although the degree of apoptosis induced using complement as a linker was not as potent as compared to the use of the goat anti-mouse IgG-Fc linker. However, the apoptotic activity of the cross-linked DR4 antibodies (at concentrations of about 1-2 microgram/ml) was comparable to the apoptotic activity of Apo-2L at similar concentrations.

### EXAMPLE 12

### Apoptosis Assay of Human Lung and Colon Tumor Cell Lines

The apoptotic activities of the monoclonal antibodies were further examined in assays to determine the cell viability of cancer cells after treatment with the antibodies or Apo-2L.

SKMES-1 cells (human lung tumor cell line; ATCC) and HCT-116 cells (human colon tumor cell line; ATCC) were seeded at 4x10⁴ cells/well in complete high glucose 50:50 medium supplemented with glutamine, penicillin and streptomycin, in tissue culture plates and allowed to attach overnight at 37ºC. The media was then removed from the wells, and 0.1 ml of antibody (anti-DR4 antibodies diluted 0.001-10 microgram/ml in complete medium) was added to selected wells. Control wells without antibody received a media change with or without Apo-2L. The plates were then incubated for 1 hour at room temperature.

The culture supernatant was removed from the wells containing the test antibodies, and 10 microgram/ml goat anti-mouse IgG-Fc (Cappel Laboratories) or rabbit complement (Cedar Lane; diluted in medium to 1:10) was added to the wells. Media was changed in the control wells. The plates were incubated overnight at 37ºC. As a control, Apo-2L (as described in Example 4) (in potassium phosphate buffer, pH 7.0) was diluted to 2 microgram/ml. 0.1 ml of the diluted Apo-2L solution was added to selected wells, and then serial three-fold dilutions were carried down the plate.

Culture supernatants were then removed from the wells by aspiration, and the plates were flooded with 0.5% crystal violet in methanol solution. After 15 minutes, the crystal violet solution was removed by flooding the plates with running tap water. The plates were then allowed to dry overnight.

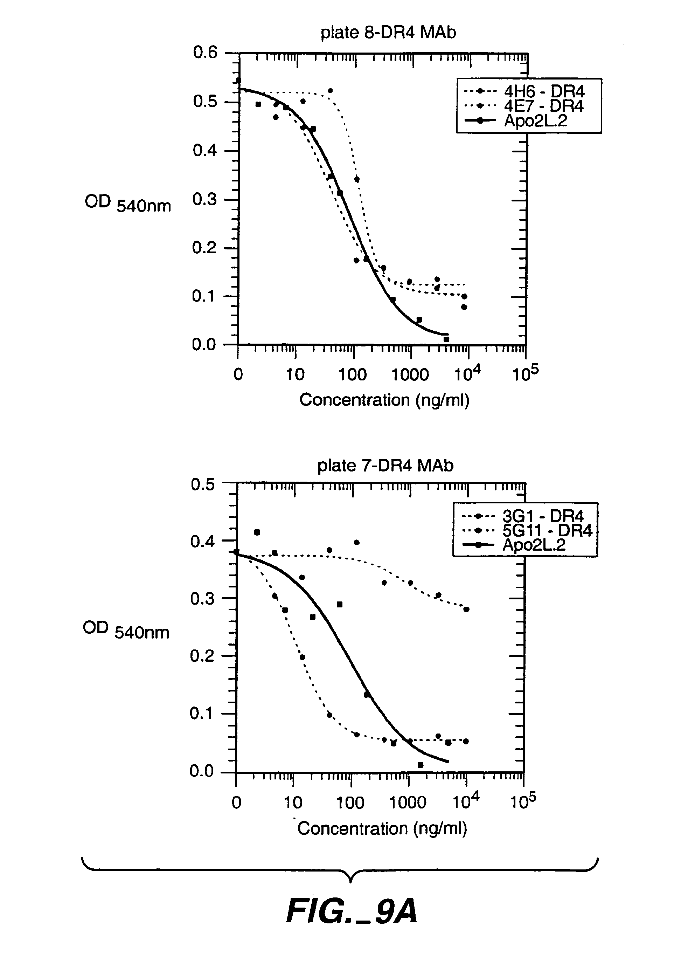

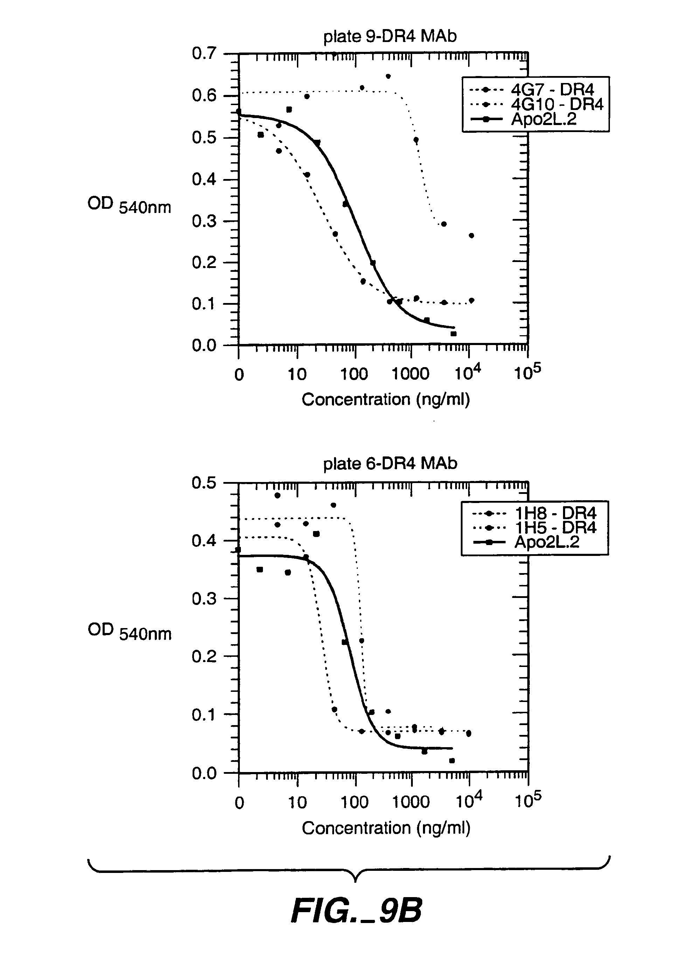

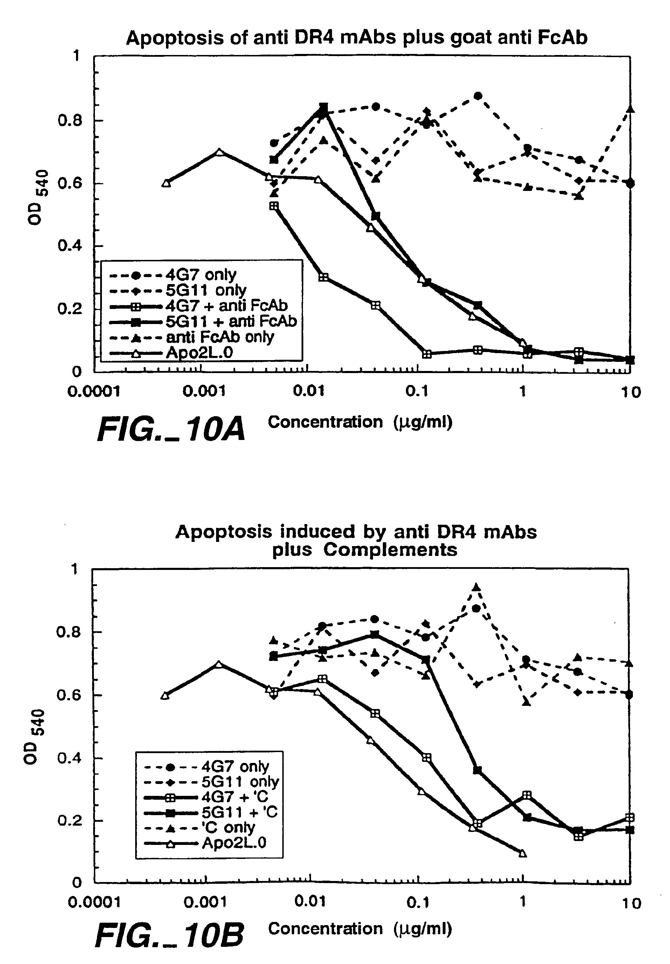

Absorbance was read on an SLT 340 ATC plate reader (Salzburg, Austria) at 540 nm. The data was analyzed using an Excel macro and 4p-fit. The results illustrating the activity of the DR4 antibodies on SKMES cells are shown in Figures 9 and 10. Figures 9 and 10A show that the 1H8.17.5, 4E7.24.3, 4G7.17.8, 4H6.17.8, 4G10.20.6, and 5G11.17.1 antibodies induced cell death of the SKMES cells when the cells were incubated with the respective antibodies plus goat anti-mouse IgG Fc. (The 1H5 antibody has also been found to induce cell death of the SKMES cells, data not shown in Figures 9 and 10A). In contrast, the 3G1.17.2 antibody did not induce cell death in the cells, even in the presence of the IgG Fc cross-linker. Figure 10B illustrates the apoptotic activity of the 4G7 (IgG2a isotype) and 5G11 (IgG2b isotype) antibodies on the SKMES cells in the presence of rabbit complement.

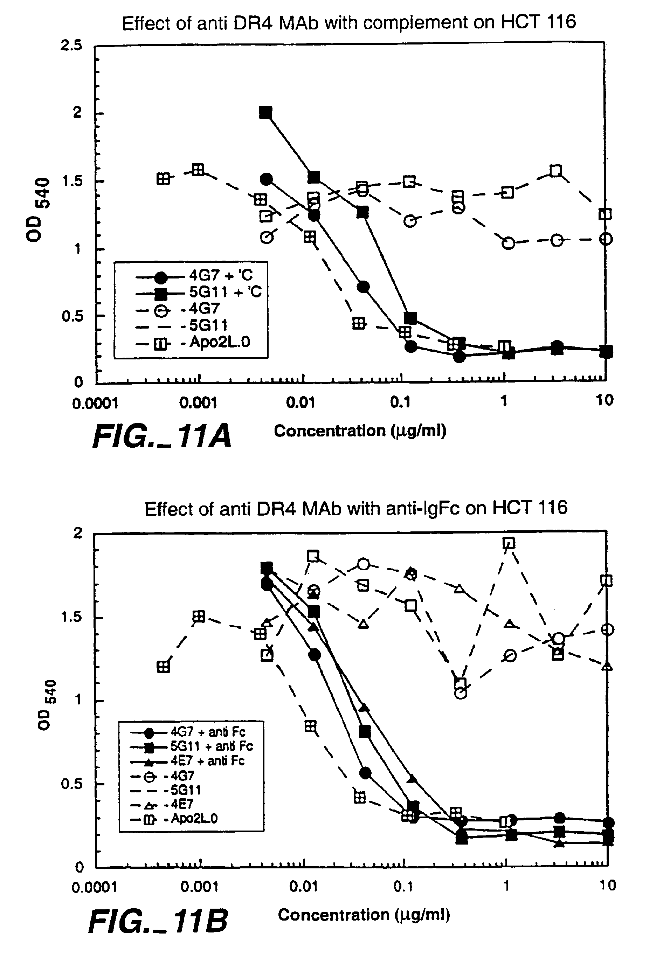

The results illustrated in Figure 11 show the activity of the DR4 antibodies on the HCT116 colon cancer cells. The IgG2 isotype DR4 antibodies, 4G7 and 5G11, induced apoptosis in the colon cancer cells in the presence of IgG Fc or complement. The DR4 antibody, 4E7 (IgG1 isotype), did not induce apoptosis in the presence of complement, although the antibody did demonstrate potent apoptotic activity in the presence of goat anti-mouse IgG Fc.

### EXAMPLE 13

### ELISA Assay to Test Binding of DR4 Antibodies to Other Apo-2L Receptors

An ELISA assay was conducted (as described in Examples 2 and 6) to determine binding of the DR4 antibodies to other known Apo-2L receptors, beside DR4.

The 5G11.17.1 antibody bound to DR4 and Apo-2, and showed some (weak) cross-reactivity to DcR1 and DcR2. The 4G10.20.6 antibody bound to DR4 and showed some (weak) cross-reactivity to Apo-2. The other antibodies, 1H8.17.5, 4G7.18.8, 1H5.24.9, and 3G1.17.2, bound to DR4 but not to any of the Apo-2, DcR1, or DcR2 receptors.

### EXAMPLE 14

### poly ADP-ribose polymerase (PARP) assay

A PARP assay was conducted to determine whether the activity induced by the IgG2 anti-DR4 antibodies was achieved by apoptosis or by conventional complement lysis.

9D cells (5x10⁵ cells in 100 µl of complete medium (described in Example 11) were incubated with 100 µl of antibody (4G7 or 5G11) (1 mg/ml) for 15 minutes on ice. Then, 300 µl of Rabbit Complement (Cedar Lane; diluted with 1.0 ml of cold distilled water followed by the addition of 2.0 ml of media) was added to the cells. The cells were then incubated overnight at 37º C. At the end of the incubation, the cells were microcentrifuged, harvested and washed once in cell wash buffer (50mM Tris-HCl, pH 7.5, 0.15 M NaCl, 1 mM CaCl₂, 1 mM MgCl₂). The cell pellets were then lysed with 50 µl of cell lysis buffer (cell wash buffer plus 1% NP40) containing protease inhibitors, incubated on ice for 30 minutes, and then spun at 13,000rpm for 10 minutes.

The cell lysate was mixed with an equal volume of 2X SDS reducing buffer. After boiling 2 minutes, proteins were separated onto a 7.5% SDS PAGE gel and transferred to immunoblot PVDF membranes (Gelman). After blocking the nonspecific binding sites with blocking buffer (Boehringer Mannheim), poly-(ADP-ribose)-polymerase was detected using HRP-rabbit anti-poly(ADP-ribose)-polymerase (Boehringer Mannheim). This antibody will detect the intact (116Kd) as well as degraded (85Kd) PARP which is generated as an early step of apoptosis. Bound anti-HRP-rabbit anti-poly-(ADP-ribose)-polymerase was detected using chemiluminescent immunoassay signal reagents according to manufacturer instructions (Amersham, Arlington Heights, IL).

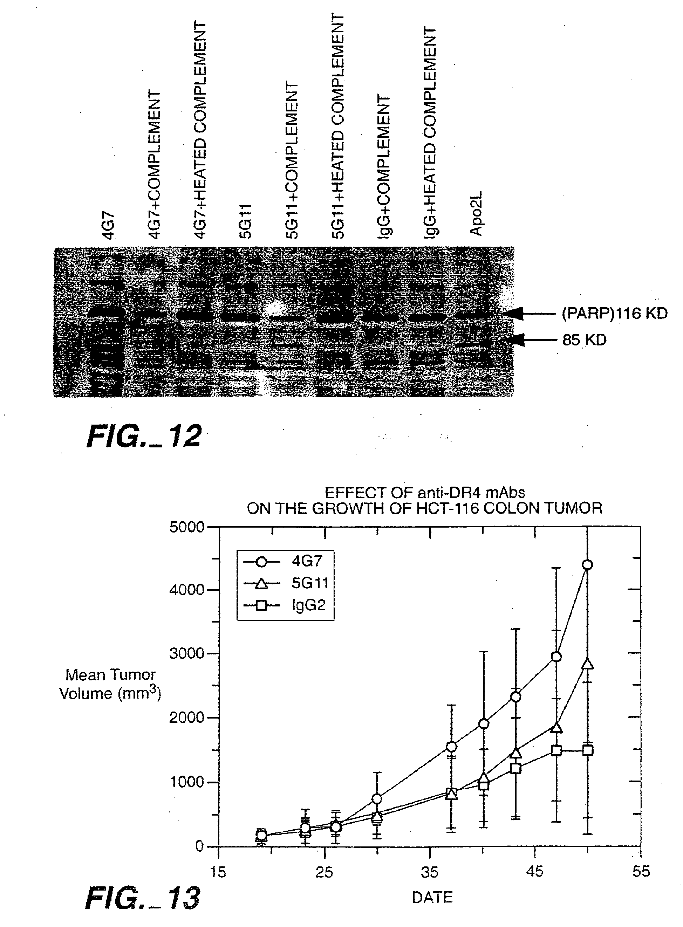

The results are shown in Figure 12. The cells treated with either 4G7 or 5G11 plus complement demonstrated the presence of cleaved 85 Kd PARP, indicating that the mechanism of the 9D cell death induced by the respective antibodies was due to apoptosis. When the complement added to the assay was heat inactivated by incubating for 30 minutes at 56ºC, the 85 Kd cleaved fragment of PARP was not detectable. The results suggest that the complement in the rabbit serum induced the oligomerization of the anti-DR4 antibodies bound to the cells, resulting in the apoptosis of the 9D cells.

### EXAMPLE 15

### In vivo Activity of DR4 Antibodies

Since the class IgG2 DR4 antibodies induced apoptosis in the presence of complement (described in the above Examples), an *in vivo* assay was conducted to determine if these antibodies may be able to induce apoptosis of tumor cells *in vivo* in the presence of native complement molecules present in the animal.

HCT116 cells (human colon tumor cell line; ATCC) or Colo205 cells (human colon tumor cell line; ATCC) were grown in high glucose F-12:DMEM (50:50) medium supplemented with 10% FCS, 2 mM glutamine, 100 µg/ml of penicillin, and 100 µg/ml streptomycin. The cells were harvested after treating with cell dissociation medium (Sigma, IAC) for 5 minutes. After washing in PBS, the tumor cells were resuspended in PBS at a concentration of 3X10⁷ cells/ml.

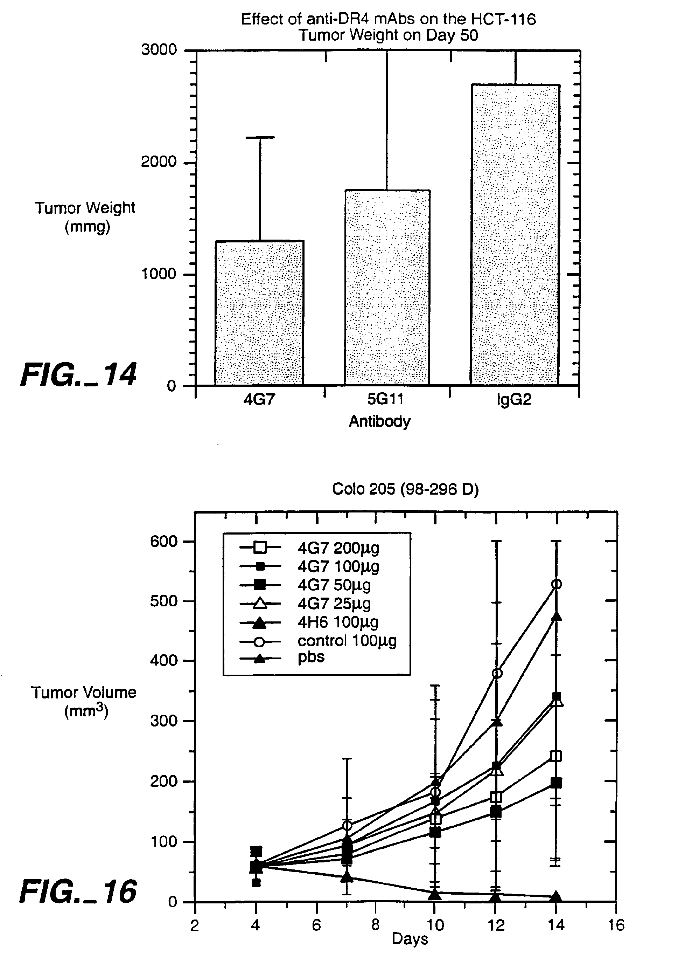

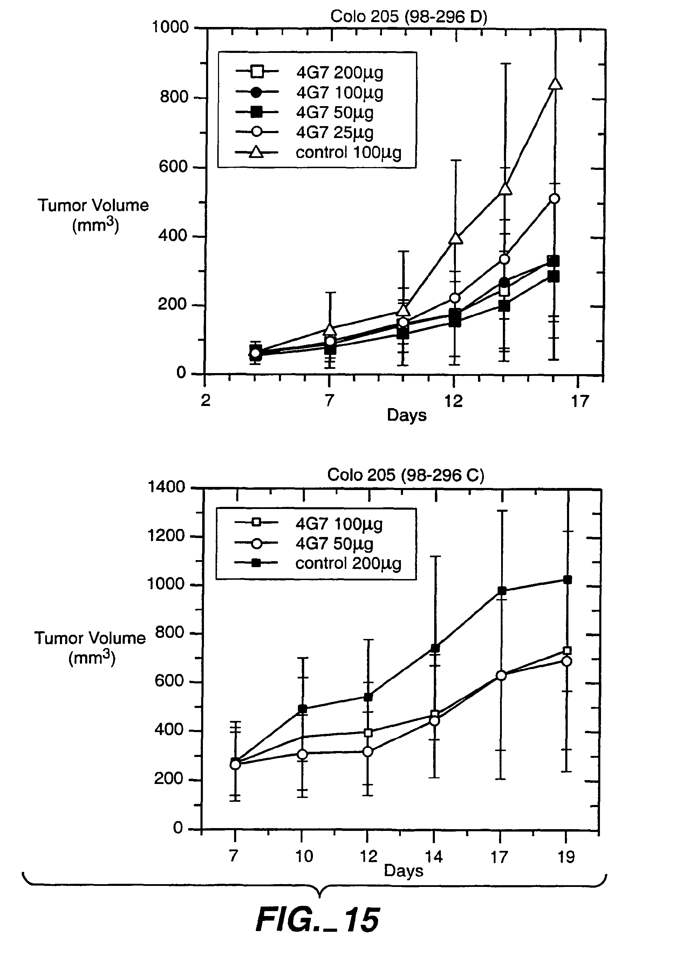

Nude mice were injected with 3-5 X 10⁶ cells subcutaneously in the dorsal area in a volume of 0.1 ml. When the tumor size in the HCT116 tumor bearing animals became a desired size, the mice were injected i.p. with 100 µg of monomeric anti-DR4 antibody in PBS three times per week, and the tumor sizes were measured three times/week. The Colo205 tumor bearing animals were injected i.p. with varying concentrations of the DR4 antibodies, 4G7 and 4H6 (as shown in Figures 15 and 16). At the end of the experiment examining the HCT116 tumors, the mice were sacrificed, and the weight of each tumor was determined.

The results illustrated in Figures 13 and 14 show that both 4G7 and 5G11 inhibited the growth of HCT116 tumors. There was approximately 35-40% and 50% growth inhibition of HCT116 tumors after treatment with antibodies 5G11 and 4G7, respectively.

The results illustrated in Figures 15 and 16 show that both 4G7 and 4H6 inhibited growth of Colo205 tumors. Fig. 15 illustrates that the antibody treatment was more effective when the size of the tumors were smaller. Fig. 16 shows that of the mice treated with 25-200 microgram of 4G7 (injected three times per week), the mice receiving the 50 microgram doses of 4G7 achieved the maximum inhibition (70%) of Colo205 tumor growth. The 4H6 antibody shrunk the Colo205 tumor growth to near zero after treatment for 10 days. At the end of 10 days treatment of 4H6 (100 microgram/injection), 4/8 mice showed no Colo205 tumor growth (data not shown). In related experiments, Applicants also found that tumor regression was similarly achieved with treatment of 4H6 at 5mg/kg once per week. It is noted that some of the tumors reappeared after administration of the 4H6 antibody was stopped, suggesting that some of the tumor cells were not completely eliminated during treatment. Histological sections of the Colo205 tumors three days after a single i.p. injection of 5mg/kg of the 4H6 antibody showed widespread apoptosis (the mice treated with control antibody of 4G7 antibody showed little apoptosis). In contrast, the extent and composition of the cellular infiltrate in the tumors appeared similar in the 4H6 antibody and control antibody treated animals. This data suggested that 4H6 antibody does exert the anti-tumor activity through induction of apoptosis in the tumor cells rather than indirectly by recruiting immune effector functions.

In further similarly conducted *in vivo* experiments using Colo205 tumor bearing nude mice, the mice were treated with the anti-DR4 antibodies above, including the 1H5 and 3G1 antibodies, at 2.5mg/kg, twice per week, starting on Day 4. On Day 22, the tumor sizes were measured and % growth inhibition was calculated based on the anti-tumor activity of the 4H6 monoclonal antibody as 100% inhibition. The tumor sizes of the PBS (control) and 4H6 antibody treated animals were 498±322 mm³ and mm³, respectively. The 3G1, 4E7, and 4H6 antibodies (all IgG1 isotype antibodies) demonstrated stronger anti-tumor activity than the IgG2 isotype antibodies, 1H5, 4G7, and mIgG2a-4H6 isotype switch variant (described below). The ranges of tumor growth inhibition by the IgG1 antibodies and the IgG2a antibodies were 42-100% and 27-30%, respectively. Despite that the 3G1 antibody exhibited relatively weak agonistic activity upon cross-linking in *vitro,* the 3G1 antibody inhibited the growth of the Colo205 tumor by 42% *in vivo.* These results suggested that the mIgG1 isotype may be more effective than the mIgG2a isotype in mediating anti-tumor activity through the DR4 receptor.

The study results also suggested that these DR4 antibodies, administered in the absence of exogenous linkers or modifiers, can be active anti-cancer agents. Although not fully understood, it is possible that the administered antibodies induced apoptosis by oligomerization through an endogenous mechanism such as interaction of the Fc region with native complement present in the animal or with Fcgamma receptors on effector cells or through spontaneous self Fc-Fc aggregation. It is believed that anti-DR4 antibodies of human Ig isotypes such as IgG1, IgG2, or IgG3 (which can fix complement), may similarly be capable of cross-linking using complement and inducing apoptosis.

To further examine the relative difference in activities of the two anti-DR4 antibodies above, a murine IgG2a isotype switch variant of the 4H6 murine monoclonal antibody was generated, and a comparison was made between its *in vitro* and *in vivo* activity and the parent 4H6 murine monoclonal.

The VH and VL genes were isolated by PCR amplification of mRNA from the corresponding 4H6 hybridoma as described in Carter et al., Proc. Natl. Acad. Sci., 89:4285-4289 (1992) using Taq polymerase. N-terminal amino acid sequences of the light and heavy chains of 4H6 were used to design the sense-strand PCR primers whereas the anti-sense PCR primers were based on consensus sequences of murine framework 4 of each chain. Amplified DNA fragments were digested with the restriction enzymes Nsi and RsrII for light chain and MluI and ApaI for heavy chain. (see Example 16 below for further details). The variable domain cDNAs of the light and heavy chains were separately assembled with the murine Ck and IgG2 CH1-CH2-CH3 domains in plasmid expression vectors. The light and heavy chain cDNA vectors were co-transfected into 293 cells for 7 days, the media was harvested, and the secreted IgG2a-4H6 form was recovered by affinity purification using Protein G.

*In vitro,* in an assay conducted essentially as described in Example 12 (except that Colo205 cells were utilized instead of HCT116 cells), the two different 4H6 isotypes showed similar activity upon cross-linking with goat anti-mouse IgG. In contrast, *in vivo,* in an assay conducted essentially as described in Example 15 (except that the animals were treated with antibodies at a dose of 2.5mg/kg, twice per week), the IgG1 isotype of 4H6 was substantially more active than its IgG2a counterpart. At a dose of 2.5 mg/kg, twice per week, IgG1-4H6 and IgG2a-4H6 inhibited growth of the Colo205 tumors by 96% and 35%, respectively, by Day 22. The anti-tumor activity of the IgG2a-4G7 antibody was similar to that of IgG2a-4H6 antibody. Accordingly, for at least those two antibodies, the isotype of the antibody appears to be more important for the *in vivo* activity than the target epitope.

### EXAMPLE 16

### Preparation of 4H6.17.8 Chimeric Antibody

Purified 4H6.17.8 antibody (see Example 2) was sequenced to obtain the N-terminal amino acids of both the heavy and light chain. The N-terminal sequence data was used to design PCR primers specific for the 5'ends of the variable regions of the light and heavy chains, while 3' primers were designed to anneal to the consensus framework 4 of each chain (Kabat, et al., Sequences of Proteins of Immunological Interest, Public Health Service, National Institutes of Health, Bethesda, MD, 1991). Briefly, a 3' degenerate primer representing all the potential framework 4 combinations for the antibody was designed. The primers were designed to add restriction enzyme sites for cloning; specifically NsiI and RsrII for light chain and MluI and ApaI for heavy chain (positions shown below in bolded text).
3' degenerate primers:
w= a/t, k=g/t, b=g/t/c, y=c/t, r=a/g, s=g/c, m=a/c, n=a/g/t/c 5' specific primers The underlined codons in SEQ ID NO:5 above correspond to those codons (Figure 18A) in the native sequence of the 4H6 antibody light chain encoding amino acids 21-26 shown in Figure 18A-C (SEQ ID NO:9). The underlined codons in SEQ ID NO:6 above correspond to those codons in the native sequence of the 4H6 antibody heavy chain encoding the first six amino acids of the heavy chain's variable domain. It is noted that the first two amino acids of the native sequence 4H6 variable domain are glutamine (Q) and valine (V) encoded by the codons cag and gtg, respectively. In contrast, in Figures 18D-18H, the first two amino acids of the heavy chain variable domain (appearing as positions 20 and 21, following the signal sequence) are shown as glutamic acid (E) and valine (V) encoded by the codons gaa and gtt, respectively. This switch in the codons encoding the first two amino acids of the heavy chain variable domain is due to the vector construct utilized; and in Figure 18D, the first two amino acids (and corresponding codons) of the variable domain (appearing at positions 20 and 21) reflect amino acids that are actually vector-derived.

Total RNA, extracted from 10⁸ cells of hybridoma 4H6.17.8 (see Example 2), with a Stratagene RNA isolation kit (200345), was used as substrate for RT-PCR. Reverse transcription was performed under standard conditions (Kawasaki, E. S. in PCR Protocols: A Guide to Methods and Applications, Innis, M. A., et al., eds. pp.21-27, Academic Press, Inc., San Diego, 1990) using the framework 4 degenerate primers and superscript II RNase H-Reverse Transcriptase (Gibco 18064-014). PCR amplification employed Taq polymerase (Perkin Elmer-Cetus), as described (Kawasaki, E. S., supra) except 2% DMSO was included in the reaction mix. Amplified DNA fragments were digested with restriction enzymes *Nsi I* and *Rsr II* (light chain) or *Mlu I* and *Apa I* (heavy chain), gel purified, and cloned into a vector, ss.vegf4chimera [see, Presta et al., Cancer Research, 57:4593-4599 (1997)]. The light and heavy chain murine variable domain cDNAs were inserted upstream and in frame to the human Ckappa and IgG1 CH1 domains. The C-terminal cysteine, which forms the disulfide bridge during F(ab')₂ generation, of the heavy chain in pAK19 (Carter et al., Bio/Technology, 10:163 (1992)), was removed to permit expression of only the Fab form of the antibody. The Fab protein was confirmed to bind specifically to its cognate receptor, DR4-IgG by a capture ELISA (performed essentially as described in Example 2 above except that HRP-sheep affinity purified IgG and anti-human IgG *F*(ab)'2 (Cappel Laboratories) were utilized at 1:2500). Once specificity was confirmed, the murine heavy chain variable domains of 4H6.17.8 were digested with restriction enzymes *Pvu II* and *Apa I*, gel purified, and cloned into the human IgG1 vector construct described in Carter et al., Proc. Natl. Acad. Sci., 89:4285 (1992) (in connection with humanization of 4D5 antibody). The first amino acid of the heavy chain was vector derived and resulted in a change of Q to E from the original or native sequence of 4H6.17.8, as described above. The second amino acid of the native sequence is V and remained a V, although encoded by a different codon, also vector derived, as described above. The variable domains of the murine light and heavy chain cDNAs were inserted upstream and in frame to the human Ckappa and IgG1 CH1-CH2-CH3 domains. The light and heavy chain chimeric cDNA vectors were co-transfected into CHO cells using standard techniques, and the antibodies secreted were then recovered by affinity purification using Protein G columns.

The encoding nucleotide sequence and putative amino acid sequence for the respective light and heavy chains of the 4H6.17.8 antibody are shown in Figures 18A-18H. The light chain included a variable domain comprising amino acids 20 to 126 of Figures 18A-18C (SEQ ID NO:9). Figures 18A-18C also show the signal sequence (amino acids 1 to 19 of Figures 18A-18C (SEQ ID NO:9)) and the human CH1 domain comprising amino acids 127 to 233 (Figures 18A-18C; SEQ ID NO:9). The heavy chain included a variable domain comprising amino acids 20 to 145 of Figures 18D-18H (SEQ ID NO:12). Figs. 18D-18H also show the signal sequence (amino acids 1 to 19 of Figures 18D-18H (SEQ ID NO:12)) and the human CH1, CH2, and CH3 domains (amino acids 146 to 476 of Figures 18D-18H (SEQ ID NO:12)).

### EXAMPLE 17

### In vitro Activities of Chimeric 4H6 Antibody

The effects of the anti-DR4 chimeric 4H6 antibody (see Example 16) on the viability of SK-MES-1 cells was determined by crystal violet staining. SK-MES-1 cells (human lung tumor cell line; ATCC) (4x10⁴ cells/100 ul/well) were incubated overnight (in DMEM/F-12 (50:50) medium supplemented with 10% FCS, 2mM glutamine and antibiotics) with serial dilutions of monoclonal antibodies with or without goat anti-mouse IgG Fc (10 µg/ml) or goat anti-human IgG Fc (10 µg/ml). The monoclonal antibodies tested included a F(ab)'2 preparation from murine 4H6.17.8, purified murine 4H.17.8 antibody (described in Example 2), and chimeric 4H6 antibody (described in Example 16). Serial dilutions of Apo2L/TRAIL (consisting of E. coli expressed, amino acids 114-281 of the Apo2L/TRAIL sequence disclosed in WO97/25428; see also, Ashkenazi et al., J. Clin. Invest., 104:155-162 (1999)) prepared in a final volume of 100 µl were added to each plate as a positive control. After incubation overnight at 37°C. the medium was removed and viable cells were stained using crystal violet as described by Flick et al., J. Immunol. Methods, 68:167-175 (1984). The plates were read on a SLT plate reader at 540 nM.

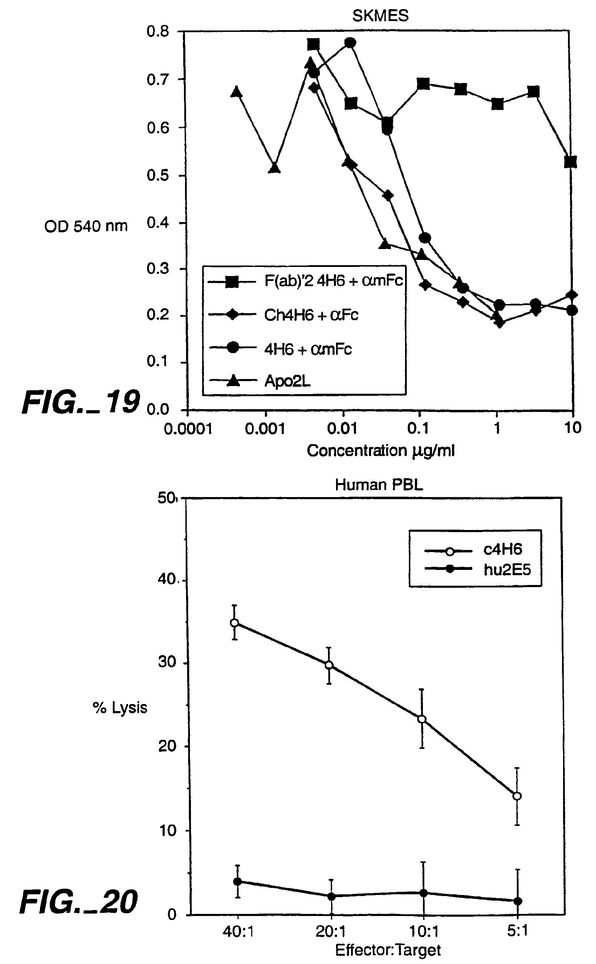

The results are shown in Figure 19. The SK-MES-1 cell killing activity of the cross-linked chimeric 4H6 antibody was comparable to the murine monoclonal 4H6.17.8.

Another assay was conducted to examine whether the chimeric 4H6 antibody induces antibody dependent cell mediated cytotoxicity (ADCC) *in vitro*.

Experiments were carried out by incubating ⁵¹Cr-labeled Colo205 cells (human colon tumor cell line; ATCC) (2 x 10⁴ cells/well in RPMI media supplemented with 10%FCS, 1% L-glutamine, 1% Penicillin-Streptomycin) with chimeric 4H6 antibody (5 µg/ml) first and then with human PBL overnight as a source of effector cells. The PBLs were purified from human whole blood by Ficoll-Hypaque centrifugation. As positive controls, ⁵¹Cr-Colo205 cells treated with chimeric 4H6 antibody plus goat anti-human IgG (10 µg/ml) (purchased from ICN Pharmaceuticals) were included. As a negative control, an anti-human IgE antibody ("2E5"; Genentech), was added. As shown in Figure 20, ⁵¹Cr-Colo205 cells treated with chimeric 4H6 antibody plus goat anti-human IgG resulted in 52% ⁵¹Cr release. At a 40:1 ratio of effector to target, there was 40% ⁵¹Cr release, suggesting that chimeric 4H6 antibody induces a significant level of ADCC. Percent ⁵¹Cr release was calculated based upon the total ⁵¹Cr release of ⁵¹Cr-Colo205 cells after 1% Triton-X100 treatment.

### EXAMPLE 18

### In vivo Activity of Chimeric 4H6 Antibody

Experiments were carried out essentially as described in Example 15. Colo205 tumor cells were grown in DMEM/F-12 (50:50) medium supplemented with 10% FCS, 2mM glutamine, and antibiotics. Female athymic nude mice (4-6 wk old, 7-8 mice per group) were injected subcutaneously with 5x10⁶ Colo205 cells in 0.2 ml PBS in the dorsal areas. Once sizes of tumors reached 50-100 mm³, mice were grouped randomly and monoclonal antibodies [purified murine 4H6.17.8 antibody (see Example 2); chimeric 4H6 antibody (see Example 16); and control IgG1 antibody] were given intraperitoneally in a volume of 0.1 ml at 5mg/kg, once per week.

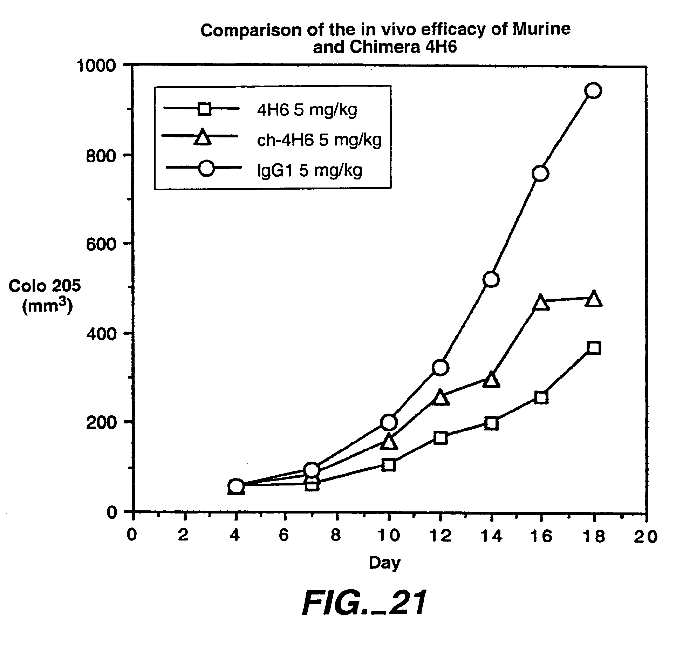

As shown in Fig. 21, chimeric 4H6 antibody demonstrated anti-tumor activity in the xenograft nude model, although the level of anti-tumor activity of the chimeric 4H6 antibody was not as potent as the murine 4H6.17.8 monoclonal antibody. The less potent anti-tumor activity of the chimeric 4H6 antibody is presently believed to be due to the heterologous system used for the *in vivo* study.

### Deposit of Material

The following materials have been deposited with the American Type Culture Collection, 10801 University Boulevard, Manassas, Virginia, USA (ATCC):

| Material | ATCC Dep. No. | Deposit Date |
|---|---|---|
| 4E7.24.3 | HB-12454 | Jan. 13, 1998 |
| 4H6.17.8 | HB-12455 | Jan. 13, 1998 |
| 1H5.25.9 | HB-12695 | April 1, 1999 - |
| 4G7.18:8 | PTA-99 | May 21, 1999 |
| 5G11.17.1 | HB-12694 | April 1, 1999 |

This deposit was made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 USC Section 122 and the Commissioner's rules pursuant thereto (including 37 CFR Section 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description.

## Claims

1. An isolated chimeric anti-DR4 antibody which comprises:
(a) a light chain variable domain, wherein said variable domain comprises amino acid residues 20 to 126 of SEQ ID NO:9;
(b) a light chain CH1 domain comprising amino acid residues 127 to 233 of SEQ ID NO:9;
(c) a heavy chain variable domain comprising amino acid residues 20 to 145 or 22 to 145 of SEQ ID NO:12; and
(d) heavy chain CH1, CH2, and CH3 domains comprising amino acid residues 146 to 476 of SEQ ID NO:12.

2. The chimeric anti-DR4 antibody of claim 1, wherein said antibody further-comprises a light chain signal sequence comprising amino acid residues 1 to 19 of SEQ ID NO:9.

3. A chimeric anti-DR4 antibody of claim 1 or claim 2, wherein said antibody is an agonist antibody which induces apoptosis in at least one type of mammalian cell.

4. The chimeric anti-DR4 antibody of claim 3, wherein said agonist antibody induces apoptosis in at least one type of mammalian cancer cell.

5. A chimeric anti-DR4 antibody according to any one of claims 1 to 4, wherein said antibody binds DR4 receptor with a binding affinity of at least 10⁹ M⁻¹ to 10¹² M⁻¹.

6. A chimeric anti-DR4 antibody according to any one of claims 1 to 5 wherein said antibody is conjugated to a cytotoxic agent or prodrug-activating enzyme.

7. An isolated nucleic acid encoding a chimeric anti-DR4 antibody according to any one of claims 1 to 6.

8. A vector comprising the isolated nucleic acid of claim 7.

9. A host cell comprising the vector of claim 8.

10. The host cell of claim 9 which is an *E*. *coli*.

11. The host cell of claim 9 which is a Chinese Hamster Ovary cell.

12. The host cell of claim 9 which is a yeast cell.

13. A method of producing a chimeric anti-DR4 antibody comprising culturing the host cell of any one of claims 9 to 12, and recovering the antibody from the host cell culture,

14. Use of a chimeric anti-DR4 antibody according to any one of claims 1 to 6 in the preparation of a medicament for inducing apoptosis in mammalian cancer cells.

15. Use according to claim 14, wherein said mammalian cancer cells comprise colon cancer cells, small cell lung cancer, non-small cell lung cancer, lung adenocarcinoma or lung squamous cell carcinoma.

## Patentansprüche

1. Isolierter chimärer Anti-DR4-Antikörper, umfassend:
(a) eine variable Leichtketten-Domäne, worin die variable Domäne die Aminosäure-Reste 20 bis 126 aus Seq.-ID Nr. 9 umfasst;
(b) eine Leichtketten-CH1-Domäne, umfassend die Aminosäure-Reste 127 bis 233 aus Seq.-ID Nr. 9;
(c) eine variable Schwerketten-Domäne, umfassend die Aminosäure-Reste 20 bis 145 oder 22 bis 145 aus Seq.-ID Nr. 12; und
(d) die Schwerketten-CH1-, -CH2- und -CH3-Domänen, umfassend die Aminosäure-Reste 146 bis 476 aus Seq.-ID Nr. 12.

2. Chimärer Anti-DR4-Antikörper nach Anspruch 1, worin der Antikörper weiters eine Leichtketten-Signalsequenz umfasst, umfassend die Aminosäure-Reste 1 bis 19 aus Seq.-ID Nr. 9.

3. Chimärer Anti-DR4-Antikörper nach Anspruch 1 oder Anspruch 2, worin der Antikörper ein Agonisten-Antikörper ist, der Apoptose in zumindest einem Säugetierzellen-Typ induziert.

4. Chimärer Anti-DR4-Antikörper nach Anspruch 3, worin der Agonisten-Antikörper Apoptose in zumindest einem Typ einer Säugetier-Krebszelle induziert.

5. Chimärer Anti-DR4-Antikörper nach einem der Ansprüche 1 bis 4, worin der Antikörper den DR4-Rezeptor mit einer Bindungsaffinität von zumindest 10⁹ M⁻¹ bis 10¹² M⁻¹ bindet.

6. Chimärer Anti-DR4-Antikörper nach einem der Ansprüche 1 bis 5, worin der Antikörper an ein zytotoxisches Mittel oder ein Prodrug-aktivierendes Enzym konjugiert ist.

7. Isolierte Nucleinsäure, kodierend für einen chimären Anti-DR4-Antikörper nach einem der Ansprüche 1 bis 6.

8. Vektor, umfassend die isolierte Nucleinsäure nach Anspruch 7.

9. Wirtszelle, umfassend den Vektor nach Anspruch 8.

10. Wirtszelle nach Anspruch 9, die eine E.-coli-Zelle ist.

11. Wirtszelle nach Anspruch 9, die eine Chinahamster-Eierstock-Zelle ist.

12. Wirtszelle nach Anspruch 9, die eine Hefe-Zelle ist.

13. Verfahren zur Produktion eines chimären Anti-DR4-Antikörpers, umfassend das Züchten der Wirtszelle nach einem der Ansprüche 9 bis 12 sowie das Gewinnen des Antikörpers aus der Wirtszellenkultur.

14. Verwendung eines chimären Anti-DR4-Antikörpers nach einem der Ansprüche 1 bis 6 zur Herstellung eines Medikaments zur Induktion von Apoptose in Säugetier-Krebszellen.

15. Verwendung nach Anspruch 14, worin die Säugetier-Krebszellen Kolon-Krebszellen, Zellen des kleinzelligen Bronchialkarzinoms, Zellen des nicht-kleinzelligen Bronchialkarzinoms, Zellen des Lungen-Adenokarzinoms oder Zellen des Lungen-Plattenepithelkarzinoms umfassen.

## Revendications

1. Anticorps anti-DR4 chimère isolé, qui comprend :
(a) un domaine variable de chaîne légère, ledit domaine variable comprenant les résidus d'aminoacides 20 à 126 de la SEQ ID N° 9 ;
(b) un domaine CH1 de chaîne légère, comprenant les résidus d'aminoacides 127 à 233 de la SEQ ID N° 9 ;
(c) un domaine variable de chaîne lourde, comprenant les résidus d'aminoacides 20 à 145 ou 22 à 145 de la SEQ ID N° 12 ; et
(d) des domaines CH1, CH2 et CH3 de chaîne lourde, comprenant les résidus d'aminoacides 146 à 476 de la SEQ ID N° 12.

2. Anticorps anti-DR4 chimère suivant la revendication 1, ledit anticorps comprenant en outre une séquence signal de chaîne légère comprenant les résidus d'aminoacides 1 à 19 de la SEQ ID N° 9.

3. Anticorps anti-DR4 chimère suivant la revendication 1 ou la revendication 2, ledit anticorps étant un anticorps agoniste qui induit une apoptose dans au moins un type de cellule de mammifère.

4. Anticorps anti-DR4 chimère suivant la revendication 3, ledit anticorps agoniste induisant une apoptose dans au moins un type de cellules cancéreuses de mammifère.

5. Anticorps anti-DR4 chimère suivant l'une quelconque des revendications 1 à 4, ledit anticorps se liant au récepteur DR4 avec une affinité de liaison d'au moins 10⁹ M⁻¹ 1 à 10¹² M⁻¹.

6. Anticorps anti-DR4 chimère suivant l'une quelconque des revendications 1 à 5, ledit anticorps étant conjugué à un agent cytotoxique ou une enzyme activatrice de précurseur de médicament.

7. Acide nucléique isolé codant pour un anticorps anti-DR4 chimère suivant l'une quelconque des revendications 1 à 6.

8. Vecteur comprenant l'acide nucléique isolé de la revendication 7.

9. Cellule hôte comprenant le vecteur de la revendication 8.

10. Cellule hôte suivant la revendication 9, qui est une cellule de *E. coli.*

11. Cellule hôte suivant la revendication 9, qui est une cellule d'ovaire d'hamster chinois.

12. Cellule hôte suivant la revendication 9, qui est une cellule de levure.

13. Procédé pour la production d'un anticorps anti-DR4 chimère, comprenant les étapes consistant à cultiver la cellule hôte de l'une quelconque des revendications 9 à 12 et à recueillir l'anticorps à partir de la culture de cellules hôtes.

14. Utilisation d'un anticorps anti-DR4 chimère suivant l'une quelconque des revendications 1 à 6 dans la préparation d'un médicament destiné à induire une apoptose dans des cellules cancéreuses de mammifère.

15. Utilisation suivant la revendication 14, dans laquelle lesdites cellules cancéreuses de mammifère comprennent des cellules de cancer du côlon, de cancer pulmonaire à petites cellules, de cancer pulmonaire non à petites cellules, d'adénocarcinome pulmonaire ou de carcinome pulmonaire à cellules squameuses.
